# EUROPEAN PATENT APPLICATION

(11) **EP 4 792 886 A2**
(43) Date of publication of application: **19.08.2026**
(21) Application number: 26180601.2
(22) Date of filing: 08.01.2016
(51) Int. Cl.: A61P 27/02

(54) **CNP PRODRUGS**

(30) Priority: 09.01.2015 EP 15150584; 24.03.2015 EP 15160457
(62) Document number(s) of the earlier application(s) in accordance with Art. 76 EPC: 16700198.1 / 3 242 689
(71) Applicant: Ascendis Pharma Growth Disorders A/S, 2900 Hellerup (DK)
(72) Inventor: Sprogøe, Kennett, 2900 Hellerup (DK); Hersel, Ulrich, 69120 Heidelberg (DE); Rau, Harald, 69120 Heidelberg (DE); Wegge, Thomas, 69120 Heidelberg (DE); Faltinger, Frank, 69120 Heidelberg (DE); Cleemann, Felix, 69120 Heidelberg (DE); Kaluza, Nora, 69120 Heidelber (DE); Bernhard, Ana, 69120 Heidelberg (DE); Buba, Annette, 69120 Heidelberg (DE); Woods, Tom, 69129 Heidelbeg (DE)
(74) Representative: Büchel, Edwin

(57) **Abstract**

The present invention relates to CNP prodrugs, pharmaceutical compositions comprising such CNP prodrugs and their uses.

## Description

The present invention relates to CNP prodrugs, a pharmaceutically acceptable salt thereof, pharmaceutical compositions comprising such CNP prodrugs or a pharmaceutically acceptable salt thereof and their uses.

Gain-of-function mutations in FGFR3 lead to achondroplasia (ACH), hypochondroplasia (HCH), and thanatophoric dysplasia (TD). These conditions, all due to increased signaling of fibroblast-growth-factor-receptor 3 (FGFR3), are characterized by a disproportionate rhizomelic dwarfism and differ in severity, which ranges from mild (HCH) to severe (ACH) and lethal (TD). FGFR3 is a key regulator of endochondral bone growth and signals through several intracellular pathways, including those of the signal transducer and activator of transcription (STAT) and mitogen-activated protein kinase (MAPK). FGFR3 constitutive activation impairs proliferation and terminal differentiation of the growth-plate chondrocytes and synthesis of the extracellular matrix. FGFR3 activation is associated with increased phosphorylation of the STAT and MAPK pathways. The MAPK signaling pathway is regulated by C-type natriuretic peptide (CNP). Binding of CNP to its receptor, natriuretic-peptide receptor B (NPR-B), inhibits FGFR3 downstream signaling and thus triggers endochondral growth and skeletal overgrowth, as observed in both mice and humans overexpressing CNP. Overproduction of CNP in the cartilage or continuous delivery of CNP through intravenous (iv) infusion normalizes the dwarfism of achondroplasic mice, suggesting that administration of CNP at supraphysiological levels is a strategy for treating ACH.

However, given its short half-life (2 min after intravenous (iv) administration) CNP as a therapeutic agent is challenging in a pediatric population because it would require continuous infusion. Furthermore, as CNP is extensively inactivated in the subcutaneous tissue iv infusion is required.

Potter (FEBS Journal 278 (2011) 1808-1817) describes the clearance of CNP to occur by two degradation routes: receptor-mediated degradation and degradation by extracellular proteases. CNP is degraded by the action of neutral endopeptidase 24.11 (NEP) and is removed by systemic circulation by natriuretic peptide clearance receptor, NPR-C, that binds to and deposits CNP into lysosomes, where CNP is degraded.

The ability of individual organs to remove molecules from the circulation is described by the extraction ratio, which is calculated by subtracting the venous concentration from the arterial concentration, and dividing this value by the arterial blood concentration of the molecule. This so-called A/V difference quantifies how efficiently the organ removes or degrades the molecule in question. In humans the CNP A/V gradients is negative for renal, hepatic and pulmonary tissue, consistent with CNP degradation occurring in these tissues.

Reducing degradation by one or both of these clearance routes, would serve to prolong the half-life of CNP.

Due to the limited size of its active site cavity, NEP preferably recognizes substrates smaller than about 3 kDa. US 8,377,884 B2 describe variants of CNP which optionally are permanently conjugated to PEG polymer to increases resistance to NEP cleavage. However, addition of PEG, even as small as 0.6 kDa, to wild-type CNP was found to reduce CNP activity, and addition of greater than about 2 or 3 kDa of PEG to CNP or variants thereof reduce CNP functional activity in a size-dependent manner. Therefore, attachment of PEG molecules larger than 2 to 3 kDa to reduce NEP degradation is accompanied by a loss of activity, which may reduce the therapeutic potential of such molecules.

In addition to negatively impacting activity of the peptide, conjugation of PEG or another macromolecule to CNP may also prevent effective distribution to the growth plate. Farnum et al. (Anat Rec A Discov Mol Cell Evol Biol. 2006 January; 288(1): 91-103) demonstrated that distribution of molecules from the systemic vasculature to the growth plate was size dependent, and that small molecules (up to 10 kDa) could distribute to the growth plate, whereas a molecular size of 40 kDa and larger prevented entry to the growth plate.

International application WO 2009/156481 A1 relates to reversible PEG-conjugates of BNP which term was defined as including all members of the family of natriuretic peptides. This application only focuses on the cardiovascular effects of this class of peptides, which are mediated through the natriuretic peptide receptor A (NPR-A). WO 2009/156481 A1 fails to disclose CNP's specific properties regarding the regulating of growth, proliferation and differentiation of cartilaginous growth plate chondrocytes, mediated via activation of the natriuretic peptide receptor B (NPR-B).

A different approach to create a NEP resistant CNP molecule and enable subcutaneous administration was described in The American Journal of Human Genetics 91, 1108-1114. BMN-111 is a modified recombinant human C-type Natriuretic Peptide (CNP) where 17 amino acids have been added to form a 39 amino acid CNP pharmacological analog. BMN-111 mimics CNP pharmacological activity at the growth plate and has an extended half-life as a result of neutral-endopeptidase (NEP) resistance that allows once-daily subcutaneous (SC) administration. As BMN-111 is a non-natural occurring peptide, the risk of inducing an immunological response is increased compared to the native peptide, and as described by Martz in "sFGFR for achondroplasia" (SciBx, Biocentury October 2013), an immunological response to BMN-111 has been observed in animal studies, with the presence of antibodies not affecting the pharmacological activity of the drug. However, BMN-111 only has a half-life of 20 minutes, which when dosed daily is associated with a short duration of exposure to efficacious drug levels.

To increase exposure to efficacious drug levels the dose of the drug having CNP activity may be increased. As natriuretic peptides are a family of hormones that may affect blood volume and blood pressure, an increase in dose may be associated with cardiovascular adverse effects. Studies of BMN-111in animals and man have demonstrated that as the dose increases, arterial blood pressure drops and heart rate increases. Doses of BMN-111 up to 15 µg/kg were associated with mild hypotension in healthy volunteers. Therefore increasing the dose of a drug having CNP activity to increase drug exposure, may be associated with unacceptable cardiovascular side effects.

In summary, there is a need for a more convenient and/or efficacious CNP treatment.

It is therefore an object of the present invention to at least partially overcome the shortcomings described above.

This object is achieved with a CNP prodrug or a pharmaceutically acceptable salt thereof, wherein the prodrug is of formula (Ia) or (Ib) 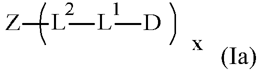 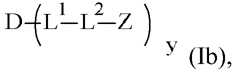 wherein
-D is a CNP moiety;
-L¹- is a reversible prodrug linker moiety;
-L²- is a single chemical bond or a spacer moiety;
-Z is a water-soluble carrier moiety;
x is an integer selected from the group consisting of 1, 2, 3, 4, 5, 6, 7, 8, 9, 10, 11, 12, 13, 14, 15 or 16; and
y is an integer selected from the group consisting of 1, 2, 3, 4 and 5.

In another aspect the present invention relates to a CNP prodrug or a pharmaceutically acceptable salt thereof comprising a conjugate D-L, wherein
-D is a CNP moiety; and
-L comprises a reversible prodrug linker moiety -L¹-;
wherein -L¹- is substituted with -L²-Z' and is optionally further substituted; wherein
-L²- is a single chemical bond or a spacer moiety; and
-Z' is a water-insoluble carrier moiety.

It is understood that a multitude of moieties -L²-L¹-D is connected to a water-insoluble carrier -Z'.

It was surprisingly found that the CNP prodrugs of the present invention and the pharmaceutically acceptable salts thereof provide an extended circulation time of the CNP in the bloodstream which leads to a more convenient and patient-friendly mode of administration, such as a once-weekly or up to once-monthly SC injection. At the same time, unmodified CNP is released which ensures effective distribution of the active agent to the growth plate. As the CNP prodrugs of the present invention have a low residual activity, i.e. binding to NPR-B, the risk of cardiovascular side effects, such as hypotension, is significantly reduced.

It was furthermore surprisingly found that the compounds of the present invention achieve more stable blood levels than those observed after daily bolus injections, which mimics more closely the physiological exposure to endogenous CNP. These more stable blood levels hold true for various dosage regiments, such as, for example, for daily administration; for administration every two days, every three days, every four days, every five days, every six days; for weekly administration; for bi-weekly administration and for monthly administration.

It was furthermore surprisingly found that a continuous release of CNP, such as from a controlled release system, such as from the prodrugs of the present invention, is more efficacious than a once-daily bolus injection.

Within the present invention the terms are used having the meaning as follows.

As used herein the term "CNP" refers all CNP polypeptides, preferably from mammalian species, more preferably from human and mammalian species, more preferably from human and murine species, as well as their variants, analogs, orthologs, homologs, and derivatives and fragments thereof, that are characterized by regulating the growth, proliferation and differentiation of cartilaginous growth plate chondrocytes. Preferably, the term "CNP" refers to the CNP polypeptide of SEQ ID NO:1 as well as its variants, homologs and derivatives exhibiting essentially the same biological activity, i.e. regulating the growth, proliferation and differentiation of cartilaginous growth plate chondrocytes. More preferably, the term "CNP" refers to the polypeptide of SEQ ID NO:1. It is equally preferred that the term "CNP" refers to SEQ ID NO:24, i.e. to a CNP moiety consisting of 38 amino acids, as well as its variants, homologs and derivatives exhibiting essentially the same biological activity, i.e. regulating the growth, proliferation and differentiation of cartilaginous growth plate chondrocytes.

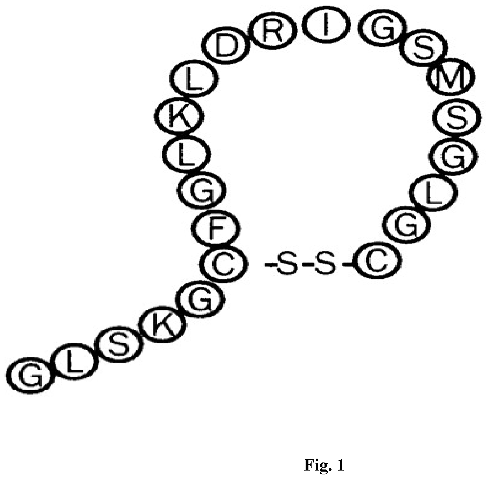

SEQ ID NO:1 has the following sequence:
GLSKGCFGLKLDRIGSMSGLGC,
wherein the cysteins at position 6 and 22 are connected through a disulfide-bridge, as illustrated in Fig. 1.

SEQ ID NO:24 has the following sequence:
LQEHPNARKYKGANKKGLSKGCFGLKLDRIGSMSGLGC,
wherin the cysteines at position 22 and 38 are connected through a disulfide-bride.

The term "CNP" also includes all CNP variants, analogs, orthologs, homologs and derivatives and fragments thereof as disclosed in WO 2009/067639 A2 and WO 2010/135541 A2, which are herewith incorporated by reference.

Accordingly, the term "CNP" also refers preferably to the following peptide sequences: SEQ ID NO:2 (CNP-53):
DLRVDTKSRAAWARLLQEHPNARKYKGANKKGLSKGCFGLKLDRIGSMSGLGC;
   SEQ ID NO:3 (G-CNP-53):
GDLRVDTKSRAAWARLLQEHPNARKYKGANKKGLSKGCFGLKLDRIGSMSGLGC;
   SEQ ID NO:4 (M-CNP-53):
MDLRVDTKSRAAWARLLQEHPNARKYKGANKKGLSKGCFGLKLDRIGSMSGLGC;
   SEQ ID NO:5 (P-CNP-53):
PDLRVDTKSRAAWARLLQEHPNARKYKGANKKGLSKGCFGLKLDRIGSMSGLGC;
   SEQ ID NO.6 (CNP-53 M48N):
DLRVDTKSRAAWARLLQEHPNARKYKGANKKGLSKGCFGLKLDRIGSNSGLGC;
   SEQ ID NO:7 (CNP-53 Δ15-31):
DLRVDTKSRAAWARGLSKGCFGLKLDRIGSMSGLGC;
   SEQ ID NO:8 (CNP-52):
LRVDTKSRAAWARLLQEHPNARKYKGANKKGLSKGCFGLKLDRIGSMSGLGC;
   SEQ ID NO:9 (CNP-51):
RVDTKSRAAWARLLQEHPNARKYKGANKKGLSKGCFGLKLDRIGSMSGLGC;
   SEQ ID NO:10 (CNP-50):
VDTKSRAAWARLLQEHPNARKYKGANKKGLSKGCFGLKLDRIGSMSGLGC;
   SEQ ID NO:11 (CNP-49):
DTKSRAAWARLLQEHPNARKYKGANKKGLSKGCFGLKLDRIGSMSGLGC;
   SEQ ID NO: 12 (CNP-48):
TKSRAAWARLLQEHPNARKYKGANKKGLSKGCFGLKLDRIGSMSGLGC;
   SEQ ID NO:13 (CNP-47):
KSRAAWARLLQEHPNARKYKGANKKGLSKGCFGLKLDRIGSMSGLGC;
   SEQ ID NO:14 (CNP-46):
SRAAWARLLQEHPNARKYKGANKKGLSKGCFGLKLDRIGSMSGLGC;
   SEQ ID NO:15 (CNP-45):
RAAWARLLQEHPNARKYKGANKKGLSKGCFGLKLDRIGSMSGLGC;
   SEQ ID NO:16 (CNP-44):
AAWARLLQEHPNARKYKGANKKGLSKGCFGLKLDRIGSMSGLGC;
   SEQ ID NO:17 (CNP-44 Δ14-22):
AAWARLLQEHPNAGLSKGCFGLKLDRIGSMSGLGC;
   SEQ ID NO:18 (CNP-44 Δ15-22):
AAWARLLQEHPNARGLSKGCFGLKLDRIGSMSGLGC;
   SEQ ID NO:19 (CNP-43):
AWARLLQEHPNARKYKGANKKGLSKGCFGLKLDRIGSMSGLGC;
   SEQ ID NO:20 (CNP-42):
WARLLQEHPNARKYKGANKKGLSKGCFGLKLDRIGSMSGLGC;
   SEQ ID NO:21 (CNP-41):
ARLLQEHPNARKYKGANKKGLSKGCFGLKLDRIGSMSGLGC;
   SEQ ID NO:22 (CNP-40):
RLLQEHPNARKYKGANKKGLSKGCFGLKLDRIGSMSGLGC;
   SEQ ID NO:23 (CNP-39):
LLQEHPNARKYKGANKKGLSKGCFGLKLDRIGSMSGLGC;
   SEQ ID NO: 24 (CNP-38):
LQEHPNARKYKGANKKGLSKGCFGLKLDRIGSMSGLGC;
   SEQ ID NO: 25 (CNP-37):
QEHPNARKYKGANKKGLSKGCFGLKLDRIGSMSGLGC;
   SEQ ID NO: 26 (CNP-37 Q1pQ, wherein pQ = pyroglutamate):
pQEHPNARKYKGANKKGLSKGCFGLKLDRIGSMSGLGC;
   SEQ ID NO:27 (G-CNP-37):
GQEHPNARKYKGANKKGLSKGCFGLKLDRIGSMSGLGC;
   SEQ ID NO:28 (P-CNP-37):
PQEHPNARKYKGANKKGLSKGCFGLKLDRIGSMSGLGC;
   SEQ ID NO:29 (M-CNP-37):
MQEHPNARKYKGANKKGLSKGCFGLKLDRIGSMSGLGC;
   SEQ ID NO:30 (PG-CNP-37):
PGQEHPNARKYKGANKKGLSKGCFGLKLDRIGSMSGLGC;
   SEQ ID NO:31 (MG-CNP-37):
MGQEHPNARKYKGANKKGLSKGCFGLKLDRIGSMSGLGC;
   SEQ ID NO:32 (CNP-37 M32N):
QEHPNARKYKGANKKGLSKGCFGLKLDRIGSNSGLGC;
   SEQ ID NO: 33 (G-CNP-37 M32N):
GQEHPNARKYKGANKKGLSKGCFGLKLDRIGSNSGLGC;
   SEQ ID NO:34 (G-CNP-37 K14Q):
GQEHPNARKYKGANQKGLSKGCFGLKLDRIGSMSGLGC;
   SEQ ID NO:35 (G-CNP-37 K14P):
GQEHPNARKYKGANPKGLSKGCFGLKLDRIGSMSGLGC;
   SEQ ID NO:36 (G-CNP-37 K14Q, Δ15):
GQEHPNARKYKGANQGLSKGCFGLKLDRIGSMSGLGC;
   SEQ ID NO:37 (G-CNP-37 K14Q, K15Q):
GQEHPNARKYKGANQQGLSKGCFGLKLDRIGSMSGLGC;
   SEQ ID NO:38 (CNP-36):
EHPNARKYKGANKKGLSKGCFGLKLDRIGSMSGLGC;
   SEQ ID NO:39 (CNP-35):
HPNARKYKGANKKGLSKGCFGLKLDRIGSMSGLGC;
   SEQ ID NO:40 (CNP-34):
PNARKYKGANKKGLSKGCFGLKLDRIGSMSGLGC;
   SEQ ID NO:41 (CNP-33):
NARKYKGANKKGLSKGCFGLKLDRIGSMSGLGC;
   SEQ ID NO:42 (CNP-32):
ARKYKGANKKGLSKGCFGLKLDRIGSMSGLGC;
   SEQ ID NO:43 (CNP-31):
RKYKGANKKGLSKGCFGLKLDRIGSMSGLGC;
   SEQ ID NO:44 (CNP-30):
KYKGANKKGLSKGCFGLKLDRIGSMSGLGC;
   SEQ ID NO:45 (CNP-29):
YKGANKKGLSKGCFGLKLDRIGSMSGLGC;
   SEQ ID NO:46 (CNP-28):
KGANKKGLSKGCFGLKLDRIGSMSGLGC;
   SEQ ID NO:47 (GHKSEVAHRF-CNP-28):
GHKSEVAHRFKGANKKGLSKGCFGLKLDRIGSMSGLGC;
   SEQ ID NO:48 (CNP-27):
GANKKGLSKGCFGLKLDRIGSMSGLGC;
   SEQ ID NO:49 (CNP-27 K4Q, K5Q):
GANQQGLSKGCFGLKLDRIGSMSGLGC;
   SEQ ID NO:50 (CNP-27 K4R,K5R):
GANRRGLSKGCFGLKLDRIGSMSGLGC;
   SEQ ID NO:51 (CNP-27 K4P,K5R):
GANPRGLSKGCFGLKLDRIGSMSGLGC;
   SEQ ID NO:52 (CNP-27 K4S,K5S):
GANSSGLSKGCFGLKLDRIGSMSGLGC;
   SEQ ID NO:53 (CNP-27 K4P,K5R):
GANGANPRGLSRGCFGLKLDRIGSMSGLGC;
   SEQ ID NO:54 (CNP-27 K4R, K5R, K9R):
GANRRGLSRGCFGLKLDRIGSMSGLGC;
   SEQ ID NO:55 (CNP-27 K4R, K5R, K9R, M22N):
GANRRGLSRGCFGLKLDRIGSNSGLGC;
   SEQ ID NO:56 (P-CNP-27 K4R, K5R, K9R):
PGANRRGLSRGCFGLKLDRIGSMSGLGC;
   SEQ ID NO:57 (M-CNP-27 K4R, K5R, K9R):
MGANRRGLSRGCFGLKLDRIGSMSGLGC;
   SEQ ID NO:58 (HSA fragment-CNP-27):
GHKSEVAHRFKGANKKGLSKGCFGLKLDRIGSMSGLG;
   SEQ ID NO:59 (HSA fragment-CNP-27 M22N):
GHKSEVAHRFKGANKKGLSKGCFGLKLDRIGSNSGLGC;
   SEQ ID NO:60 (M-HSA fragment-CNP-27):
MGHKSEVAHRFKGANKKGLSKGCFGLKLDRIGSMSGLGC;
   SEQ ID NO:61 (P-HSA fragment-CNP-27):
PGHKSEVAHRFKGANKKGLSKGCFGLKLDRIGSMSGLGC;
   SEQ ID NO:62 (CNP-26):
ANKKGLSKGCFGLKLDRIGSMSGLGC;
   SEQ ID NO:63 (CNP-25):
NKKGLSKGCFGLKLDRIGSMSGLGC;
   SEQ ID NO:64 (CNP-24):
KKGLSKGCFGLKLDRIGSMSGLGC;
   SEQ ID NO:65 (CNP-23):
KGLSKGCFGLKLDRIGSMSGLGC;
   SEQ ID NO:66 (R-CNP-22):
RGLSKGCFGLKLDRIGSMSGLGC;
   SEQ ID NO:67 (ER-CNP-22):
ERGLSKGCFGLKLDRIGSMSGLGC;
   SEQ ID NO:68 (R-CNP-22 K4R):
RGLSRGCFGLKLDRIGSMSGLGC;
   SEQ ID NO:69 (ER-CNP-22 4KR):
ERGLSRGCFGLKLDRIGSMSGLGC;
   SEQ ID NO:70 (RR-CNP-22):
RRGLSRGCFGLKLDRIGSMSGLGC;
   SEQ ID NO:71 (HRGP fragment-CNP-22):
GHHSHEQHPHGANQQGLSKGCFGLKLDRIGSMSGLGC;
   SEQ ID NO.72 (HRGP fragment-CNP-22):
GAHHPHEHDTHGANQQGLSKGCFGLKLDRIGSMSGLGC;
   SEQ ID NO:73 (HRGP fragment-CNP-22):
GHHSHEQHPHGANPRGLSKGCFGLKLDRIGSMSGLGC;
   SEQ ID NO:74 (IgG₁(F_{c}) fragment-CNP-22):
GQPREPQVYTLPPSGLSKGCFGLKLDRIGSMSGLGC;
   SEQ ID NO:75 (HSA fragment-CNP-22):
GQHKDDNPNLPRGANPRGLSKGCFGLKLDRIGSMSGLGC;
   SEQ ID NO:76 (HSA fragment-CNP-22):
GERAFKAWAVARLSQGLSKGCFGLKLDRIGSMSGLGC;
   SEQ ID NO:77 (osteocrin NPR C inhibitor fragment-CNP22):
FGIPMDRIGRNPRGLSKGCFGLKLDRIGSMSGLGC;
   SEQ ID NO:78 (FGF2 heparin-binding domain fragment-CNP22):
GKRTGQYKLGSKTGPGPKGLSKGCFGLKLDRIGSMSGLGC;
   SEQ ID NO:79 (IgG₁(F_{c}) fragment-CNP-22 K4R):
GQPREPQVYTGANQQGLSRGCFGLKLDRIGSMSGLGC;
   SEQ ID NO:80 (HSA fragment-CNP-22 K4R):
GVPQVSTSTGANQQGLSRGCFGLKLDRIGSMSGLGC;
   SEQ ID NO:81 (fibronectin fragment-CNP-22 K4R):
GQPSSSSQSTGANQQGLSRGCFGLKLDRIGSMSGLGC;
   SEQ ID NO:82 (fibronectin fragment-CNP-22 K4R):
GQTHSSGTQSGANQQGLSRGCFGLKLDRIGSMSGLGC;
   SEQ ID NO:83 (fibronectin fragment-CNP-22 K4R):
GSTGQWHSESGANQQGLSRGCFGLKLDRIGSMSGLGC;
   SEQ ID NO:84 (zinc finger fragment-CNP-22 K4R):
GSSSSSSSSSGANQQGLSRGCFGLKLDRIGSMSGLGC;
   SEQ ID NO:85 (CNP-21):
LSKGCFGLKLDRIGSMSGLGC;
   SEQ ID NO:86 (CNP-20):
SKGCFGLKLDRIGSMSGLGC;
   SEQ ID NO:87 (CNP-19):
KGCFGLKLDRIGSMSGLGC;
   SEQ ID NO:88 (CNP-18):
GCFGLKLDRIGSMSGLGC;
   SEQ ID NO:89 (CNP-17):
CFGLKLDRIGSMSGLGC;
   SEQ ID NO:90 (BNP fragment-CNP-17-BNP fragment):
SPKMVQGSGCFGLKLDRIGSMSGLGCKVLRRH;
   SEQ ID NO:91 (CNP-38 L1G):
GQEHPNARKYKGANKKGLSKGCFGLKLDRIGSMSGLGC;
   SEQ ID NO:92 (Ac-CNP-37; wherein Ac= acetyl):
Ac-QEHPNARKYKGANKKGLSKGCFGLKLDRIGSMSGLGC;

It is understood that the equivalents of the cysteines in positions 6 and 22 of SEQ ID NO:1 are also connected through a disulfide-bridge in SEQ ID NOs: 2 to 92.

More preferably, the term "CNP" refers to the sequence of SEQ ID:NOs 2, 19, 20, 21, 22, 23, 24, 25, 26, 30, 32, 38, 39, 40, 41, 42, 43, 91, 92. Even more preferably, the term "CNP" refers to the sequence of SEQ ID:NOs 23, 24, 25, 26, 38, 39, 91 and 92. In a particularly preferred embodiment the term "CNP" refers to the sequence of SEQ ID NO:24.

In a particularly preferred embodiment the term "CNP" refers to the sequence of SEQ ID NO:23, 24, 25 and 38, even more preferably to the sequence of SEQ ID NO:24 and 25 and most preferably to the sequence of SEQ ID NO:25. In an equally preferred embodiment the term "CNP" refers to the sequence of SEQ ID NO:24.

In another preferred embodiment the term "CNP" refers to a sequence of SEQ ID NO:93 QEHPNARX₁YX₂GANX₃X₄GLSX₅GCFGLX₆LDRIGSMSGLGC,
wherein X₁, X₂, X₃, X₄, X₅ and X₆ are independently of each other selected from the group consisting of K, R, P, S and Q, with the provision that at least one of X₁, X₂, X₃, X₄, X₅ and X₆ is selected from the group consisting of R, P, S and Q; preferably X₁, X₂, X₃, X₄, X₅ and X₆ are selected from the group consisting of K and R, with the provision that at least one of X₁, X₂, X₃, X₄, X₅ and X₆ is R;
even more preferably to a sequence of SEQ ID NO:94
   QEHPNARKYKGANX₁X₂GLSX₃GCFGLX₄LDRIGSMSGLGC,
wherein X₁, X₂, X₃ and X₄ are independently of each other selected from the group consisting of K, R, P, S and Q, with the provision that at least one of X₁, X₂, X₃ and X₄ is selected from the group consisting of R, P, S and Q; preferably X₁, X₂, X₃ and X₄ are selected from K and R, with the provision that at least one of X₁, X₂, X₃ and X₄ is R;
and most preferably to a sequence of SEQ ID NO:95
   QEHPNARKYKGANX₁X₂GLSKGCFGLKLDRIGSMSGLGC,
wherein X₁X₂ are selected from the group consisting of KR, RK, KP, PK, SS, RS, SR, QK, QR, KQ, RQ, RR and QQ.

It is understood that in all CNP sequences given in this specification the equivalents of the cysteines in positions 6 and 22 of SEQ ID NO:1 are also connected through a disulfide-bridge in SEQ ID NOs: 93 to 95.

It is understood that the present invention also encompasses CNP variants in which any one or more, up to all, residues susceptible to deamidation or a deamidation-like reaction (e.g., isomerization) may be converted to other residue(s) via deamidation or a deamidation-like reaction to any extent, up to 100% conversion per converted residue. In certain embodiments, the disclosure encompasses CNP variants in which:
(1) any one or more, up to all, asparagine (Asn/N) residues may be converted to aspartic acid or aspartate, and/or to isoaspartic acid or isoaspartate, via deamidation up to about 5%, 10%, 20%, 30%, 40%, 50%, 60%, 70%, 80%, 90% or 100% conversion per converted residue; or
(2) any one or more, up to all, glutamine (Gln/Q) residues may be converted to glutamic acid or glutamate, and/or to isoglutamic acid or isoglutamate, via deamidation up to about 5%, 10%, 20%, 30%, 40%, 50%, 60%, 70%, 80%, 90% or 100% conversion per converted residue; or
(3) any one or more, up to all, aspartic acid or aspartate (Asp/D) residues may be converted to isoaspartic acid or isoaspartate via a deamidation-like reaction (also called isomerization) up to about 5%, 10%, 20%, 30%, 40%, 50%, 60%, 70%, 80%, 90% or 100% conversion per converted residue; or
(4) any one or more, up to all, glutamic acid or glutamate (Glu/E) residues may be converted to isoglutamic acid or isoglutamate via a deamidation-like reaction (also called isomerization) up to about 5%, 10%, 20%, 30%, 40%, 50%, 60%, 70%, 80%, 90% or 100% conversion per converted residue;
(5) the N-terminal glutamine (if present) may be converted into pyroglutamate up to about 5%, 10%, 20%, 30%, 40%, 50%, 60%, 70%, 80%, 90% or 100% conversion; or
(5) a combination of the above.

As used herein, the term "CNP polypeptide variant" refers to a polypeptide from the same species that differs from a reference CNP polypeptide. Preferably, such reference CNP polypeptide sequence is the sequence of SEQ ID NO:1. In an equally preferred embodiment the reference CNP polypeptide sequence is the sequence of SEQ ID NO:24. Generally, differences are limited so that the amino acid sequence of the reference and the variant are closely similar overall and, in many regions, identical. Preferably, CNP polypeptide variants are at least 70%, 80%, 90%, or 95% identical to a reference CNP polypeptide, preferably the CNP polypeptide of SEQ ID NO:1.In an equally preferred embodiment the CNP polypeptide variants are at least 70%, 80%, 90%, or 95% identical to a reference CNP polypeptide, preferably the CNP polypeptide of SEQ ID NO:24. By a polypeptide having an amino acid sequence at least, for example, 95% "identical" to a query amino acid sequence, it is intended that the amino acid sequence of the subject polypeptide is identical to the query sequence except that the subject polypeptide sequence may include up to five amino acid alterations per each 100 amino acids of the query amino acid sequence. These alterations of the reference sequence may occur at the amino (N-terminal) or carboxy terminal (C-terminal) positions of the reference amino acid sequence or anywhere between those terminal positions, interspersed either individually among residues in the reference sequence or in one or more contiguous groups within the reference sequence. The query sequence may be an entire amino acid sequence of the reference sequence or any fragment specified as described herein. Preferably, the query sequence is the sequence of SEQ ID NO:1. In an equally preferred embodiment the query sequence is the sequence of SEQ ID NO:24.

Such CNP polypeptide variants may be naturally occurring variants, such as naturally occurring allelic variants encoded by one of several alternate forms of a CNP occupying a given locus on a chromosome or an organism, or isoforms encoded by naturally occurring splice variants originating from a single primary transcript. Alternatively, a CNP polypeptide variant may be a variant that is not known to occur naturally and that can be made mutagenesis techniques known in the art.

It is known in the art that one or more amino acids may be deleted from the N-terminus or C-terminus of a bioactive peptide or protein without substantial loss of biological function. Such N- and/or C-terminal deletions are also encompassed by the term CNP polypeptide variant.

It is also recognized by one of ordinary skill in the art that some amino acid sequences of CNP polypeptides can be varied without significant effect of the structure or function of the peptide. Such mutants include deletions, insertions, inversions, repeats, and substitutions selected according to general rules known in the art so as to have little effect on activity. For example, guidance concerning how to make phenotypically silent amino acid substitutions is provided in Bowie et al. (1990), Science 247:1306-1310, which is hereby incorporated by reference in its entirety, wherein the authors indicate that there are two main approaches for studying the tolerance of the amino acid sequence to change.

The term CNP polypeptide also encompasses all CNP polypeptides encoded by CNP analogs, orthologs, and/or species homologs. As used herein, the term "CNP analog" refers to CNP of different and unrelated organisms which perform the same functions in each organism but which did not originate from an ancestral structure that the organisms' ancestors had in common. Instead, analogous CNPs arose separately and then later evolved to perform the same or similar functions. In other words, analogous CNP polypeptides are polypeptides with quite different amino acid sequences but that perform the same biological activity, namely regulating the growth, proliferation and differentiation of cartilaginous growth plate chondrocytes.

As used herein the term "CNP ortholog" refers to CNP within two different species which sequences are related to each other via a common homologous CNP in an ancestral species, but which have evolved to become different from each other.

As used herein, the term "CNP homolog" refers to CNP of different organisms which perform the same functions in each organism and which originate from an ancestral structure that the organisms' ancestors had in common. In other words, homologous CNP polypeptides are polypeptides with quite similar amino acid sequences that perform the same biological activity, namely regulating the growth, proliferation and differentiation of cartilaginous growth plate chondrocytes. Preferably, CNP polypeptide homologs may be defined as polypeptides exhibiting at least 40%, 50%, 60%, 70%, 80%, 90% or 95% identity to a reference CNP polypeptide, preferably the CNP polypeptide of SEQ ID NO:1. In an equally preferred embodiment the reference CNP polypeptide is the CNP polypeptide of SEQ ID NO:24.

Thus, a CNP polypeptide according to the invention may be, for example: (i) one in which at least one of the amino acids residues is substituted with a conserved or non-conserved amino acid residue, preferably a conserved amino acid residue, and such substituted amino acid residue may or may not be one encoded by the genetic code; and/or (ii) one in which at least one of the amino acid residues includes a substituent group; and/or (iii) one in which the CNP polypeptide is fused with another compound, such as a compound to increase the half-life of the polypeptide (for example, polyethylene glycol); and/or (iv) one in which additional amino acids are fused to the CNP polypeptide, such as an IgG Fc fusion region peptide or leader or secretory sequence or a sequence which is employed for purification of the above form of the polypeptide or a pre-protein sequence.

As used herein, the term "CNP polypeptide fragment" refers to any peptide comprising a contiguous span of a part of the amino acid sequence of a CNP polypeptide, preferably the polypeptide of SEQ ID NO:1. In an equally preferred embodiment the term "CNP polypeptide fragment" refers to any peptide comprising a contiguous span of a part of the amino acid sequence of the polypeptide of SEQ ID NO:24.

More specifically, a CNP polypeptide fragment comprises at least 6, such as at least 8, at least 10 or at least 17 consecutive amino acids of a CNP polypeptide, more preferably of the polypeptide of SEQ ID NO:1. It is equally preferred that a CNP polypeptide fragment comprises at least 6, such as at least 8, at least 10 or at least 17 consecutive amino acids of the CNP polypeptide of SEQ ID NO:24. A CNP polypeptide fragment may additionally be described as sub-genuses of CNP polypeptides comprising at least 6 amino acids, wherein "at least 6" is defined as any integer between 6 and the integer representing the C-terminal amino acid of a CNP polypeptide, preferably of the polypeptide of SEQ ID No:1 or - equally preferred - of SEQ ID NO:24. Further included are species of CNP polypeptide fragments at least 6 amino acids in length, as described above, that are further specified in terms of their N-terminal and C-terminal positions. Also encompassed by the term "CNP polypeptide fragment" as individual species are all CNP polypeptide fragments, at least 6 amino acids in length, as described above, that may be particularly specified by a N-terminal and C-terminal position. That is, every combination of a N-terminal and C-terminal position that a fragment at least 6 contiguous amino acid residues in length could occupy, on any given amino acid sequence of a CNP polypeptide, preferably the CNP polypeptide of SEQ ID:NO1 or - equally preferred - of SEQ ID NO:24, is included in the present invention.

The term "CNP" also includes poly(amino acid) conjugates which have a sequence as described above, but having a backbone that comprises both amide and non-amide linkages, such as ester linkages, like for example depsipeptides. Depsipeptides are chains of amino acid residues in which the backbone comprises both amide (peptide) and ester bonds. Accordingly, the term "side chain" as used herein refers either to the moiety attached to the alpha-carbon of an amino acid moiety, if the amino acid moiety is connected through amine bonds such as in polypeptides, or to any carbon atom-comprising moiety attached to the backbone of a poly(amino acid) conjugate, such as for example in the case of depsipeptides. Preferably, the term "CNP" refers to polypeptides having a backbone formed through amide (peptide) bonds.

As the term CNP includes the above-described variants, analogs, orthologs, homologs, derivatives and fragments of CNP, all references to specific positions within a reference sequence also include the equivalent positions in variants, analogs, orthologs, homologs, derivatives and fragments of a CNP moiety, even if not specifically mentioned.

As used herein, the term "ring moiety" refers to the stretch of consecutive amino acid residues of the CNP drug or moiety that is located between two cysteine residues that form an intramolecular disulphide bridge or between homologous amino acid residues which are connected through a chemical linker. Preferably, the ring moiety is located between two cysteine residues that form an intramolecular disulphide bridge. These two cysteines correspond to the cysteines at position 22 and position 38 in the sequence of CNP-38 (SEQ ID NO:24). Accordingly, amino acids 23 to 37 are located in said ring moiety, if the CNP drug or moiety has the sequence of CNP-38.

Independently of the length of the CNP moiety, the sequence of the ring moiety of wild-type CNP is FGLKLDRIGSMSGLG (SEQ ID NO:96).

As described above, the term "CNP" relates to CNP drugs or moieties having different numbers of amino acids. The person skilled in the art understands that in CNP drugs or moieties of different lengths the positions of equivalent amino acids vary and the skilled artisan will have no difficulty identifying the two cysteines forming the disulphide bridge or their two homologous amino acid residues connected to each other through a chemical linker in longer, shorter and/or otherwise modified CNP versions.

As the term CNP includes the above-described variants, analogs, orthologs, homologs, derivatives and fragments of CNP, the term "ring moiety" also includes the corresponding variants, analogs, orthologs, homologs, derivatives and fragments of the sequence of SEQ ID NO:96. Accordingly, all references to specific positions within a reference sequence also include the equivalent positions in variants, analogs, orthologs, homologs, derivatives and fragments of a CNP moiety, even if not explicitly mentioned.

As used herein the term "pharmaceutical composition" refers to a composition containing one or more active ingredients, for example a drug or a prodrug, here specifically the CNP prodrugs of the present invention, and optionally one or more excipients, as well as any product which results, directly or indirectly, from combination, complexation or aggregation of any two or more of the ingredients of the composition, or from dissociation of one or more of the ingredients, or from other types of reactions or interactions of one or more of the ingredients.

Accordingly, the pharmaceutical compositions of the present invention encompass any composition made by admixing one or more CNP prodrugs of the present invention and optionally a pharmaceutically acceptable excipient.

As used herein the term "liquid composition" refers to a mixture comprising water-soluble CNP prodrug and one or more solvents, such as water.

The term "suspension composition" relates to a mixture comprising water-insoluble CNP prodrug, where for example the carrier Z' is a hydrogel, and one or more solvents, such as water. Due to the water-insoluble polymer, the polymeric prodrug cannot dissolve and renders the prodrug in a particulate state.

As used herein, the term "dry composition" means that a pharmaceutical composition is provided in a dry form. Suitable methods for drying are spray-drying and lyophilization, i.e. freeze-drying. Such dry composition of prodrug has a residual water content of a maximum of 10 %, preferably less than 5% and more preferably less than 2%, determined according to Karl Fischer. Preferably, the pharmaceutical composition of the present invention is dried by lyophilization.

The term "drug" as used herein refers to a substance used in the treatment, cure, prevention, or diagnosis of a disease or used to otherwise enhance physical or mental well-being. If a drug is conjugated to another moiety, the moiety of the resulting product that originated from the drug is referred to as "biologically active moiety".

As used herein the term "prodrug" refers to a biologically active moiety reversibly and covalently connected to a specialized protective group through a reversible prodrug linker moiety which is a linker moiety comprising a reversible linkage with the biologically active moiety and wherein the specialized protective group alters or eliminates undesirable properties in the parent molecule. This also includes the enhancement of desirable properties in the drug and the suppression of undesirable properties. The specialized non-toxic protective group is referred to as "carrier". A prodrug releases the reversibly and covalently bound biologically active moiety in the form of its corresponding drug. In other words, a prodrug is a conjugate comprising a biologically active moiety which is covalently and reversibly conjugated to a carrier moiety via a reversible prodrug linker moiety, which covalent and reversible conjugation of the carrier to the reversible prodrug linker moiety is either directly or through a spacer. Such conjugate releases the formerly conjugated biologically active moiety in the form of a free drug.

A "biodegradable linkage" or a "reversible linkage" is a linkage that is hydrolytically degradable, i.e. cleavable, in the absence of enzymes under physiological conditions (aqueous buffer at pH 7.4, 37°C) with a half-life ranging from one hour to six months, preferably from one hour to four months, even more preferably from one hour to three months, even more preferably from one hour to two months, even more preferably from one hour to one month. Accordingly, a stable linkage is a linkage having a half-life under physiological conditions (aqueous buffer at pH 7.4, 37°C) of more than six months.

Accordingly, a "reversible prodrug linker moiety" is a moiety which is covalently conjugated to a biologically active moiety, such as CNP, through a reversible linkage and is also covalently conjugated to a carrier moiety, such as -Z or -Z', wherein the covalent conjugation to said carrier moiety is either directly or through a spacer moiety, such as -L²-. Preferably the linkage between -Z or -Z' and -L²- is a stable linkage.

As used herein, the term "traceless prodrug linker" means a reversible prodrug linker which upon cleavage releases the drug in its free form. As used herein, the term "free form" of a drug means the drug in its unmodified, pharmacologically active form.

As used herein, the term "excipient" refers to a diluent, adjuvant, or vehicle with which the therapeutic, such as a drug or prodrug, is administered. Such pharmaceutical excipient can be sterile liquids, such as water and oils, including those of petroleum, animal, vegetable or synthetic origin, including but not limited to peanut oil, soybean oil, mineral oil, sesame oil and the like. Water is a preferred excipient when the pharmaceutical composition is administered orally. Saline and aqueous dextrose are preferred excipients when the pharmaceutical composition is administered intravenously. Saline solutions and aqueous dextrose and glycerol solutions are preferably employed as liquid excipients for injectable solutions. Suitable pharmaceutical excipients include starch, glucose, lactose, sucrose, mannitol, trehalose, gelatin, malt, rice, flour, chalk, silica gel, sodium stearate, glycerol monostearate, talc, sodium chloride, dried skim milk, glycerol, propylene, glycol, water, ethanol and the like. The pharmaceutical composition, if desired, can also contain minor amounts of wetting or emulsifying agents, pH buffering agents, like, for example, acetate, succinate, tris, carbonate, phosphate, HEPES (4-(2-hydroxyethyl)-1-piperazineethanesulfonic acid), MES (2-(*N*-morpholino)ethanesulfonic acid), or can contain detergents, like Tween, poloxamers, poloxamines, CHAPS, Igepal, or amino acids like, for example, glycine, lysine, or histidine. These pharmaceutical compositions can take the form of solutions, suspensions, emulsions, tablets, pills, capsules, powders, sustained-release formulations and the like. The pharmaceutical composition can be formulated as a suppository, with traditional binders and excipients such as triglycerides. Oral formulation can include standard excipients such as pharmaceutical grades of mannitol, lactose, starch, magnesium stearate, sodium saccharine, cellulose, magnesium carbonate, etc. Such compositions will contain a therapeutically effective amount of the drug or biologically active moiety, together with a suitable amount of excipient so as to provide the form for proper administration to the patient. The formulation should suit the mode of administration.

As used herein, the term "reagent" means a chemical compound which comprises at least one functional group for reaction with the functional group of another chemical compound or drug. It is understood that a drug comprising a functional group (such as a primary or secondary amine or hydroxyl functional group) is also a reagent.

As used herein, the term "moiety" means a part of a molecule, which lacks one or more atom(s) compared to the corresponding reagent. If, for example, a reagent of the formula "H-X-H" reacts with another reagent and becomes part of the reaction product, the corresponding moiety of the reaction product has the structure "H-X-" or "-X- " , whereas each "- " indicates attachment to another moiety. Accordingly, a biologically active moiety is released from a prodrug as a drug.

It is understood that if the sequence or chemical structure of a group of atoms is provided which group of atoms is attached to two moieties or is interrupting a moiety, said sequence or chemical structure can be attached to the two moieties in either orientation, unless explicitly stated otherwise. For example, a moiety "-C(O)N(R¹²)-" can be attached to two moieties or interrupting a moiety either as "-C(O)N(R¹²)-" or as "-N(R¹²)C(O)-". Similarly, a moiety 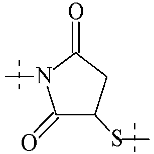 can be attached to two moieties or can interrupt a moiety either as

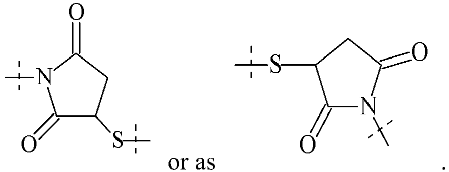

As used herein, the term "functional group" means a group of atoms which can react with other groups of atoms. Functional groups include but are not limited to the following groups: carboxylic acid (-(C=O)OH), primary or secondary amine (-NH₂, -NH-), maleimide, thiol (-SH), sulfonic acid (-(O=S=O)OH), carbonate, carbamate (-O(C=O)N<), hydroxyl (-OH), aldehyde (-(C=O)H), ketone (-(C=O)-), hydrazine (>N-N<), isocyanate, isothiocyanate, phosphoric acid (-O(P=O)OHOH), phosphonic acid (-O(P=O)OHH), haloacetyl, alkyl halide, acryloyl, aryl fluoride, hydroxylamine, disulfide, sulfonamides, sulfuric acid, vinyl sulfone, vinyl ketone, diazoalkane, oxirane, and aziridine.

In case the prodrugs of the present invention comprise one or more acidic or basic groups, the invention also comprises their corresponding pharmaceutically or toxicologically acceptable salts, in particular their pharmaceutically utilizable salts. Thus, the prodrugs of the present invention comprising acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Prodrugs of the present invention comprising one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. For the person skilled in the art further methods are known for converting the basic group into a cation like the alkylation of an amine group resulting in a positively-charge ammonium group and an appropriate counterion of the salt. If the prodrugs of the present invention simultaneously comprise acidic and basic groups, the invention also includes, in addition to the salt forms mentioned, inner salts or betaines (zwitterions). The respective salts can be obtained by customary methods which are known to the person skilled in the art like, for example by contacting these prodrugs with an organic or inorganic acid or base in a solvent or dispersant, or by anion exchange or cation exchange with other salts. The present invention also includes all salts of the prodrugs of the present invention which, owing to low physiological compatibility, are not directly suitable for use in pharmaceuticals but which can be used, for example, as intermediates for chemical reactions or for the preparation of pharmaceutically acceptable salts.

The term "pharmaceutically acceptable" means a substance that does cause harm when administered to a patient and preferably means approved by a regulatory agency, such as the EMA (Europe) and/or the FDA (US) and/or any other national regulatory agency for use in animals, preferably for use in humans.

As used herein the term "about" in combination with a numerical value is used to indicate a range ranging from and including the numerical value plus and minus no more than 10% of said numerical value, more preferably no more than 8% of said numerical value, even more preferably no more than 5% of said numerical value and most preferably no more than 2% of said numerical value. For example, the phrase "about 200" is used to mean a range ranging from and including 200 +/- 10%, i.e. ranging from and including 180 to 220; preferably 200 +/-8%, i.e. ranging from and including 184 to 216; even more preferably ranging from and including 200 +/-5%, i.e. ranging from and including 190 to 210; and most preferably 200 +/-2%, i.e. ranging from and including 196 to 204. It is understood that a percentage given as "about 20%" does not mean "20% +/- 10%", i.e. ranging from and including 10 to 30%, but "about 20%" means ranging from and including 18 to 22%, i.e. plus and minus 10% of the numerical value which is 20.

As used herein, the term "polymer" means a molecule comprising repeating structural units, i.e. the monomers, connected by chemical bonds in a linear, circular, branched, crosslinked or dendrimeric way or a combination thereof, which may be of synthetic or biological origin or a combination of both. It is understood that a polymer may also comprise one or more other chemical group(s) and/or moiety/moieties, such as, for example, one or more functional group(s). Preferably, a soluble polymer has a molecular weight of at least 0.5 kDa, e.g. a molecular weight of at least 1 kDa, a molecular weight of at least 2 kDa, a molecular weight of at least 3 kDa or a molecular weight of at least 5 kDa. If the polymer is soluble, it preferable has a molecular weight of at most 1000 kDa, such as at most 750 kDa, such as at most 500 kDa, such as at most 300 kDa, such as at most 200 kDa, such as at most 100 kDa. It is understood that for insoluble polymers, such as hydrogels, no meaningful molecular weight ranges can be provided.

As used herein, the term "polymeric" means a reagent or a moiety comprising one or more polymer(s) or polymer moiety/moieties. A polymeric reagent or moiety may optionally also comprise one or more other moiety/moieties, which are preferably selected from the group consisting of:
• C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
• linkages selected from the group comprising 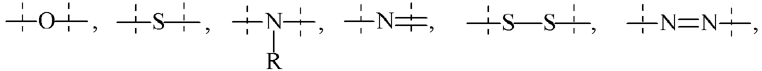 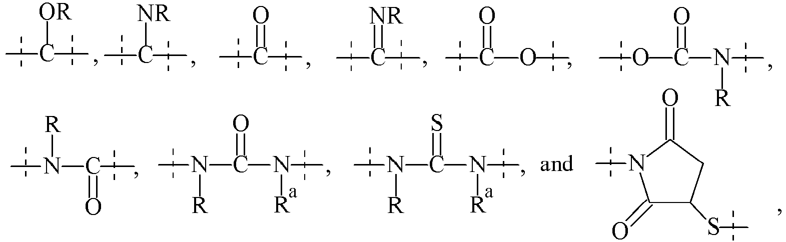 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

The person skilled in the art understands that the polymerization products obtained from a polymerization reaction do not all have the same molecular weight, but rather exhibit a molecular weight distribution. Consequently, the molecular weight ranges, molecular weights, ranges of numbers of monomers in a polymer and numbers of monomers in a polymer as used herein, refer to the number average molecular weight and number average of monomers, i.e. to the arithmetic mean of the molecular weight of the polymer or polymeric moiety and the arithmetic mean of the number of monomers of the polymer or polymeric moiety.

Accordingly, in a polymeric moiety comprising "x" monomer units any integer given for "x" therefore corresponds to the arithmetic mean number of monomers. Any range of integers given for "x" provides the range of integers in which the arithmetic mean numbers of monomers lies. An integer for "x" given as "about x" means that the arithmetic mean numbers of monomers lies in a range of integers of x +/- 10%, preferably x +/- 8%, more preferably x +/- 5% and most preferably x +/- 2%.

As used herein, the term "number average molecular weight" means the ordinary arithmetic mean of the molecular weights of the individual polymers.

As used herein the term "water-soluble" with reference to a carrier means that when such carrier is part of the CNP prodrug of the present invention at least 1 g of the CNP prodrug comprising such water-soluble carrier can be dissolved in one liter of water at 20°C to form a homogeneous solution. Accordingly, the term "water-insoluble" with reference to a carrier means that when such carrier is part of the CNP prodrug of the present invention less than 1 g of the CNP prodrug comprising such water-insoluble carrier can be dissolved in one liter of water at 20°C to form a homogeneous solution.

As used herein, the term "hydrogel" means a hydrophilic or amphiphilic polymeric network composed of homopolymers or copolymers, which is insoluble due to the presence of covalent chemical crosslinks. The crosslinks provide the network structure and physical integrity.

As used herein the term "thermogelling" means a compound that is a liquid or a low viscosity solution having a viscosity of less than 500 cps at 25°C at a shear rate of about 0.1 /second at a low temperature, which low temperature ranges between about 0°C to about 10°C, but which is a higher viscosity compound of less than 10000 cps at 25°C at a shear rate of about 0.1/second at a higher temperature, which higher temperature ranges between about 30°C to about 40°C, such as at about 37°C.

As used herein, the term "PEG-based" in relation to a moiety or reagent means that said moiety or reagent comprises PEG. Preferably, a PEG-based moiety or reagent comprises at least 10% (w/w) PEG, such as at least 20% (w/w) PEG, such as at least 30% (w/w) PEG, such as at least 40% (w/w) PEG, such as at least 50% (w/w), such as at least 60 (w/w) PEG, such as at least 70% (w/w) PEG, such as at least 80% (w/w) PEG, such as at least 90% (w/w) PEG, such as at least 95%. The remaining weight percentage of the PEG-based moiety or reagent are other moieties preferably selected from the following moieties and linkages:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 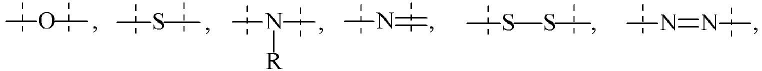 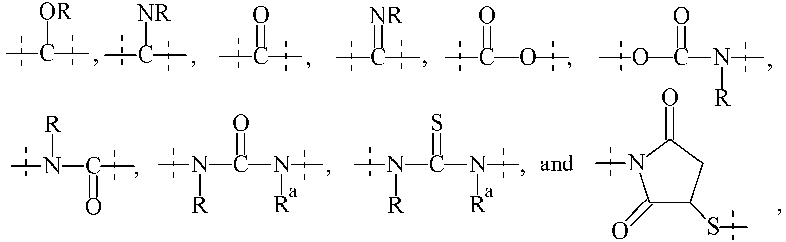 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

As used herein, the term "PEG-based comprising at least X% PEG" in relation to a moiety or reagent means that said moiety or reagent comprises at least X% (w/w) ethylene glycol units (-CH₂CH₂O-), wherein the ethylene glycol units may be arranged blockwise, alternating or may be randomly distributed within the moiety or reagent and preferably all ethylene glycol units of said moiety or reagent are present in one block; the remaining weight percentage of the PEG-based moiety or reagent are other moieties preferably selected from the following moieties and linkages:
- C₁₋₅₀ alkyl, C₂₋₅₀ alkenyl, C₂₋₅₀ alkynyl, C₃₋₁₀ cycloalkyl, 3- to 10-membered heterocyclyl, 8- to 11-membered heterobicyclyl, phenyl, naphthyl, indenyl, indanyl, and tetralinyl; and
- linkages selected from the group comprising 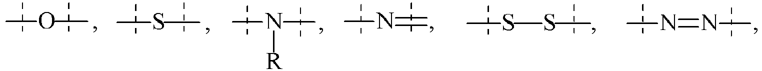 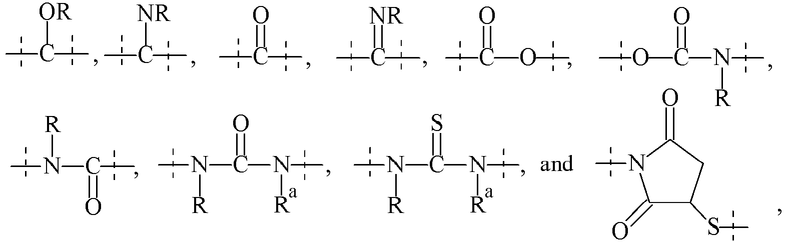 wherein
   dashed lines indicate attachment to the remainder of the moiety or reagent, and
   -R and -R^{a} are independently of each other selected from the group consisting of -H, methyl, ethyl, propyl, butyl, pentyl and hexyl.

The term "hyaluronic acid-based comprising at least X% hyaluronic acid" is used accordingly.

The term "substituted" as used herein means that one or more -H atom(s) of a molecule or moiety are replaced by a different atom or a group of atoms, which are referred to as "substituent".

Preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
-R^{x1}, -R^{x1a}, -R^{x1b} are independently of each other selected from the group consisting of -H, -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-; -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(O)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4}, -N(R^{x4}) C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x4b}), -OC(O)N(R^{x4}R^{x4a}), and C₁₋₆ alkyl; wherein C₁₋₆ alkyl is optionally substituted with one or more halogen, which are the same or different;
each -R^{x3}, -R^{x3a}, -R^{x4}, -R^{x4a}, -R^{x4b} is independently selected from the group consisting of -H and C₁₋₆ alkyl; wherein C₁₋₆ alkyl is optionally substituted with one or more halogen, which are the same or different.

More preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₁₀ alkyl, C₂₋₁₀ alkenyl, and C₂₋₁₀ alkynyl; wherein -T⁰, C₁₋₁₀ alkyl, C₂₋₁₀ alkenyl, and C₂₋₁₀ alkynyl are optionally substituted with one or more -R^{x2}, which are the same or different and wherein C₁₋₁₀ alkyl, C₂₋₁₀ alkenyl, and C₂₋₁₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each -R^{x1}, -R^{x1a}, -R^{x1b}, -R^{x3}, -R^{x3a} is independently selected from the group consisting of -H, halogen, C₁₋₆ alkyl, C₂₋₆ alkenyl, and C₂₋₆ alkynyl;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(O)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4}, -N(R^{x4}) C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x4b}), -OC(O)N(R^{x4}R^{x4a}), and C₁₋₆ alkyl; wherein C₁₋₆ alkyl is optionally substituted with one or more halogen, which are the same or different;
each -R^{x4}, -R^{x4a}, -R^{x4b} is independently selected from the group consisting of -H, halogen, C₁₋₆ alkyl, C₂₋₆ alkenyl, and C₂₋₆ alkynyl;

Even more preferably, the one or more further optional substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO₂, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₆ alkyl, C₂₋₆ alkenyl, and C₂₋₆ alkynyl; wherein -T⁰, C₁₋₆ alkyl, C₂₋₆ alkenyl, and C₂₋₆ alkynyl are optionally substituted with one or more -R^{x2}, which are the same or different and wherein C₁₋₆ alkyl, C₂₋₆ alkenyl, and C₂₋₆ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-, -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each -R^{x1}, -R^{x1a}, -R^{x1b}, -R^{x2}, -R^{x3}, -R^{x3a} is independently selected from the group consisting of -H, halogen, C₁₋₆ alkyl, C₂₋₆ alkenyl, and C₂₋₆ alkynyl;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different.

Preferably, a maximum of 6 -H atoms of an optionally substituted molecule are independently replaced by a substituent, e.g. 5 -H atoms are independently replaced by a substituent, 4 -H atoms are independently replaced by a substituent, 3 -H atoms are independently replaced by a substituent, 2 -H atoms are independently replaced by a substituent, or 1 -H atom is replaced by a substituent.

The term "interrupted" means that a moiety is inserted between two carbon atoms or - if the insertion is at one of the moiety's ends - between a carbon or heteroatom and a hydrogen atom, preferably between a carbon and a hydrogen atom.

As used herein, the term "C₁₋₄ alkyl" alone or in combination means a straight-chain or branched alkyl moiety having 1 to 4 carbon atoms. If present at the end of a molecule, examples of straight-chain or branched C₁₋₄ alkyl are methyl, ethyl, n-propyl, isopropyl, n-butyl, isobutyl, sec-butyl and tert-butyl. When two moieties of a molecule are linked by the C₁₋₄ alkyl, then examples for such C₁₋₄ alkyl groups are -CH₂-, -CH₂-CH₂-, -CH(CH₃)-, -CH₂-CH₂-CH₂-, -CH(C₂H₅)-, -C(CH₃)₂-. Each hydrogen of a C₁₋₄ alkyl carbon may optionally be replaced by a substituent as defined above. Optionally, a C₁₋₄ alkyl may be interrupted by one or more moieties as defined below.

As used herein, the term "C₁₋₆ alkyl" alone or in combination means a straight-chain or branched alkyl moiety having 1 to 6 carbon atoms. If present at the end of a molecule, examples of straight-chain and branched C₁₋₆ alkyl groups are methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpentyl, 3-methylpentyl, 2,2-dimethylbutyl, 2,3-dimethylbutyl and 3,3-dimethylpropyl. When two moieties of a molecule are linked by the C₁₋₆ alkyl group, then examples for such C₁₋₆ alkyl groups are -CH₂-, -CH₂-CH₂-, -CH(CH₃)-, -CH₂-CH₂-CH₂-, -CH(C₂H₅)- and -C(CH₃)₂-. Each hydrogen atom of a C₁₋₆ carbon may optionally be replaced by a substituent as defined above. Optionally, a C₁₋₆ alkyl may be interrupted by one or more moieties as defined below.

Accordingly, "C₁₋₁₀ alkyl", "C₁₋₂₀ alkyl" or "C₁₋₅₀ alkyl" means an alkyl chain having 1 to 10, 1 to 20 or 1 to 50 carbon atoms, respectively, wherein each hydrogen atom of the C₁₋₁₀, C₁₋₂₀ or C₁₋₅₀ carbon may optionally be replaced by a substituent as defined above. Optionally, a C₁₋₁₀ or C₁₋₅₀ alkyl may be interrupted by one or more moieties as defined below.

As used herein, the term "C₂₋₆ alkenyl" alone or in combination means a straight-chain or branched hydrocarbon moiety comprising at least one carbon-carbon double bond having 2 to 6 carbon atoms. If present at the end of a molecule, examples are -CH=CH₂, -CH=CH-CH₃, -CH₂-CH=CH₂, -CH=CHCH₂-CH₃ and -CH=CH-CH=CH₂. When two moieties of a molecule are linked by the C₂₋₆ alkenyl group, then an example for such C₂₋₆ alkenyl is -CH=CH-. Each hydrogen atom of a C₂₋₆ alkenyl moiety may optionally be replaced by a substituent as defined above. Optionally, a C₂₋₆ alkenyl may be interrupted by one or more moieties as defined below.

Accordingly, the term "C₂₋₁₀ alkenyl", "C₂₋₂₀ alkenyl" or "C₂₋₅₀ alkenyl" alone or in combination means a straight-chain or branched hydrocarbon moiety comprising at least one carbon-carbon double bond having 2 to 10, 2 to 20 or 2 to 50 carbon atoms. Each hydrogen atom of a C₂₋₁₀ alkenyl, C₂₋₂₀ alkenyl or C₂₋₅₀ alkenyl group may optionally be replaced by a substituent as defined above. Optionally, a C₂₋₁₀ alkenyl, C₂₋₂₀ alkenyl or C₂₋₅₀ alkenyl may be interrupted by one or more moieties as defined below.

As used herein, the term "C₂₋₆ alkynyl" alone or in combination means straight-chain or branched hydrocarbon moiety comprising at least one carbon-carbon triple bond having 2 to 6 carbon atoms. If present at the end of a molecule, examples are -C≡CH, -CH₂-C≡CH, CH₂-CH₂-C≡CH and CH₂-C≡C-CH₃. When two moieties of a molecule are linked by the alkynyl group, then an example is -C≡C-. Each hydrogen atom of a C₂₋₆ alkynyl group may optionally be replaced by a substituent as defined above. Optionally, one or more double bond(s) may occur. Optionally, a C₂₋₆ alkynyl may be interrupted by one or more moieties as defined below.

Accordingly, as used herein, the term "C₂₋₁₀ alkynyl", "C₂₋₂₀ alkynyl" and "C₂₋₅₀ alkynyl" alone or in combination means a straight-chain or branched hydrocarbon moiety comprising at least one carbon-carbon triple bond having 2 to 10, 2 to 20 or 2 to 50 carbon atoms, respectively. Each hydrogen atom of a C₂₋₁₀ alkynyl, C₂₋₂₀ alkynyl or C₂₋₅₀ alkynyl group may optionally be replaced by a substituent as defined above. Optionally, one or more double bond(s) may occur. Optionally, a C₂₋₁₀ alkynyl, C₂₋₂₀ alkynyl or C₂₋₅₀ alkynyl may be interrupted by one or more moieties as defined below.

As mentioned above, a C₁₋₄ alkyl, C₁₋₆ alkyl, C₁₋₁₀ alkyl, C₁₋₂₀ alkyl, C₁₋₅₀ alkyl, C₂₋₆ alkenyl, C₂₋₁₀ alkenyl, C₂₋₂₀ alkenyl, C₂₋₅₀ alkenyl, C₂₋₆ alkynyl, C₂₋₁₀ alkynyl, C₂₋₂₀ alkenyl or C₂₋₅₀ alkynyl may optionally be interrupted by one or more moieties which are preferably selected from the group consisting of 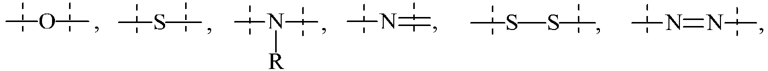 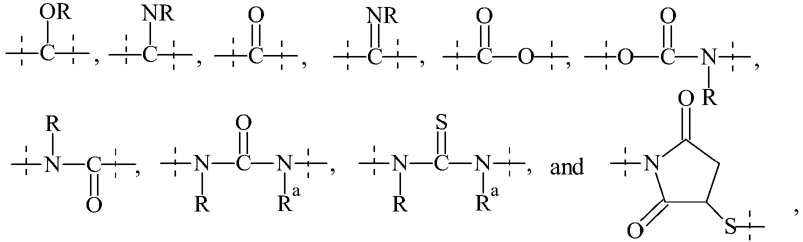 wherein
dashed lines indicate attachment to the remainder of the moiety or reagent; and
-R and -R^{a} are independently of each other selected from the group consisting of -H, methyl, ethyl, propyl, butyl, pentyl and hexyl.

As used herein, the term "C₃₋₁₀ cycloalkyl" means a cyclic alkyl chain having 3 to 10 carbon atoms, which may be saturated or unsaturated, e.g. cyclopropyl, cyclobutyl, cyclopentyl, cyclohexyl, cyclohexenyl, cycloheptyl, cyclooctyl, cyclononyl or cyclodecyl. Each hydrogen atom of a C₃₋₁₀ cycloalkyl carbon may be replaced by a substituent as defined above. The term "C₃₋₁₀ cycloalkyl" also includes bridged bicycles like norbornane or norbornene.

The term "8- to 30-membered carbopolycyclyl" or "8- to 30-membered carbopolycycle" means a cyclic moiety of two or more rings with 8 to 30 ring atoms, where two neighboring rings share at least one ring atom and that may contain up to the maximum number of double bonds (aromatic or non-aromatic ring which is fully, partially or un-saturated). Preferably a 8- to 30-membered carbopolycyclyl means a cyclic moiety of two, three, four or five rings, more preferably of two, three or four rings.

As used herein, the term "3- to 10-membered heterocyclyl" or "3- to 10-membered heterocycle" means a ring with 3, 4, 5, 6, 7, 8, 9 or 10 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom.

Examples for 3- to 10-membered heterocycles include but are not limited to aziridine, oxirane, thiirane, azirine, oxirene, thiirene, azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine and homopiperazine. Each hydrogen atom of a 3- to 10-membered heterocyclyl or 3- to 10-membered heterocyclic group may be replaced by a substituent as defined below.

As used herein, the term "8- to 11-membered heterobicyclyl" or "8- to 11-membered heterobicycle" means a heterocyclic moiety of two rings with 8 to 11 ring atoms, where at least one ring atom is shared by both rings and that may contain up to the maximum number of double bonds (aromatic or non-aromatic ring which is fully, partially or un-saturated) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for an 8- to 11-membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine and pteridine. The term 8- to 11-membered heterobicycle also includes spiro structures of two rings like 1,4-dioxa-8-azaspiro[4.5]decane or bridged heterocycles like 8-aza-bicyclo[3.2.1]octane. Each hydrogen atom of an 8- to 11-membered heterobicyclyl or 8- to 11-membered heterobicycle carbon may be replaced by a substituent as defined below.

Similary, the term "8- to 30-membered heteropolycyclyl" or "8- to 30-membered heteropolycycle" means a heterocyclic moiety of more than two rings with 8 to 30 ring atoms, preferably of three, four or five rings, where two neighboring rings share at least one ring atom and that may contain up to the maximum number of double bonds (aromatic or non-aromatic ring which is fully, partially or unsaturated), wherein at least one ring atom up to 10 ring atoms are replaced by a heteroatom selected from the group of sulfur (including -S(O)-, -S(O)₂-), oxygen and nitrogen (including =N(O)-) and wherein the ring is linked to the rest of a molecule via a carbon or nitrogen atom.

It is understood that the phrase "the pair R^{x}/R^{y} is joined together with the atom to which they are attached to form a C₃₋₁₀ cycloalkyl or a 3- to 10-membered heterocyclyl" in relation with a moiety of the structure 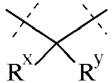 means that Rx and Ry form the following structure: 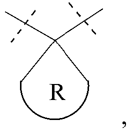 wherein R is C₃₋₁₀ cycloalkyl or 3- to 10-membered heterocyclyl.

It is also understood that the phrase "the pair R^{x}/R^{y} is joint together with the atoms to which they are attached to form a ring A" in relation with a moiety of the structure 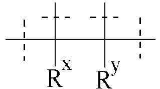 means that R^{x} and R^{y} form the following structure:

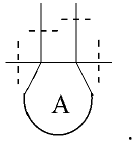

As used herein, the term "terminal alkyne" means a moiety

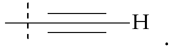

As used herein, "halogen" means fluoro, chloro, bromo or iodo. It is generally preferred that halogen is fluoro or chloro.

In general, the term "comprise" or "comprising" also encompasses "consist of" or "consisting of".

Preferably -D has the sequence of SEQ ID NO:24, SEQ ID NO:25 or SEQ ID NO:30, even more preferably of SEQ ID NO:24 and SEQ ID NO:25.

In one embodiment -D has the sequence of SEQ ID NO:25.

In another embodiment -D has the sequence of SEQ ID NO:30.

In a preferred embodiment -D has the sequence of SEQ ID NO:24.

The moiety -L¹- is a reversible prodrug linker from which the drug, i.e. CNP, is released in its free form, i.e. it is a traceless prodrug linker. Suitable prodrug linkers are known in the art, such as for example the reversible prodrug linker moieties disclosed in WO 2005/099768 A2, WO 2006/136586 A2, WO 2011/089216 A1 and WO 2013/024053 A1, which are incorporated by reference herewith.

In another embodiment -L¹- is a reversible prodrug linker as described in WO 2011/012722 A1, WO 2011/089214 A1, WO 2011/089215 A1, WO 2013/024052 A1 and WO 2013/160340 A1 which are incorporated by reference herewith.

The moiety -L¹- can be connected to -D through any type of linkage, provided that it is reversible. Preferably, -L¹- is connected to -D through a linkage selected from the group consisting of amide, ester, carbamate, acetal, aminal, imine, oxime, hydrazone, disulfide and acylguanidine. Even more preferably -L¹- is connected to -D through a linkage selected from the group consisting of amide, ester, carbamate and acylguanidin.

In a preferred embodiment, the moiety -L¹- is connected to -D through an amide linkage. It is understood that amide linkages generally are not reversible, but that in the present invention neighboring groups comprised in -L¹- render the amide linkage reversible.

A particularly preferred moiety -L¹- is disclosed in WO 2009/095479 A2. Accordingly, in one preferred embodiment the moiety -L¹- is of formula (II): 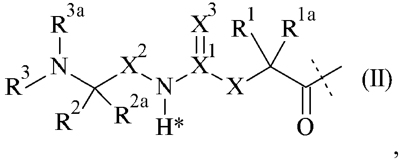 wherein the dashed line indicates the attachment to a nitrogen of -D which is a CNP moiety by forming an amide bond;
-X- is -C(R⁴R^{4a})-; -N(R⁴)-; -O-; -C(R⁴R^{4a})-C(R⁵R^{5a})-; -C(R⁵R^{5a})-C(R⁴R^{4a})-; -C(R⁴R^{4a})-N(R⁶)-; -N(R⁶)-C(R⁴R^{4a})-; -C(R⁴R^{4a})-O-; -O-C(R⁴R^{4a})-; or -C(R⁷R^{7a})-;
X¹ is C; or S(O);
-X²- is -C(R⁸R^{8a})-; or -C(R⁸R^{8a})-C(R⁹R^{9a})-;
   =X³ is =O; =S; or =N-CN;
-R¹, -R^{1a}, -R², -R^{2a}, -R⁴, -R^{4a}, -R⁵, -R^{5a}, -R⁶, -R⁸, -R^{8a}, -R⁹, -R^{9a} are independently selected from the group consisting of -H; and C₁₋₆ alkyl;
-R³, -R^{3a} are independently selected from the group consisting of -H; and C₁₋₆ alkyl, provided that in case one of -R³, -R^{3a} or both are other than -H they are connected to N to which they are attached through an SP³-hybridized carbon atom;
-R⁷ is -N(R¹⁰R^{10a}); or -NR¹⁰-(C=O)-R¹¹;
-R^{7a}, -R¹⁰, -R^{10a}, -R¹¹ are independently of each other -H; or C₁₋₆ alkyl;
optionally, one or more of the pairs -R^{1a}/-R^{4a}, -R^{1a}/-R^{5a}, -R^{1a}/-R^{7a}, -R^{4a}/-R^{5a}, -R^{8a}/-R^{9a} form a chemical bond;
optionally, one or more of the pairs -R¹/-R^{1a}, -R²/-R^{2a}, -R⁴/-R^{4a}, -R⁵/-R^{5a}, -R⁸/-R^{8a}, -R⁹/-R^{9a} are joined together with the atom to which they are attached to form a C₃₋₁₀ cycloalkyl; or 3- to 10-membered heterocyclyl;
optionally, one or more of the pairs -R¹/-R⁴, -R¹/-R⁵, -R¹/-R⁶, -R¹/-R^{7a}, -R⁴/-R⁵, -R⁴/-R⁶, -R⁸/-R⁹, -R²/-R³ are joined together with the atoms to which they are attached to form a ring A;
optionally, R³/R^{3a} are joined together with the nitrogen atom to which they are attached to form a 3- to 10-membered heterocycle;
A is selected from the group consisting of phenyl; naphthyl; indenyl; indanyl; tetralinyl; C₃₋₁₀ cycloalkyl; 3- to 10-membered heterocyclyl; and 8- to 11-membered heterobicyclyl; and
wherein -L¹- is substituted with -L²-Z or -L²-Z' and wherein -L¹- is optionally further substituted, provided that the hydrogen marked with the asterisk in formula (II) is not replaced by -L²-Z or -L²-Z' or a substituent;
   wherein
   - -L²-: is a single chemical bond or a spacer;
   - -Z: is a water-soluble carrier; and
   - -Z': is a water-insoluble carrier.

Preferably -L¹- of formula (II) is substituted with one moiety -L²-Z or -L²-Z'.

In one embodiment -L¹- of formula (II) is not further substituted.

It is understood that if -R³/-R^{3a} of formula (II) are joined together with the nitrogen atom to which they are attached to form a 3- to 10-membered heterocycle, only such 3- to 10-membered heterocycles may be formed in which the atoms directly attached to the nitrogen are SP³-hybridized carbon atoms. In other words, such 3- to 10-membered heterocycle formed by -R³/-R^{3a} together with the nitrogen atom to which they are attached has the following structure: 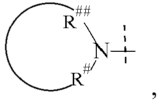 wherein
the dashed line indicates attachment to the rest of -L¹-;
the ring comprises 3 to 10 atoms comprising at least one nitrogen; and
R^{#} and R^{##} represent an SP³-hydridized carbon atom.

It is also understood that the 3- to 10-membered heterocycle may be further substituted.

Exemplary embodiments of suitable 3- to 10-membered heterocycles formed by -R³/-R^{3a} of formula (II) together with the nitrogen atom to which they are attached are the following: 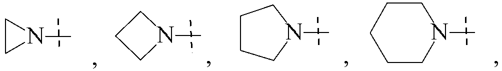 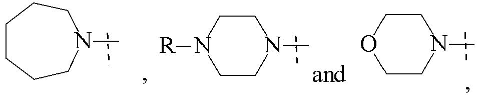 wherein
dashed lines indicate attachment to the rest of the molecule; and
-R is selected from the group consisting of -H and C₁₋₆ alkyl.

-L¹- of formula (II) may optionally be further substituted. In general, any substituent may be used as far as the cleavage principle is not affected, i.e. the hydrogen marked with the asterisk in formula (II) is not replaced and the nitrogen of the moiety 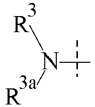 of formula (II) remains part of a primary, secondary or tertiary amine, i.e. -R³ and -R^{3a} are independently of each other -H or are connected to -N< through an SP³-hybridized carbon atom.

In one embodiment -R¹ or -R^{1a} of formula (II) is substituted with -L²-Z or -L²-Z'. In another embodiment -R² or -R^{2a} of formula (II) is substituted with -L²-Z or -L²-Z'. In another embodiment -R³ or -R^{3a} of formula (II) is substituted with -L²-Z or -L²-Z'. In another embodiment -R⁴ of formula (II) is substituted with -L²-Z or -L²-Z'. In another embodiment -R⁵ or -R^{5a} of formula (II) is substituted with -L²-Z or -L²-Z'. In another embodiment -R⁶ of formula (II) is substituted with -L²-Z or -L²-Z'. In another embodiment -R⁷ or -R^{7a} of formula (II) is substituted with -L²-Z or -L²-Z'. In another embodiment -R⁸ or -R^{8a} of formula (II) is substituted with -L²-Z or -L²-Z'. In another embodiment -R⁹ or -R^{9a} of formula (II) is substituted with -L²-Z or -L²-Z'.

Most preferably -R⁴ of formula (II) is substituted with -L²-Z or -L²-Z'.

Preferably, -X- of formula (II) is -C(R⁴R^{4a})- or -N(R⁴)-. Most preferably, -X- of formula (II) is -C(R⁴R^{4a})-.

Preferably, X¹ of formula (II) is C.

Preferably, =X³ of formula (II) is =O.

Preferably, -X²- of formula (II) is -C(R⁸R^{8a})-.

Preferably -R⁸ and -R^{8a} of formula (II) are independently selected from the group consisting of -H, methyl and ethyl. More preferably at least one of -R⁸ and -R^{8a} of formula (II) is -H. Even more preferably both -R⁸ and -R^{8a} of formula (II) are -H.

Preferably, -R¹ and -R^{1a} of formula (II) are independently selected from the group consisting of -H, methyl and ethyl. More preferably, at least one of -R¹ and -R^{1a} of formula (II) is -H. Even more preferably both -R¹ and -R^{1a} of formula (II) are -H.

Preferably, -R² and -R^{2a} of formula (II) are independently selected from the group consisting of -H, methyl and ethyl. More preferably, at least one of -R² and -R^{2a} of formula (II) is -H. Even more preferably both -R² and -R^{2a} of formula (II) are H.

Preferably, -R³ and -R^{3a} of formula (II) are independently selected from the group consisting of -H, methyl, ethyl, propyl and butyl. Even more preferably at least one of -R³ and -R^{3a} of formula (II) is methyl. In an equally preferred embodiment -R³ and -R^{3a} of formula (II) are both -H. In another equally preferred embodiment -R³ and -R^{3a} of formula (II) are both methyl.

Preferably, -R³ of formula (II) is -H and -R^{3a} of formula (II) is methyl.

Preferably, -R⁴ and -R^{4a} of formula (II) are independently selected from the group consisting of -H, methyl and ethyl. More preferably, at least one of -R⁴ and -R^{4a} of formula (II) is -H. Even more preferably both -R⁴ and -R^{4a} of formula (II) are -H.

Preferably the moiety -L¹- is of formula (IIa): 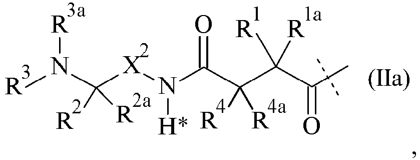
wherein the dashed line indicates the attachment to a nitrogen of -D which is a CNP moiety by forming an amide bond;
-R¹, -R^{1a}, -R², -R^{2a}, -R³, -R^{3a}, -R⁴, -R^{4a} and -X²- are used as defined in formula (II); and wherein -L¹- is substituted with -L²-Z or -L²-Z' and wherein -L¹- is optionally further substituted, provided that the hydrogen marked with the asterisk in formula (IIa) is not replaced by -L²-Z or -L²-Z' or a substituent.

Preferably -L¹- of formula (IIa) is substituted with one moiety -L²-Z or -L²-Z'.

Preferably the moiety -L¹- of formula (IIa) is not further substituted.

Preferably, -R¹ and -R^{1a} of formula (IIa) are independently selected from the group consisting of -H, methyl and ethyl. More preferably, at least one of -R¹ and -R^{1a} of formula (IIa) is -H. Even more preferably both -R¹ and -R^{1a} of formula (IIa) are -H.

Preferably, -R⁴ and -R^{4a} of formula (IIa) are independently selected from the group consisting of -H, methyl and ethyl. More preferably, at least one of -R⁴ and -R^{4a} of formula (IIa) is -H. Even more preferably both -R⁴ and -R^{4a} of formula (IIa) are -H.

Preferably, -X²- of formula (IIa) is -C(R⁸R^{8a})-.

Preferably -R⁸ and -R^{8a} of formula (IIa) are independently selected from the group consisting of -H, methyl and ethyl. More preferably at least one of -R⁸ and -R^{8a} of formula (IIa) is -H. Even more preferably both -R⁸ and -R^{8a} of formula (IIa) are -H.

Preferably, -R² and -R^{2a} of formula (IIa) are independently selected from the group consisting of -H, methyl and ethyl. More preferably, at least one of -R² and -R^{2a} of formula (IIa) is -H. Even more preferably both -R² and -R^{2a} of formula (IIa) are H.

Preferably, -R³ and -R^{3a} of formula (IIa) are independently selected from the group consisting of -H, methyl, ethyl, propyl and butyl. Even more preferably at least one of -R³ and -R^{3a} of formula (IIa) is methyl. In an equally preferred embodiment -R³ and -R^{3a} of formula (IIa) are both -H. In another equally preferred embodiment -R³ and -R^{3a} of formula (IIa) are both methyl.

Preferably, -R³ of formula (IIa) is -H and -R^{3a} of formula (IIa) is methyl.

Preferably the moiety -L¹- is of formula (IIb): 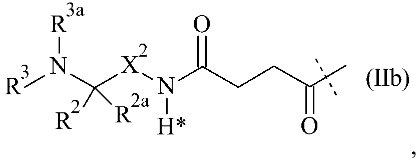
wherein the dashed line indicates the attachment to a nitrogen of -D which is a CNP moiety by forming an amide bond;
-R², -R^{2a}, -R³, -R^{3a} and -X²- are used as defined in formula (II); and
wherein -L¹- is substituted with -L²-Z or -L²-Z' and wherein -L¹- is optionally further substituted, provided that the hydrogen marked with the asterisk in formula (IIb) is not replaced by -L²-Z or -L²-Z' or a substituent.

Preferably -L¹- of formula (IIb) is substituted with one moiety -L²-Z or -L²-Z'.

Preferably the moiety -L¹- of formula (IIb) is not further substituted.

Preferably, -X²- of formula (IIb) is -C(R⁸R^{8a})-.

Preferably -R⁸ and -R^{8a} of formula (IIb) are independently selected from the group consisting of -H, methyl and ethyl. More preferably at least one of -R⁸ and -R^{8a} of formula (IIb) is -H. Even more preferably both -R⁸ and -R^{8a} of formula (IIb) are -H.

Preferably, -R² and -R^{2a} of formula (IIb) are independently selected from the group consisting of -H, methyl and ethyl. More preferably, at least one of -R² and -R^{2a} of formula (IIb) is -H. Even more preferably both -R² and -R^{2a} of formula (IIb) are H.

Preferably, -R³ and -R^{3a} of formula (IIb) are independently selected from the group consisting of -H, methyl, ethyl, propyl and butyl. Even more preferably at least one of -R³ and -R^{3a} of formula (IIb) is methyl. In an equally preferred embodiment -R³ and -R^{3a} of formula (IIb) are both -H. In another equally preferred embodiment -R³ and -R^{3a} of formula (IIb) are both methyl.

Most preferably, -R³ of formula (IIb) is -H and -R^{3a} of formula (IIb) is methyl.

Even more preferably the moiety -L¹- is of formula (IIb'): 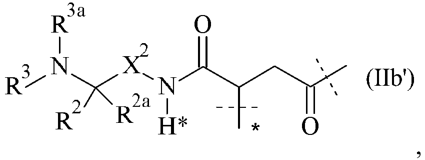 wherein
wherein the dashed line indicates the attachment to a nitrogen of D which is a CNP moiety by forming an amide bond;
the dashed line marked with the asterisk indicates attachment to -L²-;
-R², -R^{2a}, -R³, -R^{3a} and -X²- are used as defined in formula (II); and
wherein -L¹- is optionally further substituted, provided that the hydrogen marked with the asterisk in formula (IIb') is not replaced by a substituent.

Preferably the moiety -L¹- of formula (IIb') is not further substituted.

Preferably, -X²- of formula (IIb') is -C(R⁸R^{8a})-.

Preferably -R⁸ and -R^{8a} of formula (IIb') are independently selected from the group consisting of -H, methyl and ethyl. More preferably at least one of -R⁸ and -R^{8a} of formula (IIb') is -H. Even more preferably both -R⁸ and -R^{8a} of formula (IIb') are -H.

Preferably, -R² and -R^{2a} of formula (IIb') are independently selected from the group consisting of -H, methyl and ethyl. More preferably, at least one of -R² and -R^{2a} of formula (IIb') is -H. Even more preferably both -R² and -R^{2a} of formula (IIb') are H.

Preferably, -R³ and -R^{3a} of formula (IIb') are independently selected from the group consisting of -H, methyl, ethyl, propyl and butyl. Even more preferably at least one of -R³ and -R^{3a} of formula (IIb') is methyl. In an equally preferred embodiment -R³ and -R^{3a} of formula (IIb') are both -H. In another equally preferred embodiment -R³ and -R^{3a} of formula (IIb') are both methyl.

Most preferably, -R³ of formula (IIb') is -H and -R^{3a} of formula (IIb') is methyl.

Preferably the moiety -L¹- is of formula (IIc): 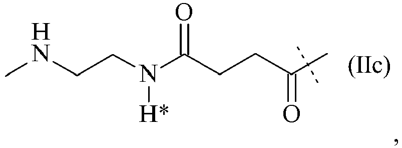
wherein the dashed line indicates the attachment to a nitrogen of -D which is a CNP moiety by forming an amide bond; and
wherein -L¹- is substituted with -L²-Z or -L²-Z' and wherein -L¹- is optionally further substituted, provided that the hydrogen marked with the asterisk in formula (IIc) is not replaced by -L²-Z or -L²-Z' or a substituent.

Preferably -L¹- of formula (IIc) is substituted with one moiety -L²-Z or -L²-Z'.

Preferably the moiety -L¹- of formula (IIc) is not further substituted.

In another preferred embodiment the moiety -L¹- is of formula (IIc-a): 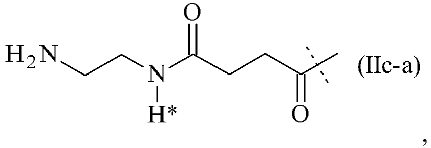
wherein the dashed line indicates the attachment to a nitrogen of -D which is a CNP moiety by forming an amide bond; and
wherein -L¹- is substituted with -L²-Z or -L²-Z' and wherein -L¹- is optionally further substituted, provided that the hydrogen marked with the asterisk in formula (IIc) is not replaced by -L²-Z or -L²-Z' or a substituent.

Preferably -L¹- of formula (IIc-a) is substituted with one moiety -L²-Z or -L²-Z'.

Preferably the moiety -L¹- of formula (IIc-a) is not further substituted.

In another preferred embodiment the moiety -L¹- is of formula (IIc-b): 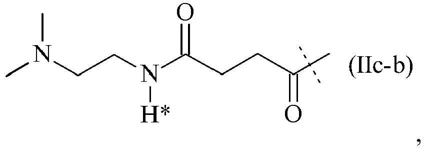
wherein the dashed line indicates the attachment to a nitrogen of -D which is a CNP moiety by forming an amide bond; and
wherein -L¹- is substituted with -L²-Z or -L²-Z' and wherein -L¹- is optionally further substituted, provided that the hydrogen marked with the asterisk in formula (IIc) is not replaced by -L²-Z or -L²-Z' or a substituent.

Preferably -L¹- of formula (IIc-b) is substituted with one moiety -L²-Z or -L²-Z'.

Preferably the moiety -L¹- of formula (IIc-b) is not further substituted.

Even more preferably the moiety -L¹- is selected from the group consisting of formula (IIc-i), (IIc-ii), (IIc-iii), (IIc-iv) and (IIc-v): 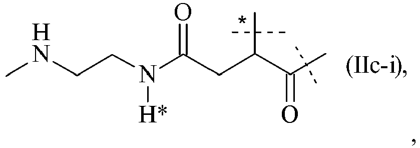 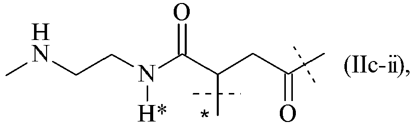 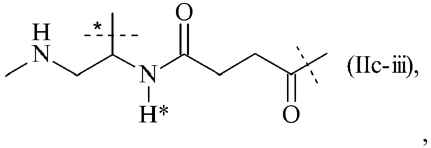 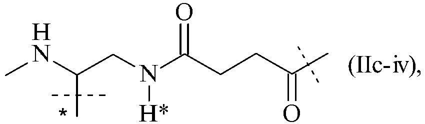 and 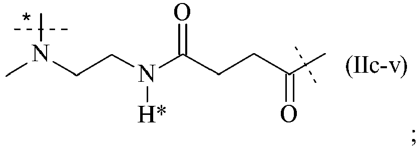 wherein
the unmarked dashed line indicates the attachment to a nitrogen of -D which is a CNP moiety by forming an amide bond; and
the dashed line marked with the asterisk indicates attachment to -L²-Z or -L²-Z'; and -L¹- is optionally further substituted, provided that the hydrogen marked with the asterisk in formula (IIc-i), (IIc-ii), (IIc-iii), (IIc-iv) and (IIc-v) is not replaced by a substituent.

Preferably, the moiety -L¹- of formula (IIc-i), (IIc-ii), (IIc-iii), (IIc-iv) and (IIc-v) is not further substituted.

In a particularly preferred embodiment the moiety -L¹- is 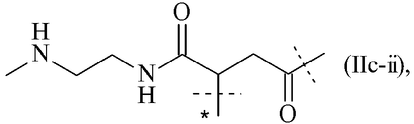 wherein
the unmarked dashed line indicates the attachment to a nitrogen of -D which is a CNP moiety by forming an amide bond; and
the dashed line marked with the asterisk indicates attachment to -L²-Z or -L²-Z'.

Preferably -L¹- of formula (IIc-ii) is substituted with one moiety -L²-Z or -L²-Z'.

In an equally preferred embodiment the moiety -L¹- is selected from the group consisting of formula (IIc-i'), (IIc-ii'), (IIc-iii'), (IIc-iv') and (IIc-v'): 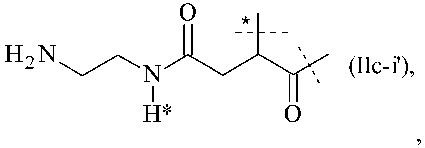 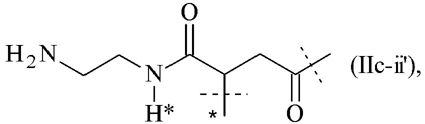 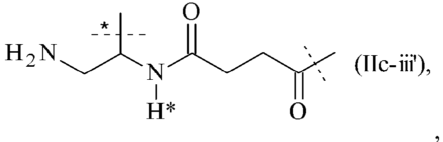 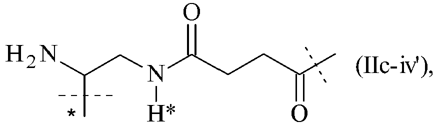 and 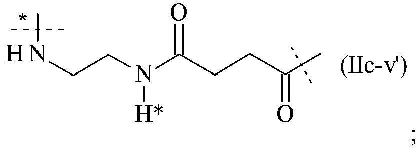 wherein
the unmarked dashed line indicates the attachment to a nitrogen of -D which is a CNP moiety by forming an amide bond; and
the dashed line marked with the asterisk indicates attachment to -L²-Z or -L²-Z'; and -L¹- is optionally further substituted, provided that the hydrogen marked with the asterisk in formula (IIc-i'), (IIc-ii'), (IIc-iii'), (IIc-iv') and (IIc-v') is not replaced by a substituent.

Preferably, the moiety -L¹- of formula (IIc-i'), (IIc-ii'), (IIc-iii'), (IIc-iv') and (IIc-v') is not further substituted.

In another particularly preferred embodiment the moiety -L¹- is 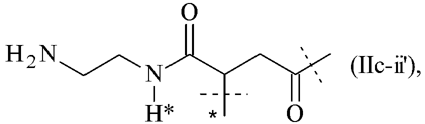 wherein
the unmarked dashed line indicates the attachment to a nitrogen of -D which is a CNP moiety by forming an amide bond; and
the dashed line marked with the asterisk indicates attachment to -L²-Z or -L²-Z'.

Preferably -L¹- of formula (IIc-ii') is substituted with one moiety -L²-Z or -L²-Z'.

In an equally preferred embodiment the moiety -L¹- is selected from the group consisting of formula (IIc-i''), (IIc-ii"), (IIc-iii'') and (IIc-iv''): 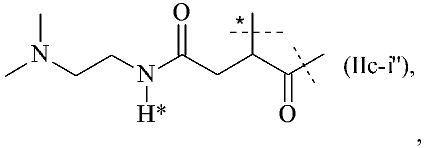 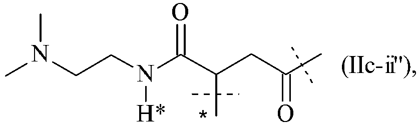 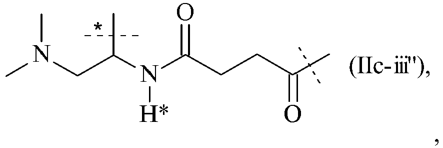 and 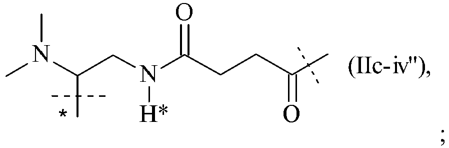 wherein
the unmarked dashed line indicates the attachment to a nitrogen of -D which is a CNP moiety by forming an amide bond; and
the dashed line marked with the asterisk indicates attachment to -L²-Z or -L²-Z'; and -L¹- is optionally further substituted, provided that the hydrogen marked with the asterisk in formula (IIc-i''), (IIc-ii''), (IIc-iii'') and (IIc-iv'') is not replaced by a substituent.

Preferably, the moiety -L¹- of formula (IIc-i''), (IIc-ii''), (IIc-iii'') and (IIc-iv'') is not further substituted.

In another particularly preferred embodiment the moiety -L¹- is 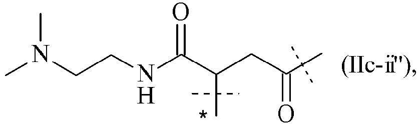 wherein
the unmarked dashed line indicates the attachment to a nitrogen of -D which is a CNP moiety by forming an amide bond; and
the dashed line marked with the asterisk indicates attachment to -L²-Z or -L²-Z'.

Preferably -L¹- of formula (IIc-ii'') is substituted with one moiety -L²-Z or -L²-Z'.

The optional further substituents of -L¹- of formula (II), (IIa), (IIb), (IIb'), (IIc), (IIc-i), (IIc-ii), (IIc-iii), (IIc-iv), (IIc-v), (IIc-i'), (IIc-ii'), (IIc-iii'), (IIc-iv'), (IIc-v'), (IIc-i''), (IIc-ii''), (IIc-iii₎ and (IIc-iv'') are preferably as described above.

Another particularly preferred moiety -L¹- is disclosed in unpublished European patent application 14180004, which corresponds to the international application with the application number PCT/EP2015/067929. Accordingly, in another preferred embodiment the moiety -L¹- is of formula (III): 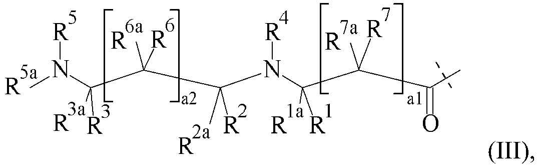 wherein
the dashed line indicates attachment to a primary or secondary amine or hydroxyl of D by forming an amide or ester linkage, respectively;
-R¹, -R^{1a}, -R², -R^{2a}, -R³ and -R^{3a} are independently of each other selected from the group consisting
   of -H, -C(R⁸R^{8a}R^{8b}), -C(=O)R⁸, -C≡N, -C(=NR⁸)R^{8a}, -CR⁸(=CR^{8a}R^{8b}), -C≡CR⁸ and -T;
-R⁴, -R⁵ and -R^{5a} are independently of each other selected from the group consisting of -H, -C(R⁹R^{9a}R^{9b}) and -T;
a1 and a2 are independently of each other 0 or 1;
each -R⁶, -R^{6a}, -R⁷, -R^{7a}, -R⁸, -R^{8a}, -R^{8b}, -R⁹, -R^{9a}, -R^{9b} are independently of each other selected from the group consisting of -H, halogen, -CN, -COOR¹⁰, -OR¹⁰, -C(O)R¹⁰, -C(O)N(R¹⁰R^{10a}), -S(O)₂N(R¹⁰R^{10a}), -S(O)N(R¹⁰R^{10a}), -S(O)₂R¹⁰, -S(O)R¹⁰, -N(R¹⁰)S(O)₂N(R^{10a}R^{10b}), -SR¹⁰, -N(R¹⁰R^{10a}), -NO₂, -OC(O)R¹⁰, -N(R¹⁰)C(O)R^{10a}, -N(R¹⁰)S(O)₂R^{10a}, -N(R¹⁰)S(O)R^{10a}, -N(R¹⁰)C(O)OR^{10a}, -N(R¹⁰)C(O)N(R^{10a}R^{10b}), -OC(O)N(R¹⁰R^{10a}), -T, C₁₋₂₀ alkyl, C₂₋₂₀ alkenyl, and C₂₋₂₀ alkynyl; wherein -T, C₁₋₂₀ alkyl, C₂₋₂₀ alkenyl, and C₂₋₂₀ alkynyl are optionally substituted with one or more -R¹¹, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R¹²)-, -S(O)₂N(R¹²)-, -S(O)N(R¹²)-, -S(O)₂-, -S(O)-, -N(R¹²)S(O)₂N(R^{12a})-, -S-, -N(R¹²)-, -OC(OR¹²)(R^{12a})-, -N(R¹²)C(O)N(R^{12a})-, and -OC(O)N(R¹²)-;
each -R¹⁰, -R^{10a}, -R^{10b} is independently selected from the group consisting of -H, -T, C₁₋₂₀ alkyl, C₂₋₂₀ alkenyl, and C₂₋₂₀ alkynyl; wherein -T, C₁₋₂₀ alkyl, C₂₋₂₀ alkenyl, and C₂₋₂₀ alkynyl are optionally substituted with one or more -R¹¹, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R¹²)-, -S(O)₂N(R¹²)-, -S(O)N(R¹²)-, -S(O)₂-, -S(O)-, -N(R¹²)S(O)₂N(R^{12a})-, -S-, -N(R¹²)-, -OC(OR¹²)(R^{12a})-, -N(R¹²)C(O)N(R^{12a})-, and -OC(O)N(R¹²)-;
each T is independently of each other selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T is independently optionally substituted with one or more -R¹¹, which are the same or different;
each -R¹¹ is independently of each other selected from halogen, -CN, oxo (=O), -COOR¹³, -OR¹³, -C(O)R¹³, -C(O)N(R¹³R^{13a}), -S(O)₂N(R¹³R^{13a}), -S(O)N(R¹³R^{13a}), -S(O)₂R¹³, -S(O)R¹³, -N(R¹³)S(O)₂N(R^{13a}R^{13b}), -SR¹³, -N(R¹³R^{13a}), -NO₂, -OC(O)R¹³, -N(R¹³)C(O)R^{13a}, -N(R¹³)S(O)₂R^{13a}, -N(R¹³)S(O)R^{13a}, -N(R¹³)C(O)OR^{13a}, -N(R¹³)C(O)N(R^{13a}R^{13b}), -OC(O)N(R¹³R^{13a}), and C₁₋₆ alkyl; wherein C₁₋₆ alkyl is optionally substituted with one or more halogen, which are the same or different;
each -R¹², -R^{12a}, -R¹³, -R^{13a}, -R^{13b} is independently selected from the group consisting of -H, and C₁₋₆ alkyl; wherein C₁₋₆ alkyl is optionally substituted with one or more halogen, which are the same or different;
optionally, one or more of the pairs -R¹/-R^{1a}, -R²/-R^{2a}, -R³/-R^{3a}, -R⁶/-R^{6a}, -R⁷/-R^{7a} are joined together with the atom to which they are attached to form a C₃₋₁₀ cycloalkyl or a 3- to 10-membered heterocyclyl;
optionally, one or more of the pairs -R¹/-R², -R¹/-R³, -R¹/-R⁴, -R¹/-R⁵, -R¹/-R⁶, -R¹/-R⁷, -R²/-R³, -R²/-R⁴, -R²/-R⁵, -R²/-R⁶, -R²/-R⁷, -R³/-R⁴, -R³/-R⁵, -R³/-R⁶, -R³/-R⁷, -R⁴/-R⁵, -R⁴/-R⁶, -R⁴/-R⁷, -R⁵/-R⁶, -R⁵/-R⁷, -R⁶/-R⁷ are joint together with the atoms to which they are attached to form a ring A;
A is selected from the group consisting of phenyl; naphthyl; indenyl; indanyl; tetralinyl; C₃₋₁₀ cycloalkyl; 3- to 10-membered heterocyclyl; and 8- to 11-membered heterobicyclyl;
wherein -L¹- is substituted with -L²-Z or -L²-Z' and wherein -L¹- is optionally further substituted;
   wherein
   - -L²-: is a single chemical bond or a spacer;
   - -Z: is a water-soluble carrier; and
   - -Z': is a water-insoluble carrier.

The optional further substituents of -L¹- of formula (III) are preferably as described above.

Preferably -L¹- of formula (III) is substituted with one moiety -L²-Z or -L²-Z'.

In one embodiment -L¹- of formula (III) is not further substituted.

Additional preferred embodiments for -L¹- are disclosed in EP1536334B1, WO2009/009712A1, WO2008/034122A1, WO2009/143412A2, WO2011/082368A2, and US8618124B2, which are herewith incorporated by reference in their entirety.

Additional preferred embodiments for -L¹- are disclosed in US8946405B2 and US8754190B2, which are herewith incorporated by reference in their entirety. Accordingly, a preferred moiety -L¹- is of formula (IV): 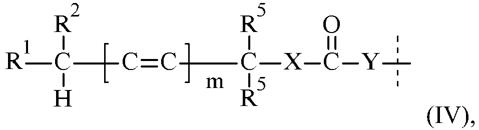 wherein
the dashed line indicates attachment to -D which is a CNP moiety and wherein attachment is through a functional group of -D selected from the group consisting of -OH, -SH and -NH₂;
m is 0 or 1;
at least one or both of -R¹ and -R² is/are independently of each other selected from the group consisting of -CN, -NO₂, optionally substituted aryl, optionally substituted heteroaryl, optionally substituted alkenyl, optionally substituted alkynyl, -C(O)R³, -S(O)R³, -S(O)₂R³, and -SR⁴,
one and only one of -R¹ and -R² is selected from the group consisting of -H, optionally substituted alkyl, optionally substituted arylalkyl, and optionally substituted heteroarylalkyl;
-R³ is selected from the group consisting of -H, optionally substituted alkyl, optionally substituted aryl, optionally substituted arylalkyl, optionally substituted heteroaryl, optionally substituted heteroarylalkyl, -OR⁹ and -N(R⁹)₂;
-R⁴ is selected from the group consisting of optionally substituted alkyl, optionally substituted aryl, optionally substituted arylalkyl, optionally substituted heteroaryl, and optionally substituted heteroarylalkyl;
each -R⁵ is independently selected from the group consisting of -H, optionally substituted alkyl, optionally substituted alkenylalkyl, optionally substituted alkynylalkyl, optionally substituted aryl, optionally substituted arylalkyl, optionally substituted heteroaryl and optionally substituted heteroarylalkyl;
-R⁹ is selected from the group consisting of -H and optionally substituted alkyl;
-Y- is absent and -X- is -O- or -S-; or
-Y- is -N(Q)CH₂- and -X- is -O-;
Q is selected from the group consisting of optionally substituted alkyl, optionally substituted aryl, optionally substituted arylalkyl, optionally substituted heteroaryl and optionally substituted heteroarylalkyl;
optionally, -R¹ and -R² may be joined to form a 3 to 8-membered ring; and
optionally, both -R⁹ together with the nitrogen to which they are attached form a heterocyclic ring;
wherein -L¹- is substituted with -L²-Z or -L²-Z' and wherein -L¹- is optionally further substituted;
   wherein
   - -L²-: is a single chemical bond or a spacer;
   - -Z: is a water-soluble carrier; and
   - -Z': is a water-insoluble carrier.

Only in the context of formula (IV) the terms used have the following meaning:
The term "alkyl" as used herein includes linear, branched or cyclic saturated hydrocarbon groups of 1 to 8 carbons, or in some embodiments 1 to 6 or 1 to 4 carbon atoms.

The term "alkoxy" includes alkyl groups bonded to oxygen, including methoxy, ethoxy, isopropoxy, cyclopropoxy, cyclobutoxy, and similar.

The term "alkenyl" includes non-aromatic unsaturated hydrocarbons with carbon-carbon double bonds.

The term "alkynyl" includes non-aromatic unsaturated hydrocarbons with carbon-carbon triple bonds.

The term "aryl" includes aromatic hydrocarbon groups of 6 to 18 carbons, preferably 6 to 10 carbons, including groups such as phenyl, naphthyl, and anthracenyl. The term "heteroaryl" includes aromatic rings comprising 3 to 15 carbons containing at least one N, O or S atom, preferably 3 to 7 carbons containing at least one N, O or S atom, including groups such as pyrrolyl, pyridyl, pyrimidinyl, imidazolyl, oxazolyl, isoxazolyl, thiazolyl, isothiazolyl, quinolyl, indolyl, indenyl, and similar.

In some instance, alkenyl, alkynyl, aryl or heteroaryl moieties may be coupled to the remainder of the molecule through an alkylene linkage. Under those circumstances, the substituent will be referred to as alkenylalkyl, alkynylalkyl, arylalkyl or heteroarylalkyl, indicating that an alkylene moiety is between the alkenyl, alkynyl, aryl or heteroaryl moiety and the molecule to which the alkenyl, alkynyl, aryl or heteroaryl is coupled.

The term "halogen" includes bromo, fluoro, chloro and iodo.

The term "heterocyclic ring" refers to a 4 to 8 membered aromatic or non-aromatic ring comprising 3 to 7 carbon atoms and at least one N, O, or S atom. Examples are piperidinyl, piperazinyl, tetrahydropyranyl, pyrrolidine, and tetrahydrofuranyl, as well as the exemplary groups provided for the term "heteroaryl" above.

When a ring system is optionally substituted, suitable substituents are selected from the group consisting of alkyl, alkenyl, alkynyl, or an additional ring, each optionally further substituted. Optional substituents on any group, including the above, include halo, nitro, cyano, -OR, -SR, -NR₂, -OCOR, -NRCOR, -COOR, -CONR₂, -SOR, -SO₂R, -SONR₂, -SO₂N R₂, wherein each R is independently alkyl, alkenyl, alkynyl, aryl or heteroaryl, or two R groups taken together with the atoms to which they are attached form a ring.

Preferably -L¹- of formula (IV) is substituted with one moiety -L²-Z or -L²-Z'.

An additional preferred embodiment for -L¹- is disclosed in WO2013/036857A1, which is herewith incorporated by reference in its entirety. Accordingly, a preferred moiety -L¹- is of formula (V): 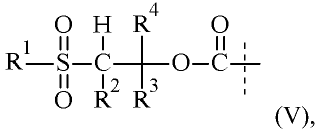 wherein
the dashed line indicates attachment to -D which is a CNP moiety and wherein attachment is through an amine functional group of -D;
   - -R¹: is selected from the group consisting of optionally substituted C₁-C₆ linear, branched, or cyclic alkyl; optionally substituted aryl; optionally substituted heteroaryl; alkoxy; and -NR⁵₂;
   - -R²: is selected from the group consisting of -H; optionally substituted C₁-C₆ alkyl; optionally substituted aryl; and optionally substituted heteroaryl;
   - -R³: is selected from the group consisting of -H; optionally substituted C₁-C₆ alkyl; optionally substituted aryl; and optionally substituted heteroaryl;
   - -R⁴ is: selected from the group consisting of -H; optionally substituted C₁-C₆ alkyl; optionally substituted aryl; and optionally substituted heteroaryl;
   - each -R⁵: is independently of each other selected from the group consisting of -H; optionally substituted C₁-C₆ alkyl; optionally substituted aryl; and optionally substituted heteroaryl; or when taken together two -R⁵ can be cycloalkyl or cycloheteroalkyl;
wherein -L¹- is substituted with -L²-Z or -L²-Z' and wherein -L¹- is optionally further substituted;
   wherein
   - -L²-: is a single chemical bond or a spacer;
   - -Z: is a water-soluble carrier; and
   - -Z': is a water-insoluble carrier.

Only in the context of formula (V) the terms used have the following meaning:
"Alkyl", "alkenyl", and "alkynyl" include linear, branched or cyclic hydrocarbon groups of 1-8 carbons or 1-6 carbons or 1-4 carbons wherein alkyl is a saturated hydrocarbon, alkenyl includes one or more carbon-carbon double bonds and alkynyl includes one or more carbon-carbon triple bonds. Unless otherwise specified these contain 1-6 C.

"Aryl" includes aromatic hydrocarbon groups of 6-18 carbons, preferably 6-10 carbons, including groups such as phenyl, naphthyl, and anthracene "Heteroaryl" includes aromatic rings comprising 3-15 carbons containing at least one N, O or S atom, preferably 3-7 carbons containing at least one N, O or S atom, including groups such as pyrrolyl, pyridyl, pyrimidinyl, imidazolyl, oxazolyl, isoxazolyl, thiszolyl, isothiazolyl, quinolyl, indolyl, indenyl, and similar.

The term "substituted" means an alkyl, alkenyl, alkynyl, aryl, or heteroaryl group comprising one or more substituent groups in place of one or more hydrogen atoms. Substituents may generally be selected from halogen including F, Cl, Br, and I; lower alkyl including linear, branched, and cyclic; lower haloalkyl including fluoroalkyl, chloroalkyl, bromoalkyl, and iodoalkyl; OH; lower alkoxy including linear, branched, and cyclic; SH; lower alkylthio including linear, branched and cyclic; amino, alkylamino, dialkylamino, silyl including alkylsilyl, alkoxysilyl, and arylsilyl; nitro; cyano; carbonyl; carboxylic acid, carboxylic ester, carboxylic amide, aminocarbonyl; aminoacyl; carbamate; urea; thiocarbamate; thiourea; ketne; sulfone; sulfonamide; aryl including phenyl, naphthyl, and anthracenyl; heteroaryl including 5-member heteroaryls including as pyrrole, imidazole, furan, thiophene, oxazole, thiazole, isoxazole, isothiazole, thiadiazole, triazole, oxadiazole, and tetrazole, 6-member heteroaryls including pyridine, pyrimidine, pyrazine, and fused heteroaryls including benzofuran, benzothiophene, benzoxazole, benzimidazole, indole, benzothiazole, benzisoxazole, and benzisothiazole.

Preferably -L¹- of formula (V) is substituted with one moiety -L²-Z or -L²-Z'.

A further preferred embodiment for -L¹- is disclosed in US7585837B2, which is herewith incorporated by reference in its entirety. Accordingly, a preferred moiety -L¹- is of formula (VI): 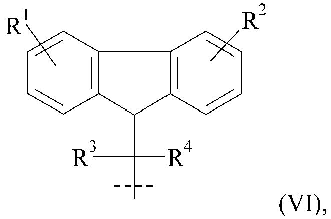 wherein
the dashed line indicates attachment to -D which is a CNP moiety and wherein attachment is through an amine functional group of -D;
R¹ and R² are independently selected from the group consisting of hydrogen, alkyl, alkoxy, alkoxyalkyl, aryl, alkaryl, aralkyl, halogen, nitro, -SO₃H, -SO₂NHR⁵, amino, ammonium, carboxyl, PO₃H₂, and OPO₃H₂;
R³, R⁴, and R⁵ are independently selected from the group consisting of hydrogen, alkyl, and aryl;
wherein -L¹- is substituted with -L²-Z or -L²-Z' and wherein -L¹- is optionally further substituted;
   wherein
   - -L²-: is a single chemical bond or a spacer;
   - -Z: is a water-soluble carrier; and
   - -Z': is a water-insoluble carrier.

Suitable substituents for formulas (VI) are alkyl (such as C₁₋₆ alkyl), alkenyl (such as C₂₋₆ alkenyl), alkynyl (such as C₂₋₆ alkynyl), aryl (such as phenyl), heteroalkyl, heteroalkenyl, heteroalkynyl, heteroaryl (such as aromatic 4 to 7 membered heterocycle) or halogen moieties.

Only in the context of formula (VI) the terms used have the following meaning:
The terms "alkyl", "alkoxy", "alkoxyalkyl", "aryl", "alkaryl" and "aralkyl" mean alkyl radicals of 1-8, preferably 1-4 carbon atoms, e.g. methyl, ethyl, propyl, isopropyl and butyl, and aryl radicals of 6-10 carbon atoms, e.g. phenyl and naphthyl. The term "halogen" includes bromo, fluoro, chloro and iodo.

Preferably -L¹- of formula (VI) is substituted with one moiety -L²-Z or -L²-Z'.

A further preferred embodiment for -L¹- is disclosed in WO2002/089789A1, which is herewith incorporated by reference in its entirety. Accordingly, a preferred moiety -L¹- is of formula (VII): 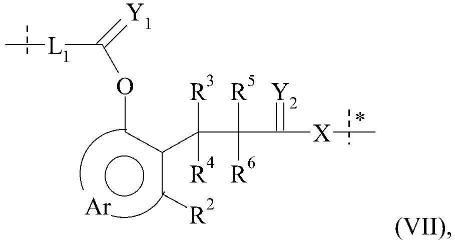 wherein
the dashed line indicates attachment to -D which is a CNP moiety and wherein attachment is through an amine functional group of -D;
L₁ is a bifunctional linking group,
Y₁ and Y₂ are independently O, S or NR⁷;
R², R³, R⁴, R⁵, R⁶ and R⁷ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy, and C₁₋₆ heteroalkoxy;
Ar is a moiety which when included in formula (VII) forms a multisubstituted aromatic hydrocarbon or a multi-substituted heterocyclic group;
X is a chemical bond or a moiety that is actively transported into a target cell, a hydrophobic moiety, or a combination thereof,
y is 0 or 1;
wherein -L¹- is substituted with -L²-Z or -L²-Z' and wherein -L¹- is optionally further substituted;
   wherein
   - -L²-: is a single chemical bond or a spacer;
   - -Z: is a water-soluble carrier; and
   - -Z': is a water-insoluble carrier.

Only in the context of formula (VII) the terms used have the following meaning:
The term "alkyl" shall be understood to include, e.g. straight, branched, substituted C₁₋₁₂ alkyls, including alkoxy, C₃₋₈ cycloalkyls or substituted cycloalkyls, etc.

The term "substituted" shall be understood to include adding or replacing one or more atoms contained within a functional group or compounds with one or more different atoms.

Substituted alkyls include carboxyalkyls, aminoalkyls, dialkylaminos, hydroxyalkyls and mercaptoalkyls; substtued cycloalkyls include moieties such as 4-chlorocyclohexyl; aryls include moieties such as napthyl; substituted aryls include moieties such as 3-bromo-phenyl; aralkyls include moieties such as toluyl; heteroalkyls include moieties such as ethylthiophene; substituted heteroalkyls include moieties such as 3-methoxythiophone; alkoxy includes moieities such as methoxy; and phenoxy includes moieties such as 3-nitrophenoxy. Halo- shall be understood to include fluoro, chloro, iodo and bromo.

Preferably -L¹- of formula (VII) is substituted with one moiety -L²-Z or -L²-Z'.

In another preferred embodiment -L¹- comprises a substructure of formula (VIII) 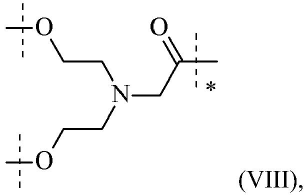 wherein
the dashed line marked with the asterisk indicates attachment to a nitrogen of -D which is a CNP moiety by forming an amide bond;
the unmarked dashed lines indicate attachment to the remainder of -L¹-; and wherein -L¹- is substituted with -L²-Z or -L²-Z' and wherein -L¹- is optionally further substituted;
   wherein
   - -L²-: is a single chemical bond or a spacer;
   - -Z: is a water-soluble carrier; and
   - -Z': is a water-insoluble carrier.

Preferably -L¹- of formula (VIII) is substituted with one moiety -L²-Z or -L²-Z'.

In one embodiment -L¹- of formula (VIII) is not further substituted.

In another preferred embodiment -L¹- comprises a substructure of formula (IX) 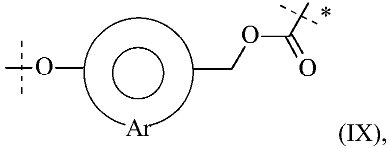 wherein
the dashed line marked with the asterisk indicates attachment to a nitrogen of -D which is a CNP moiety by forming a carbamate bond;
the unmarked dashed lines indicate attachment to the remainder of -L¹-; and wherein -L¹- is substituted with -L²-Z or -L²-Z' and wherein -L¹- is optionally further substituted;
   wherein
   - -L²-: is a single chemical bond or a spacer;
   - -Z: is a water-soluble carrier; and
   - -Z': is a water-insoluble carrier.

Preferably -L¹- of formula (IX) is substituted with one moiety -L²-Z or -L²-Z'.

In one embodiment -L¹- of formula (IX) is not further substituted.

The moiety -L¹- may be connected to -D through any functional group of -D and is preferably connected to -D through an amine functional group of -D. This may be the N-terminal amine functional group or an amine functional group provided by a lysine side chain, i.e. by the lysine at position 4 or 10, if the CNP has the sequence of SEQ ID NO: 1; by the lysines at position 7, 9, 13, 14, 18 and 24, if the CNP has the sequence of SEQ ID NO:38; by the lysines at position 8, 10, 14, 15, 19 or 25, if the CNP has the sequence of SEQ ID NO:25; by the lysines at position 9, 11, 15, 16, 20 and 26, if the CNP has the sequence of SEQ ID NO:24; and by the lysines at position 10, 12, 16, 17, 21 and 27, if the CNP moiety is of SEQ ID NO:23.

In one embodiment the CNP moiety is connected to -L¹- through the N-terminal amine functional group of the CNP moiety.

In another embodiment the CNP moiety is connected to -L¹- through the amine functional group provided by the side chain of the lysine at position 4, if the CNP moiety has the sequence of SEQ ID NO:1.

In another embodiment the CNP moiety is connected to -L¹- through the amine functional group provided by the side chain of the lysine at position 10, if the CNP moiety has the sequence of SEQ ID NO:1.

In another embodiment the CNP moiety is connected to -L¹- through the amine functional group provided by the side chain of the lysine at position 8, if the CNP moiety has the sequence of SEQ ID NO:25.

In another embodiment the CNP moiety is connected to -L¹- through the amine functional group provided by the side chain of the lysine at position 10, if the CNP moiety has the sequence of SEQ ID NO:25.

In another embodiment the CNP moiety is connected to -L¹- through the amine functional group provided by the side chain of the lysine at position 14, if the CNP moiety has the sequence of SEQ ID NO:25.

In another embodiment the CNP moiety is connected to -L¹- through the amine functional group provided by the side chain of the lysine at position 15, if the CNP moiety has the sequence of SEQ ID NO:25.

In another embodiment the CNP moiety is connected to -L¹- through the amine functional group provided by the side chain of the lysine at position 19, if the CNP moiety has the sequence of SEQ ID NO:25.

In another embodiment the CNP moiety is connected to -L¹- through the amine functional group provided by the side chain of the lysine at position 25, if the CNP moiety has the sequence of SEQ ID NO:25.

In another embodiment the CNP moiety is connected to -L¹- through the amine functional group provided by the side chain of the lysine at position 9, if the CNP moiety has the sequence of SEQ ID NO:24.

In another embodiment the CNP moiety is connected to -L¹- through the amine functional group provided by the side chain of the lysine at position 11, if the CNP moiety has the sequence of SEQ ID NO:24.

In another embodiment the CNP moiety is connected to -L¹- through the amine functional group provided by the side chain of the lysine at position 15, if the CNP moiety has the sequence of SEQ ID NO:24.

In another embodiment the CNP moiety is connected to -L¹- through the amine functional group provided by the side chain of the lysine at position 16, if the CNP moiety has the sequence of SEQ ID NO:24.

In another embodiment the CNP moiety is connected to -L¹- through the amine functional group provided by the side chain of the lysine at position 20, if the CNP moiety has the sequence of SEQ ID NO:24.

In another embodiment the CNP moiety is connected to -L¹- through the amine functional group provided by the side chain of the lysine at position 26, if the CNP moiety has the sequence of SEQ ID NO:24.

Most preferably the CNP moiety has the sequence of SEQ ID NO:24 and is connected to -L¹- through the amine functional group provided by the side chain of the lysine at position 26.

It was surprisingly found that attachment of -L¹- to the ring of CNP significantly reduces the CNP prodrug's affinity to NPR-B compared to attachment at the N-terminus or to the non-ring part of CNP which reduced affinity to NPR-B in turn reduces the risk of cardiovascular side effects, such as hypotension.

Accordingly, -L¹- is preferably conjugated to the side chain of an amino acid residue of said ring moiety of -D or to the backbone of said ring moiety of -D. Even more preferably, -L¹- is covalently and reversibly conjugated to the side chain of an amino acid residue of said ring moiety of -D.

Said amino acid residue located in the ring moiety of -D is preferably any amino acid having a functional group.

Preferably, the amino acid residue of the ring moiety of -D to which -L¹- is conjugated comprises a functional group selected from the group consisting of carboxylic acid, primary and secondary amine, maleimide, thiol, sulfonic acid, carbonate, carbamate, hydroxyl, aldehyde, ketone, hydrazine, isocyanate, isothiocyanate, phosphoric acid, phosphonic acid, haloacetyl, alkyl halide, acryloyl, aryl fluoride, hydroxylamine, sulfate, disulfide, vinyl sulfone, vinyl ketone, diazoalkane, oxirane, guanidine and aziridine. Most preferably the amino acid residue of the ring moiety of -D to which -L¹- is conjugated comprises a functional group selected from the group consisting hydroxyl, primary and secondary amine and guanidine.

The moiety -L¹- may be connected to -D through any type of linkage, provided that it is reversible. Preferably, -L¹- is connected to -D through a linkage selected from the group consisting of amide, ester, carbamate, acetal, aminal, imine, oxime, hydrazone, disulfide and acylguanidine. Even more preferably -L¹- is connected to -D through a linkage selected from the group consisting of amide, ester, carbamate and acylguanidine.

In one embodiment -L¹- is connected to -D through an ester linkage.

In another embodiment -L¹- is connected to -D through a carbamate linkage.

In another embodiment -L¹- is connected to -D through an acylguanidine.

In a preferred embodiment -L¹- is connected to -D through an amide linkage.

The amino acid residue of the ring moiety of -D to which -L¹- is conjugated is selected from the group consisting of proteinogenic amino acid residues and non-proteinogenic amino acid residues.

In one embodiment the amino acid residue of the ring moiety of -D to which -L¹- is conjugated is a non-proteinogenic amino acid.

In a preferred embodiment the amino acid residue of the ring moiety of -D to which -L¹- is conjugated is a proteinogenic amino acid. Even more preferably said amino acid is selected from the group consisting of histidine, lysine, tryptophan, serine, threonine, tyrosine, aspartic acid, glutamic acid and arginine. Even more preferably said amino acid is selected from the group consisting of lysine, aspartic acid, arginine and serine. Even more preferably said amino acid is selected from the group consisting of lysine, arginine and serine.

In one embodiment the amino acid residue of the ring moiety of -D to which -L¹- is conjugated is a histidine. It is understood that such histidine does not occur in the sequence of SEQ ID NO:96 and that it may only be present in variants, analogs, orthologs, homologs and derivatives thereof.

In one embodiment the amino acid residue of the ring moiety of -D to which -L¹- is conjugated is a tryptophan. It is understood that such tryptophan does not occur in the sequence of SEQ ID NO:96 and that it may only be present in variants, analogs, orthologs, homologs and derivatives thereof.

In one embodiment the amino acid residue of the ring moiety of -D to which -L¹- is conjugated is a threonine. It is understood that such threonine does not occur in the sequence of SEQ ID NO:96 and that it may only be present in variants, analogs, orthologs, homologs and derivatives thereof.

In one embodiment the amino acid residue of the ring moiety of -D to which -L¹- is conjugated is a tyrosine. It is understood that such tyrosine does not occur in the sequence of SEQ ID NO:96 and that it may only be present in variants, analogs, orthologs, homologs and derivatives thereof.

In one embodiment the amino acid residue of the ring moiety of -D to which -L¹- is conjugated is a glutamic acid. It is understood that such glutamic acid does not occur in the sequence of SEQ ID NO:96 and that it may only be present in variants, analogs, orthologs, homologs and derivatives thereof.

In one embodiment the amino acid residue of the ring moiety of -D to which -L¹- is conjugated is a lysine. Preferably, said amino acid is the lysine at position 4 of SEQ ID NO:96 which corresponds to the lysine at position 26 of SEQ ID NO:24.

In another embodiment the amino acid residue of the ring moiety of -D to which -L¹- is conjugated is an aspartic acid. Preferably, said amino acid is the aspartic acid at position 6 of SEQ ID NO:96 which corresponds to the aspartic acid at position 28 of SEQ ID NO:24.

In another embodiment the amino acid residue of the ring moiety of -D to which -L¹- is conjugated is an arginine. Preferably, said amino acid is the arginine at position 7 of SEQ ID NO:96 which corresponds to the arginine at position 29 of SEQ ID NO:24.

In another embodiment the amino acid residue of the ring moiety of -D to which -L¹- is conjugated a serine. Preferably, said amino acid is the serine at position 10 or 12 of SEQ ID NO:96. In one embodiment said amino acid is the serine at position 10 of SEQ ID NO:96 which corresponds to the serine at position 32 of SEQ ID NO:24. In another embodiment said amino acid is the serine at position 12 of SEQ ID NO:96 which corresponds to the serine at position 34 of SEQ ID NO:24.

In a preferred embodiment the amino acid residue of the ring moiety of -D to which -L¹- is conjugated is a lysine. Most preferably, -D has the sequence of SEQ ID NO:24 and -L¹- is conjugated to the lysine at position 26.

It was also surprisingly found that an increase in the lengths of the CNP sequence is beneficial with regard to NEP-stability: CNP-22 was more susceptible towards NEP-degradation than CNP-34 which in turn was more susceptible than CNP-38.

In the prodrugs of the present invention -L²- is a chemical bond or a spacer moiety.

In one embodiment -L²- is a chemical bond.

In another embodiment -L²- is a spacer moiety.

When -L²- is other than a single chemical bond, -L²- is preferably selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₅₀ alkyl, C₂₋₅₀ alkenyl, and C₂₋₅₀ alkynyl; wherein -T, C₁₋₅₀ alkyl, C₂₋₅₀ alkenyl, and C₂₋₅₀ alkynyl are optionally substituted with one or more -R^{y2}, which are the same or different, and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
each -R^{y2} is independently selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently selected from the group consisting of -H, and C₁₋₆ alkyl, wherein C₁₋₆ alkyl is optionally substituted with one or more halogen, which are the same or different.

When -L²- is other than a single chemical bond, -L²- is even more preferably selected from -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₂₀ alkyl, C₂₋₂₀ alkenyl, and C₂₋₂₀ alkynyl are optionally substituted with one or more -R^{y2}, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently of each other selected from the group consisting of -H, -T, C₁₋₁₀ alkyl, C₂₋₁₀ alkenyl, and C₂₋₁₀ alkynyl; wherein -T, C₁₋₁₀ alkyl, C₂₋₁₀ alkenyl, and C₂₋₁₀ alkynyl are optionally substituted with one or more -R^{y2}, which are the same or different, and wherein C₁₋₁₀ alkyl, C₂₋₁₀ alkenyl, and C₂₋₁₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
-R^{y2} is selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5}) C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

When -L²- is other than a single chemical bond, -L²- is even more preferably selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-, -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
-R^{y1} and -R^{y1a} are independently selected from the group consisting of -H, -T, C₁₋₁₀ alkyl, C₂₋₁₀ alkenyl, and C₂₋₁₀ alkynyl;
each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl;
each -R^{y2} is independently selected from the group consisting of halogen, and C₁₋₆ alkyl; and
each -R^{y3}, -R^{y3a}, -R^{y4}, -R^{y4a}, -R^{y5}, -R^{y5a} and -R^{y5b} is independently of each other selected from the group consisting of -H, and C₁₋₆ alkyl; wherein C₁₋₆ alkyl is optionally substituted with one or more halogen, which are the same or different.

Even more preferably, -L²- is a C₁₋₂₀ alkyl chain, which is optionally interrupted by one or more groups independently selected from -O-, -T- and -C(O)N(R^{y1})-; and which C₁₋₂₀ alkyl chain is optionally substituted with one or more groups independently selected from -OH, -T and -C(O)N(R^{y6}R^{y6a}); wherein -R^{y1}, -R^{y6}, -R^{y6a} are independently selected from the group consisting of H and C₁₋₄ alkyl and wherein T is selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl.

Preferably, -L²- has a molecular weight in the range of from 14 g/mol to 750 g/mol.

Preferably, -L²- comprises a moiety selected from 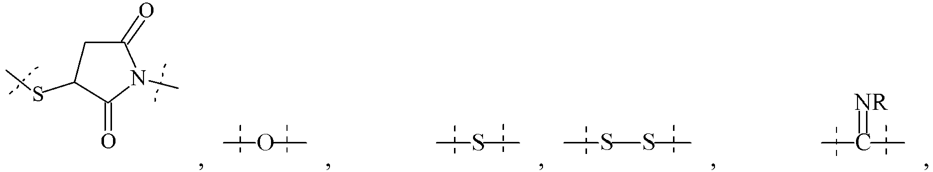 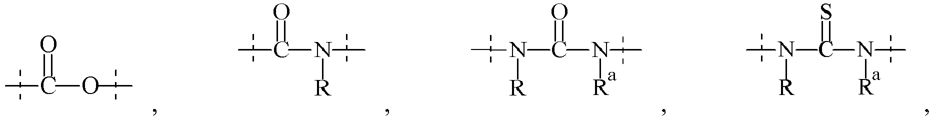 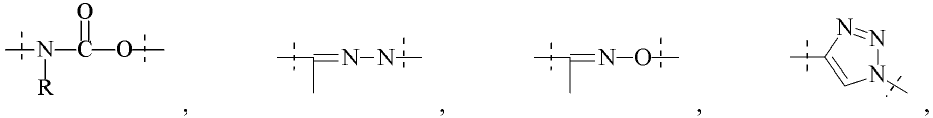 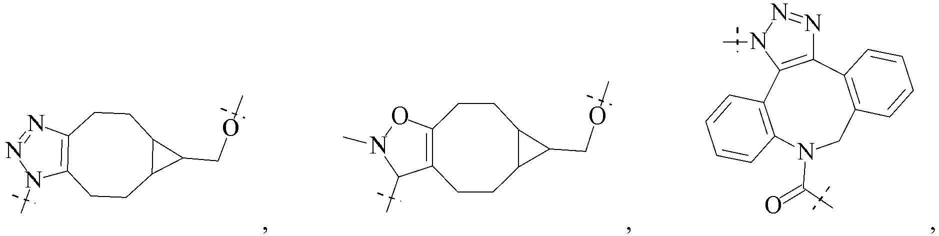 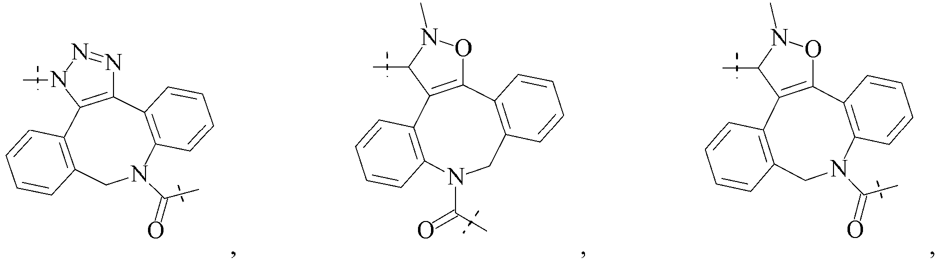 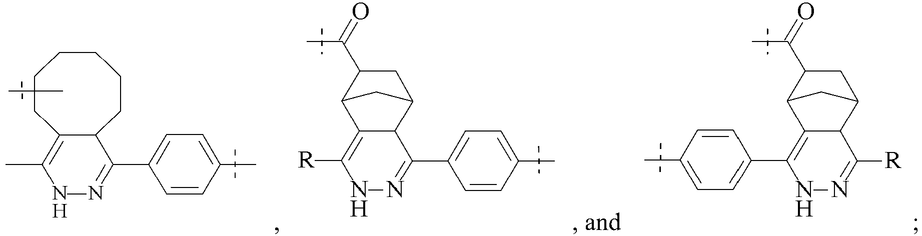 wherein
dashed lines indicate attachment to the rest of -L²-, -L¹-, -Z and/or -Z', respectively; and
-R and -R^{a} are independently of each other selected from the group consisting of -H, methyl, ethyl, propyl, butyl, pentyl and hexyl.

In one preferred embodiment -L²- has a chain lengths of 1 to 20 atoms.

As used herein the term "chain length" with regard to the moiety -L²- refers to the number of atoms of -L²- present in the shortest connection between -L¹- and -Z.

Preferably, -L²- is of formula (i) 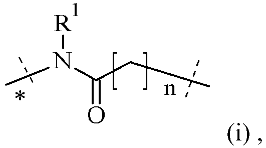 wherein
the dashed line marked with the asterisk indicates attachment to -L¹-;
the unmarked dashed line indicates attachment to -Z or -Z';
-R¹ is selected from the group consisting of -H, C₁₋₆ alkyl, C₂₋₆ alkenyl and C₂₋₆ alkynyl;
n is selected from the group consisting of 0, 1, 2, 3, 4, 5, 6, 7, 8, 9, 10, 11, 12, 13, 14, 15, 16, 17 and 18; and
wherein the moiety of formula (i) is optionally further substituted.

Preferably -R¹ of formula (i) is selected from the group consisting of -H, methyl, ethyl, propyl, and butyl. Even more preferably -R¹ of formula (i) is selected from the group consisting of -H, methyl, ethyl and propyl. Even more preferably -R¹ of formula (i) is selected from the group consisting of -H and methyl. Most preferably -R¹ of formula (i) is methyl.

Preferably n of formula (i) is selected from the group consisting of 0, 1, 2, 3, 4, 5, 6, 7, 8, 9 and 10. Even more preferably n of formula (i) is selected from the group consisting of 0, 1, 2, 3, 4 and 5. Even more preferably n of formula (i) is selected from the group consisting of 0, 1, 2 and 3. Even more preferably n of formula (i) is selected from the group consisting of 0 and 1. Most preferably n of formula (i) is 0.

In one preferred embodiment -L²- is a moiety selected from the group consisting of 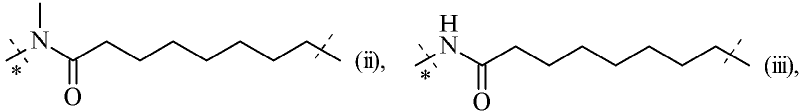 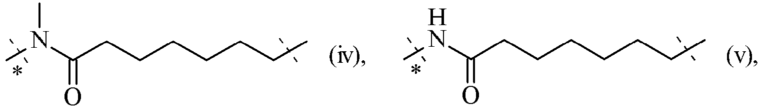 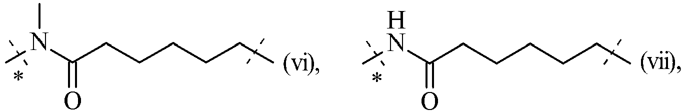 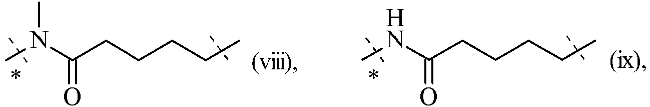 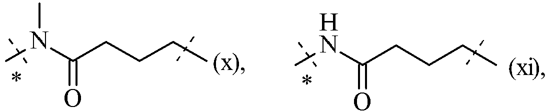 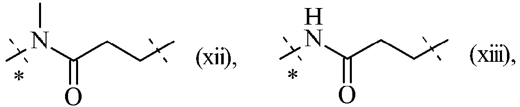 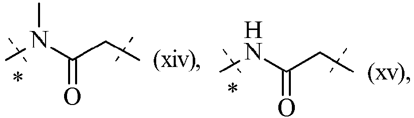 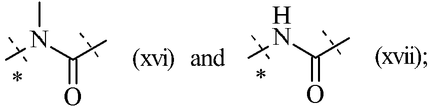 wherein
the dashed line marked with the asterisk indicates attachment to -L¹-;
the unmarked dashed line indicates attachment to -Z or -Z'; and
wherein the moieties (ii), (iii), (iv), (v), (vi), (vii), (viii), (ix), (x), (xi), (xii), (xiii), (xiv), (xv), (xvi) and (xvii) are optionally further substituted.

In a preferred embodiment -L²- is selected from the group consisting of 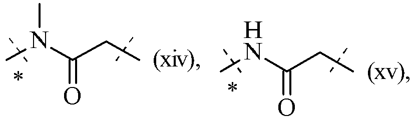 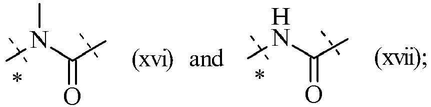 wherein
the dashed line marked with the asterisk indicates attachment to -L¹-; and
the unmarked dashed line indicates attachment to -Z or -Z'.

Even more preferred -L²- is selected from the group consisting of 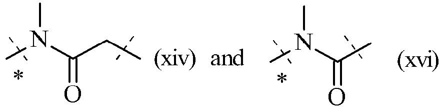 wherein
the dashed line marked with the asterisk indicates attachment to -L¹-; and
the unmarked dashed line indicates attachment to -Z or -Z'.

Even more preferably -L²- is 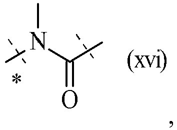 wherein
the dashed line marked with the asterisk indicates attachment to -L¹-; and
the unmarked dashed line indicates attachment to -Z or -Z'.

In one preferred embodiment the moiety -L¹-L²- is selected from the group consisting of 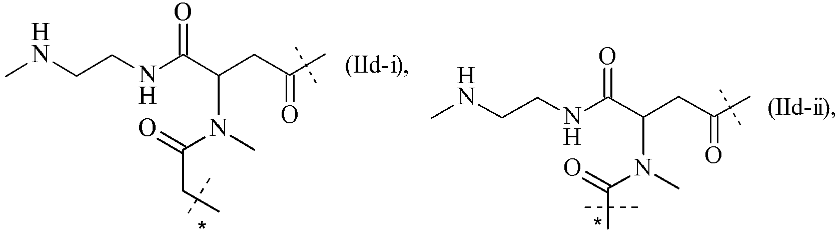 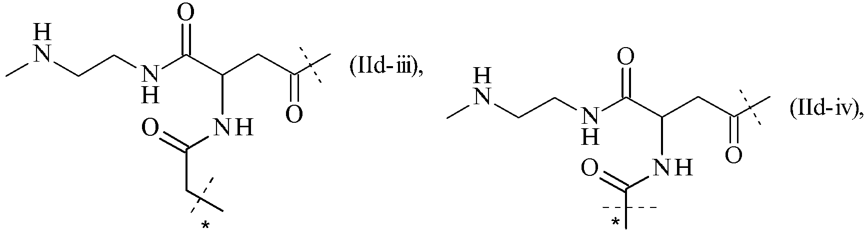 wherein
the unmarked dashed line indicates the attachment to a nitrogen of -D which is a CNP moiety by forming an amide bond; and
the dashed line marked with the asterisk indicates attachment to -Z or -Z'.

In an even more preferred embodiment the moiety -L¹-L²- is 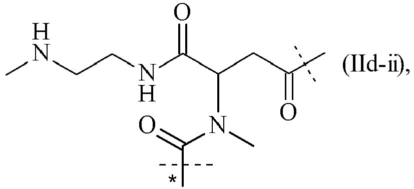 wherein
the unmarked dashed line indicates the attachment to a nitrogen of -D which is a CNP moiety by forming an amide bond; and
the dashed line marked with the asterisk indicates attachment to -Z or -Z'.

In a most preferred embodiment the moiety -L¹-L²- is of formula (IId-ii ') 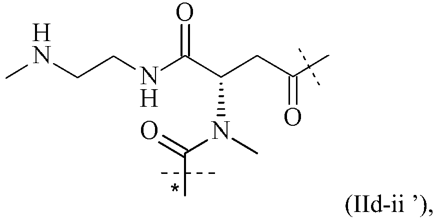 wherein
the unmarked dashed line indicates the attachment to a nitrogen of -D which is a CNP moiety by forming an amide bond; and
the dashed line marked with the asterisk indicates attachment to -Z or -Z'.

In another preferred embodiment the moiety -L¹-L²- is selected from the group consisting of 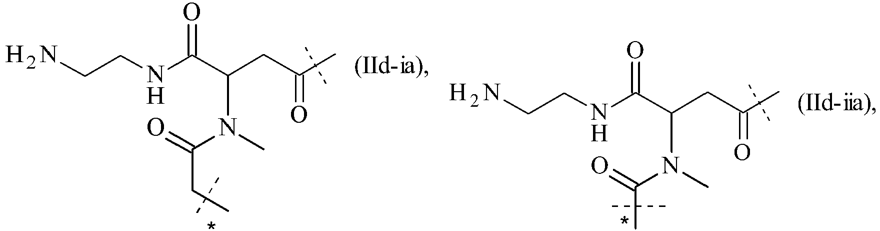 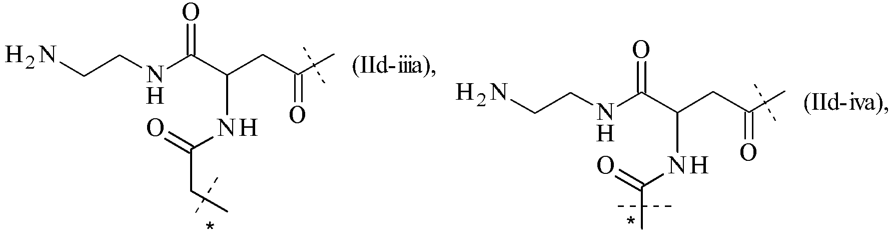 wherein
the unmarked dashed line indicates the attachment to a nitrogen of -D which is a CNP moiety by forming an amide bond; and
the dashed line marked with the asterisk indicates attachment to -Z or -Z'.

In an even more preferred embodiment the moiety -L¹-L²- is 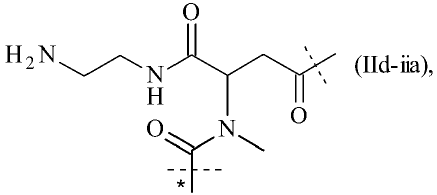 wherein
the unmarked dashed line indicates the attachment to a nitrogen of -D which is a CNP moiety by forming an amide bond; and
the dashed line marked with the asterisk indicates attachment to -Z or -Z'.

In a most preferred embodiment the moiety -L¹-L²- is of formula (IId-iia') 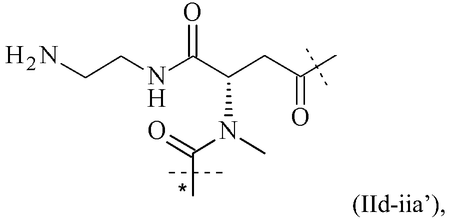 wherein
the unmarked dashed line indicates the attachment to a nitrogen of -D which is a CNP moiety by forming an amide bond; and
the dashed line marked with the asterisk indicates attachment to -Z or -Z'.

In another preferred embodiment the moiety -L¹-L²- is selected from the group consisting of 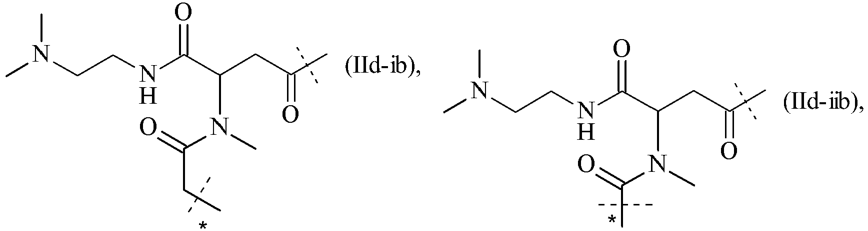 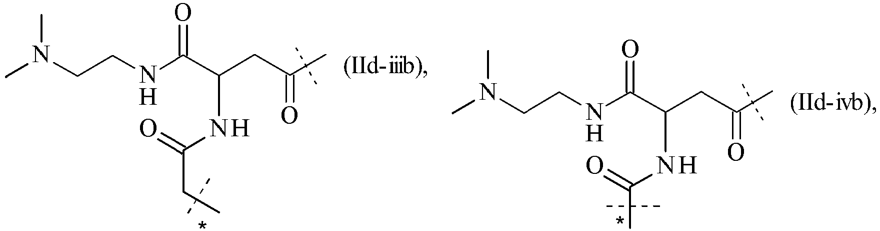 wherein
the unmarked dashed line indicates the attachment to a nitrogen of -D which is a CNP moiety by forming an amide bond; and
the dashed line marked with the asterisk indicates attachment to -Z or -Z'.

In an even more preferred embodiment the moiety -L¹-L²- is 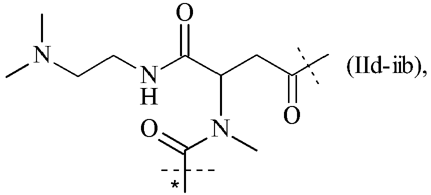 wherein
the unmarked dashed line indicates the attachment to a nitrogen of -D which is a CNP moiety by forming an amide bond; and
the dashed line marked with the asterisk indicates attachment to -Z or -Z'.

In a most preferred embodiment the moiety -L¹-L²- is of formula (IId-iib') 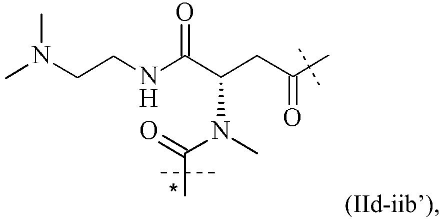 wherein
the unmarked dashed line indicates the attachment to a nitrogen of -D which is a CNP moiety by forming an amide bond; and
the dashed line marked with the asterisk indicates attachment to -Z or -Z'.

The moiety -L²- can be attached to -L¹- by replacing any -H present.

Preferably, one to five, preferably one, of the hydrogen(s) given by -R¹, -R^{1a}, -R², -R^{2a}, -R³, -R^{3a}, -R⁴, -R^{4a}, -R⁵, -R^{5a}, -R⁶, -R⁷, -R^{7a}, -R⁸, -R^{8a}, -R⁹, -R^{9a}, -R¹⁰, -R^{10a} and/or -R¹¹ of formula (II) are replaced by -L²-. Preferably, one to five, preferably one, of the hydrogen(s) given by -R¹, -R^{1a}, -R², -R^{2a}, -R³, -R^{3a}, -R⁴, -R⁵, -R^{5a}, -R⁶, -R^{6a}, -R⁷, -R^{7a}, -R⁸, -R^{8a}, -R^{8b}, -R⁹, -R^{9a}, -R^{9b}, -R¹⁰, -R^{10a}, -R^{10b}, -R¹¹, -R¹², -R^{12a}, -R¹³, -R^{13a} and/or -R^{13b} of formula (III) are replaced by -L²-.

Preferably, -Z has a molecular weight ranging from 5 to 200 kDa. Even more preferably, -Z has a molecular weight ranging from 8 to 100 kDa, even more preferably ranging from 10 to 80 kDa, even more preferably from 12 to 60, even more preferably from 15 to 40 and most preferably -Z has a molecular weight of about 20 kDa. In another equally preferred embodiment -Z has a molecular weight of about 40 kDa.

The carrier -Z comprises a C₈₋₂₄ alkyl or a polymer. Preferably, -Z comprises a polymer, preferably a polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

In one embodiment such water-soluble carrier -Z comprises a protein. Preferred proteins are selected from the group consisting of carboxyl*-*terminal peptide of the chorionic gonadotropin as described in US 2012/0035101 A1 which are herewith incorporated by reference; albumin; XTEN sequences as described in WO 2011123813 A2 which are herewith incorporated by reference; proline/alanine random coil sequences as described in WO 2011/144756 A1 which are herewith incorporated by reference; proline/alanine/serine random coil sequences as described in WO 2008/155134 A1 and WO 2013/024049 A1 which are herewith incorporated by reference; and Fc fusion proteins.

In another preferred embodiment, -Z comprises a fatty acid derivate. Preferred fatty acid derivatives are those disclosed in WO 2005/027978 A2 and WO 2014/060512 A1 which are herewith incorporated by reference.

In another preferred embodiment -Z is a hyaluronic acid-based polymer.

In one embodiment -Z is a carrier as disclosed in WO 2012/02047 A1 which is herewith incorporated by reference.

In another embodiment -Z is a carrier as disclosed in WO 2013/024048 A1 which is herewith incorporated by reference.

In another preferred embodiment -Z is a PEG-based polymer. Even more preferably -Z is a branched or multi-arm PEG-based polymer. Most preferably, -Z is a multi-arm PEG-based polymer. Even more preferably, -Z is a multi-arm PEG-based polymer having at least 4 PEG-based arms.

Preferably, such branched or multi-arm PEG-based polymer -Z, preferably multi-arm PEG-based polymer -Z, is connected to a multitude of moieties -L²-L¹-D, wherein each moiety -L²-L¹-D is preferably connected to the end of a branch or arm, preferably to the end of an arm. Preferably such branched or multi-arm PEG-based polymer -Z, preferably multi-arm PEG-based polymer -Z, is connected to 2, 3, 4, 5, 6, 7, 8, 9, 10, 11, 12, 13, 14, 15 or 16 moieties -L²-L¹-D. Even more preferably, such branched or multi-arm PEG-based polymer -Z, preferably multi-arm PEG-based polymer -Z, is connected to 2, 3, 4, 6 or 8 moieties -L²-L¹-D. Even more preferably such branched or multi-arm PEG-based polymer -Z, preferably multi-arm PEG-based polymer -Z, is connected to 2, 4 or 6 moieties -L²-L¹-D, even more preferably such branched or multi-arm PEG-based polymer -Z, preferably multi-arm PEG-based polymer -Z, is connected to 4 or 6 moieties -L²-L¹-D, and most preferably such branched or multi-arm PEG-based polymer -Z, preferably multi-arm PEG-based polymer -Z, is connected to 4 moieties -L²-L¹-D.

It is advantageous if more than one moiety -L²-L¹-D is connected to one moiety -Z, because this ensures a sufficiently high drug load which allows the presentation of a pharmaceutically effective dose of CNP in a small volume which in turn increases convenience for patients.

A preferred water-soluble PEG-based carrier -Z is a multi-arm PEG derivative as, for instance, detailed in the products list of JenKem Technology, USA (accessed by download from http://www.jenkemusa.com/Pages/PEGProducts.aspx on Dec 18, 2014), such as a 4-arm-PEG derivative, in particular a 4-arm-PEG comprising a pentaerythritol core, an 8-arm-PEG derivative comprising a hexaglycerin core, and an 8-arm-PEG derivative comprising a tripentaerythritol core. More preferably, the water-soluble PEG-based carrier -Z comprises a moiety selected from:
a 4-arm PEG Amine comprising a pentaerythritol core: 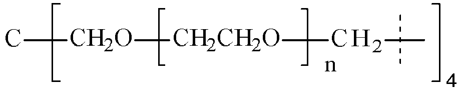
with n ranging from 20 to 500;
an 8-arm PEG Amine comprising a hexaglycerin core: 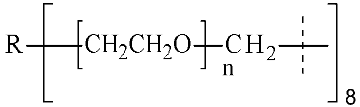
with n ranging from 20 to 500; and
R = hexaglycerin or tripentaerythritol core structure; and
a 6-arm PEG Amine comprising a sorbitol or dipentaerythritol core: 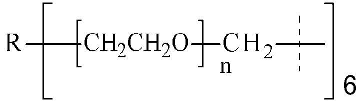
with n ranging from 20 to 500; and
R = comprising a sorbitol or dipentaerythritol core;
and wherein dashed lines indicate attachment to the rest of the CNP prodrug.

x of formula (Ia) is an integer selected from the group consisting of 1, 2, 3, 4, 5, 6, 7, 8, 9, 10, 11, 12, 13, 14, 15 and 16. Preferably, x is an integer selected from the group consisting of 2, 3, 4, 6 and 8. More preferably x is an integer selected from the group consisting of 2, 4, and 6. Even more preferably x is an integer selected from the group consisting of 4 and 6 and most preferably x is 4.

y of formula (Ib) is an integer selected from the group consisting of 1, 2, 3, 4 or 5. Preferably, y is an integer selected from the group consisting of 1, 2 or 3. In one preferred embodiment y is 1. In an equally preferred embodiment y is 2.

In another preferred embodiment one moiety-L²-L¹-D is connected to one moiety -Z.

In a particularly preferred embodiment -Z is a branched polymer. In one embodiment -Z is a branched polymer having one, two, three, four, five or six branching points. Preferably, -Z is a branched polymer having one, two or three branching points. In one embodiment -Z is a branched polymer having one branching point. In another embodiment -Z is a branched polymer having two branching points. In another embodiment -Z is a branched polymer having three branching points.

A branching point is preferably selected from the group consisting of -N<, -CH< and >C<.

Preferably such branched moiety -Z is PEG-based.

Preferably, such branched moiety -Z has a molecular weight of at least 10 kDa.

In one embodiment such branched moiety -Z has a molecular weight ranging from and including 10 kDa to 500 kDa, more preferably ranging from and including 10 kDa to 250 Da, even more preferably ranging from and including 10 kDa to 150 kDa, even more preferably ranging from and including 12 kDa to 100 kDa and most preferably ranging from and including 15 kDa to 80 kDa.

Preferably, such branched moiety -Z has a molecular weight ranging from and including 10 kDa to 80 kDa. In one embodiment the molecular weight is about 10 kDa. In another embodiment the molecular weight of such branched moiety -Z is about 20 kDa. In another embodiment the molecular weight of such branched moiety -Z is about 30 kDa. In another embodiment the molecular weight of such a branched moiety -Z is about 40 kDa. In another embodiment the molecular weight of such a branched moiety -Z is about 50 kDa. In another embodiment the molecular weight of such a branched moiety -Z is about 60 kDa. In another embodiment the molecular weight of such a branched moiety -Z is about 70 kDa. In another embodiment the molecular weight of such a branched moiety -Z is about 80 kDa. Most preferably, such branched moiety -Z has a molecular weight of about 40 kDa.

Applicants surprisingly found that an N-terminal attachment of a moiety -L¹-L²-Z is significantly more efficient with regard to increasing NEP-stability than attachment at an internal site and that the least efficient attachment site with regard to increasing NEP-stability is at the ring part of a CNP moiety. However, applicants surprisingly found that this disadvantage of attachment to the ring with regard to increasing NEP-stability can be compensated by using a branched moiety -Z having a molecular weight of at least 10 kDa, such as at least 12 kDa, such as at least 15 kDa, such as at least 18 kDa, such as at least 20 kDa, such as at least 24 kDa, such as at least 25 kDa, such as at least 27 kDa, such as at least 30 kDa. Preferably, such branched moiety -Z has a molecular weight of no more than 500 kDa, preferably of no more than 250 kDa, preferably of no more than 200 Da, preferably of no more than 150 kDa and most preferably no more than 100 kDa. Most preferably such branched moiety -Z has a molecular weight of about 40 kDa. Consequently, the use of such branched moiety -Z at the ring part of the CNP moiety does not only lead to increased NEP-stability, but combines increased NEP-stability with the reduced NPR-B binding associated with attachment to the ring.

Preferably, -Z or -Z' comprises a moiety

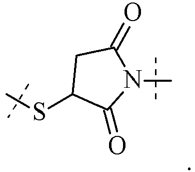

In one embodiment -Z comprises a moiety of formula (a) 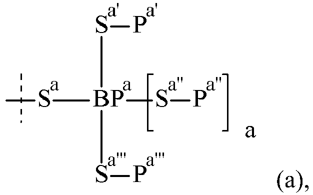 wherein
the dashed line indicates attachment to -L²- or to the remainder of -Z;
BP^{a} is a branching point selected from the group consisting of -N<, -CR< and >C<;
-R is selected from the group consisting of -H and C₁₋₆ alkyl;
a is 0 if BP^{a} is -N< or -CR< and n is 1 if BP^{a} is >C<;
-S^{a}-, -S^{a'}-, -S^{a"}- and -S^{a‴}- are independently of each other a chemical bond or are selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each -T- is independently optionally substituted with one or more -R¹, which are the same or different;
each -R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different;
each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different; and
-P^{a'}, -P^{a''} and -P^{a‴} are independently a polymeric moiety.

In one embodiment BP^{a} of formula (a) is -N<.

In another embodiment BP^{a} of formula (a) is -CR<. Preferably, -R is -H. Accordingly, a of formula (a) is preferably 0.

In another embodiment BP^{a} of formula (a) is >C<.

In one embodiment -S^{a}- of formula (a) is a chemical bond.

In another embodiment -S^{a}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-, -S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a}- of formula (a) is selected from the group consisting of methyl, ethyl, propyl, butyl, which are optionally interrupted by one or more chemical groups selected from the group consisting of -O-, -C(O)- and -C(O)N(R⁴)-.

In one embodiment -S^{a'}- of formula (a) is a chemical bond.

In another embodiment -S^{a'}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-, -S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a'}- of formula (a) is selected from the group consisting of methyl, ethyl, propyl, butyl, which are optionally interrupted by one or more chemical groups selected from the group consisting of -O-, -C(O)- and -C(O)N(R⁴)-.

In one embodiment -S^{a"}- of formula (a) is a chemical bond.

In another embodiment -S^{a"}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-,-S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a}"- of formula (a) is selected from the group consisting of methyl, ethyl, propyl, butyl, which are optionally interrupted by one or more chemical groups selected from the group consisting of -O-, -C(O)- and -C(O)N(R⁴)-.

In one embodiment -S^{a‴}- of formula (a) is a chemical bond.

In another embodiment -S^{a‴}- of formula (a) is selected from the group consisting of C₁₋₁₀ alkyl, C₂₋₁₀ alkenyl and C₂₋₁₀ alkynyl, which C₁₋₁₀ alkyl, C₂₋₁₀ alkenyl and C₂₋₁₀ alkynyl are optionally interrupted by one or more chemical groups selected from the group consisting of -C(O)O-, -O-, -C(O)-, -C(O)N(R⁴)-, -S(O)₂N(R⁴)-, -S(O)N(R⁴)-,-S(O)₂-, -S(O)-, -N(R⁴)S(O)₂N(R^{4a})-, -S-, -N(R⁴)-, -OC(OR⁴)(R^{4a})-, -N(R⁴)C(O)N(R^{4a})-, and -OC(O)N(R⁴)-; wherein -R⁴ and -R^{4a} are independently selected from the group consisting of -H, methyl, ethyl, propyl and butyl. Preferably -S^{a‴}- of formula (a) is selected from the group consisting of methyl, ethyl, propyl, butyl, which are optionally interrupted by one or more chemical groups selected from the group consisting of -O-, -C(O)- and -C(O)N(R⁴)-.

Preferably, -P^{a'}, -P^{a"} and -P^{a‴} of formula (a) independently comprise a polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

More preferably, -P^{a'}, -P^{a"} and -P^{a‴} of formula (a) independently comprise a PEG-based moiety. Even more preferably, -P^{a'}, -P^{a"} and -P^{a‴} of formula (a) independently comprise a PEG-based moiety comprising at least 20% PEG, even more preferably at least 30%, even more preferably at least 40% PEG, even more preferably at least 50% PEG, even more preferably at least 60% PEG, even more preferably at least 70% PEG, even more preferably at least 80% PEG and most preferably at least 90% PEG.

Preferably, -P^{a'}, -P^{a"} and -P^{a‴} of formula (a) independently have a molecular weight ranging from and including 5 kDa to 50 kDa, more preferably have a molecular weight ranging from and including 5 kDa to 40 kDa, even more preferably ranging from and including 7.5 kDa to 35 kDa, even more preferably ranging from and 7.5 to 30 kDa, even more preferably ranging from and including 10 to 30 kDa.

In one embodiment -P^{a'}, -P^{a"} and -P^{a‴} of formula (a) have a molecular weight of about 5 kDa.

In another embodiment -P^{a'}, -P^{a"} and -P^{a‴} of formula (a) have a molecular weight of about 7.5 kDa.

In another embodiment -P^{a'}, -P^{a"} and -P^{a‴} of formula (a) have a molecular weight of about 10 kDa.

In another embodiment -P^{a'}, -P^{a"} and -P^{a‴} of formula (a) have a molecular weight of about 12.5 kDa.

In another embodiment -P^{a'}, -P^{a"} and -P^{a‴} of formula (a) have a molecular weight of about 15 kDa.

In another embodiment -P^{a'}, -P^{a"} and -P^{a‴} of formula (a) have a molecular weight of about 20 kDa.

In one embodiment -Z comprises one moiety of formula (a).

In another embodiment -Z comprises two moieties of formula (a).

In another embodiment -Z comprises three moieties of formula (a).

In another embodiment -Z comprises four moieties of formula (a).

In another embodiment -Z comprises five moieties of formula (a).

In another embodiment -Z comprises six moieties of formula (a).

In a preferred embodiment -Z comprises two moieties of formula (a).

In a preferred embodiment -Z comprises a moiety of formula (b) 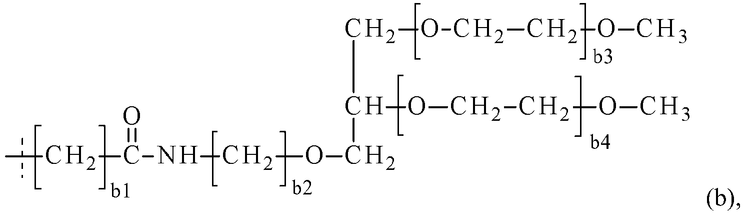 wherein
the dashed line indicates attachment to -L²- or to the remainder of -Z;
b1 is selected from the group consisting of 0, 1, 2, 3, 4, 5, 6, 7 and 8;
b2 is selected from the group consisting of 1, 2, 3, 4, 5, 6, 7 and 8;
b3 is an integer ranging from and including 150 to 1000; preferably ranging from and including 150 to 500; and most preferably ranging from and including 200 to 460; and b4 is an integer ranging from and including 150 to 1000; preferably ranging from and including 150 to 500; and most preferably ranging from and including 200 to 460.

Preferably, b3 and b4 of formula (b) are the same integer.

In one preferred embodiment b3 and b4 both an integer ranging from 200 to 250 and most preferably b3 and b4 of formula (b) are about 225.

In another preferred embodiment b3 and b4 are both an integer ranging from 400 to 500 and most preferably b3 and b4 of formula (b) are about 450.

Preferably, b1 of formula (b) is selected from the group consisting of 0, 1, 2, 3 and 4. More preferably b1 of formula (b) is selected from the group consisting of 1, 2 and 3. Most preferably b1 of formula (b) is 2.

Preferably, b2 of formula (b) is selected from the group consisting of 1, 2, 3, 4 and 5. More preferably b2 of formula (b) is selected from the group consisting of 2, 3 and 4. Most preferably b2 of formula (b) is 3.

In one particularly preferred embodiment b1 of formula (b) is 2, b2 of formula (b) is 3, and b3 and b4 are both about 450.

In another particularly preferred embodiment b1 of formula (b) is 2, b2 of formula (b) is 3, and b3 and b4 are both about 225.

In one embodiment -Z comprises one moiety of formula (b).

In another embodiment -Z comprises two moieties of formula (b).

In another embodiment -Z comprises three moieties of formula (b).

In another embodiment -Z comprises four moieties of formula (b).

In another embodiment -Z comprises five moieties of formula (b).

In another embodiment -Z comprises six moieties of formula (b).

In a preferred embodiment -Z comprises two moieties of formula (b).

In an even more preferred embodiment -Z comprises a moiety of formula (c) 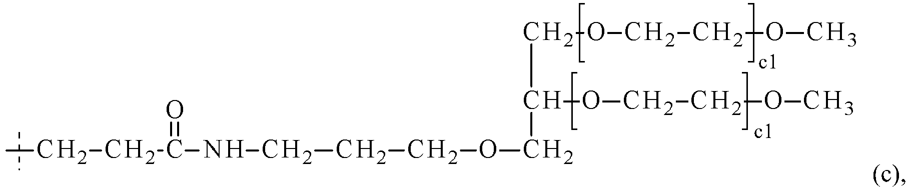 wherein
the dashed line indicates attachment to -L²- or to the remainder of -Z;
c1 and c2 are independently an integer ranging from and including 150 to 500; preferably ranging from and including 200 to 460.

Preferably both c1 and c2 of formula (c) are the same integer.

In one preferred embodiment c1 and c2 of formula (c) range from and include 200 to 250 and most preferably are about 225. In another preferred embodiment c1 and c2 of formula (c) range from and include 400 to 500 and most preferably are about 450.

In a preferred embodiment the moiety -Z is a branched PEG-based polymer comprising at least 10% PEG, has one branching point and two PEG-based polymer arms and has a molecular weight of about 40 kDa. Accordingly, each of the two PEG-based polymer arms has a molecular weight of about 20 kDa. Preferably the branching point is -CH<.

In one embodiment -Z comprises one moiety of formula (c).

In another embodiment -Z comprises two moieties of formula (c).

In another embodiment -Z comprises three moieties of formula (c).

In another embodiment -Z comprises four moieties of formula (c).

In another embodiment -Z comprises five moieties of formula (c).

In another embodiment -Z comprises six moieties of formula (c).

In a preferred embodiment -Z comprises two moieties of formula (c).

In one preferred embodiment the moiety -Z is of formula (d) 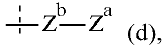 wherein
the dashed line indicates attachment to -L²-;
-Z^{b}- is selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
   each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each -T- is independently optionally substituted with one or more -R¹, which are the same or different;
   each -R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different;
   each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different;
      and
-Z^{a} is 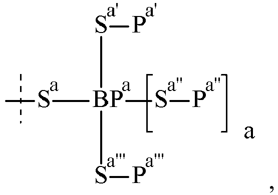
wherein
BP^{a}, -S^{a}-, -S^{a'}-, -S^{a"}-, -S^{a‴}-, -P^{a'}, -P^{a"}, -P^{a‴} and a are used as defined for formula (a).

Preferred embodiments of BP^{a}, -S^{a}-, -S^{a'}-, -S^{a"}-, -S^{a‴}-, -P^{a'}, -P^{a"}, -P^{a‴} of formula (d) are as defined above for formula (a).

In an even more preferred embodiment the moiety -Z is of formula (e) 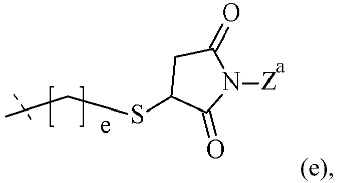 wherein
the dashed line indicates attachment to -L²-;
e is selected from the group consisting of 1, 2, 3, 4, 5, 6, 7, 8, 9, 10, 11, 12, 13, 14 and 15; and
-Z^{a} is 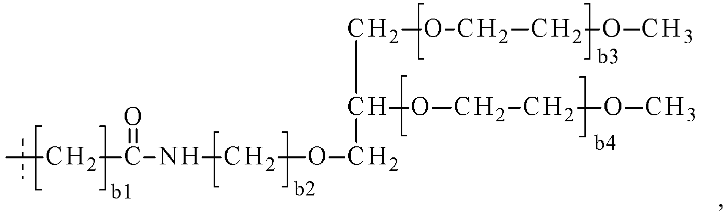 wherein
   b1 is selected from the group consisting of 0, 1, 2, 3, 4, 5, 6, 7 and 8;
   b2 is selected from the group consisting of 1, 2, 3, 4, 5, 6, 7 and 8;
   b3 is an integer ranging from and including 150 to 1000; preferably ranging from and including 150 to 500; and most preferably ranging from and including 200 to 460; and
   b4 is an integer ranging from and including 150 to 1000; preferably ranging from and including 150 to 500; and most preferably ranging from and including 200 to 460.

Preferred embodiments for b1, b2, b3 and b4 of formula (e) are as defined above for formula (b).

In one embodiment e of formula (e) is 1. In another embodiment e of formula (e) is 2. In another embodiment e of formula (e) is 3. In another embodiment e of formula (e) is 4. In another embodiment e of formula (e) is 5. In another embodiment e of formula (e) is 6. In another embodiment e of formula (e) is 7. In another embodiment e of formula (e) is 8. In another embodiment e of formula (e) is 9. In another embodiment e of formula (e) is 10. In another embodiment e of formula (e) is 11. In another embodiment e of formula (e) is 12. In another embodiment e of formula (e) is 13. In another embodiment e of formula (e) is 14. In another embodiment e of formula (e) is 15.

Preferably e of formula (e) is selected from the group consisting of 2, 3, 4, 5, 6, 7, 8 and 9. Even more preferably, e of formula (e) is selected from 3, 4, 5 and 6. Most preferably e of formula (e) is 5.

Preferably e of formula (e) is 5, b1 of formula (e) is 2, b2 of formula (e) is 3 and b3 and b4 of formula (e) are both about 450.

In another preferred embodiment the moiety -Z is a branched PEG-based polymer comprising at least 10% PEG, has three branching points and four PEG-based polymer arms and has a molecular weight of about 40 kDa. Accordingly, each of the four PEG-based polymer arms has a molecular weight of about 10 kDa. Preferably each of the three branching points is -CH<.

In a preferred embodiment the moiety -Z is of formula (f) 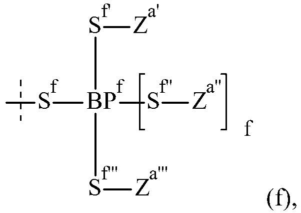 wherein
the dashed line indicates attachment to -L²-;
BP^{f} is a branching point selected from the group consisting of -N<, -CR< and >C<;
-R is selected from the group consisting of -H and C₁₋₆ alkyl;
f is 0 if BP^{f} is -N< or -CR< and f is 1 if BP^{f} is >C<;
-S^{f}-, -S^{f'}-, -S^{f"}- and -S^{f‴}- are independently either a chemical bond or are independently selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
   each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each -T- is independently optionally substituted with one or more -R¹, which are the same or different;
   each R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different;
   each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different;
      and
-z^{a'}, -z^{a"} and -Z^{a‴} are independently 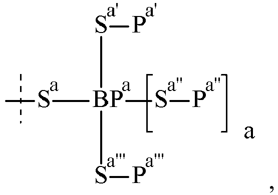 wherein
   BP^{a}, -S^{a}-, -S^{a'}-, -S^{a"}-, -S^{a‴}-, -P^{a'}, -P^{a"}, -P^{a‴} and a are used as defined for formula (a).

Preferred embodiments of BP^{a}, -S^{a}-, -S^{a'}-, -S^{a"}-, -S^{a‴}-, -P^{a'}, -P^{a"} and -P^{a‴} of formula (f) are as defined above for formula (a).

Preferably BP² of formula (f) is -CR< and r is 0. Preferably -R is -H.

Preferably -S^{f}- of formula (f) is a chemical bond.

Preferably, -Z^{a'}, -Z^{a"} and -z^{a‴} of formula (f) have the same structure. Preferably, -Z^{a'}, -Z^{a"} and -Z^{a‴} of formula (f) are of formula (b).

Preferably -S^{f}- of formula (f) is a chemical bond, BP^{a} of formula (f) is -CR< with -R being -H. Even more preferably -S^{f}- of formula (f) is a chemical bond, BP^{a} of formula (f) is -CR< with -R being -H and -Z^{a'}, -Z^{a"} and -Z^{a‴} of formula (f) are of formula (b).

Even more preferably -Z is of formula (g) 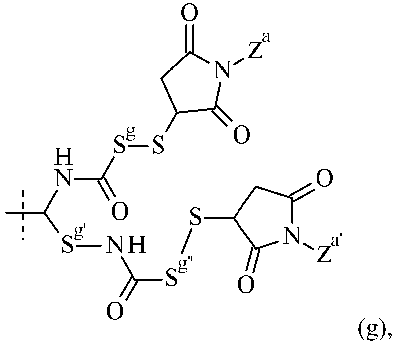 wherein
the dashed line indicates attachment to -L²-;
-S^{g}-, -S^{g'}- and -S^{g"}- are independently selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
   each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each -T- is independently optionally substituted with one or more -R¹, which are the same or different; each R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different;
   each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different;
      and
-Z^{a} and -Z^{a'} are independently 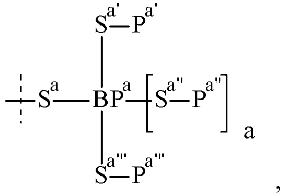
wherein
BP^{a}, -S^{a}-, -S^{a'}-, -S^{a"}-, -S^{a‴}-, -P^{a'}, -P^{a"}, -P^{a‴} and a are used as defined for formula (a).

Preferred embodiments of BP^{a}, -S^{a}-, -S^{a'}-, -S^{a"}-, -S^{a‴}-, -P^{a'}, -P^{a"} and -P^{a‴} of formula (g) are as defined above for formula (a).

Preferably, -S^{g}- of formula (g) is selected from the group consisting of C₁₋₆ alkyl, C₂₋₆ alkenyl and C₂₋₆ alkynyl, which are optionally substituted with one or more -R¹, which is the same or different,
wherein
-R¹ is selected from the group consisting of halogen, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different; and
-R³, -R^{3a} and -R^{3b} are independently selected from -H, methyl, ethyl, propyl and butyl.

Even more preferably -S^{g}- of formula (g) is selected from C₁₋₆ alkyl.

Preferably, -S^{g'}- of formula (g) is selected from the group consisting of C₁₋₆ alkyl, C₂₋₆ alkenyl and C₂₋₆ alkynyl, which are optionally substituted with one or more -R¹, which is the same or different,
wherein
-R¹ is selected from the group consisting of halogen, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different; and
-R³, -R^{3a} and -R^{3b} are independently selected from -H, methyl, ethyl, propyl and butyl.

Even more preferably -S^{g'}- of formula (g) is selected from C₁₋₆ alkyl.

Preferably, -S^{g''}- of formula (g) is selected from the group consisting of C₁₋₆ alkyl, C₂₋₆ alkenyl and C₂₋₆ alkynyl, which are optionally substituted with one or more -R¹, which is the same or different,
wherein
-R¹ is selected from the group consisting of halogen, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different; and
-R³, -R^{3a} and -R^{3b} are independently selected from -H, methyl, ethyl, propyl and butyl.

Even more preferably -S^{g"}- of formula (g) is selected from C₁₋₆ alkyl.

Preferably, -Z^{a} and -Z^{a'} of formula (g) have the same structure. Preferably, -Z^{a} and -Z^{a'} of formula (g) are of formula (b).

Even more preferably -Z is of formula (h) 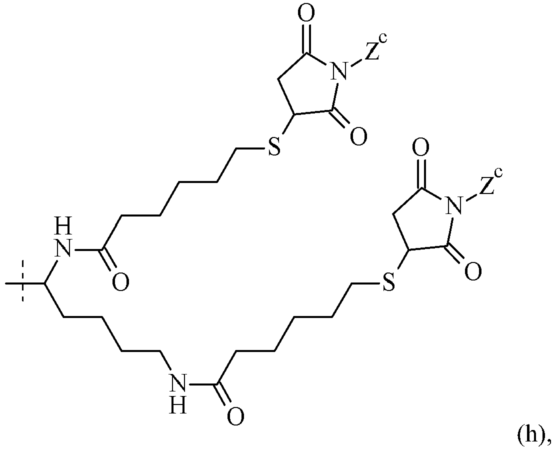 wherein
the dashed line indicates attachment to -L²-; and
each -Z^{c} is a moiety 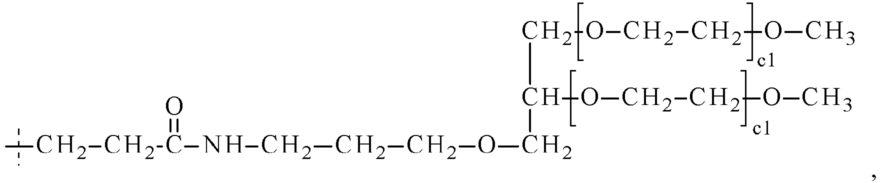 wherein
   each c1 is an integer independently ranging from about 200 to 250.

Preferably both c1 of formula (h) are the same.

Preferably both c1 of formula (h) are about 225.

In an even more preferred embodiment the moiety -Z is of formula (h-i) 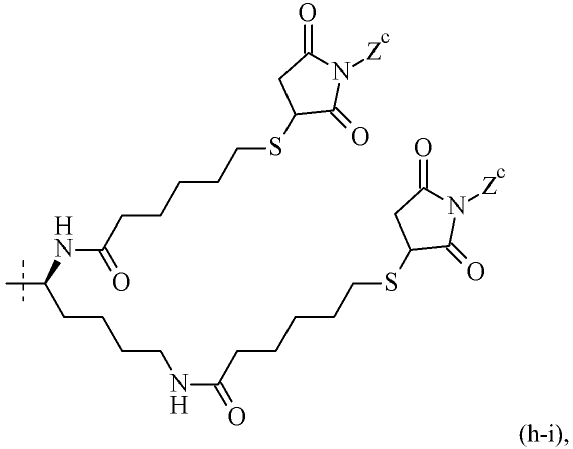 wherein
the dashed line indicates attachment to -L²-; and
each -Z^{c} is a moiety 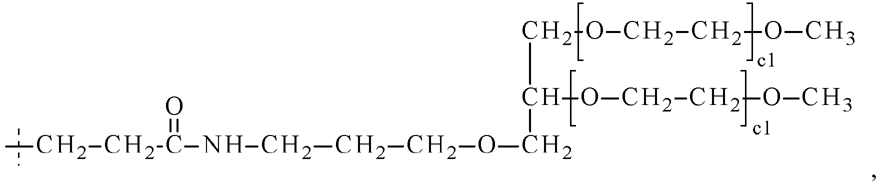 each c1 is an integer independently ranging from 200 to 250.

Preferably both c1 of formula (h-i) are the same.

Preferably both c1 of formula (h-i) are about 225.

Preferably, the CNP prodrug of the present invention is of formula (Ia).

Preferably the CNP prodrug of the present invention is of formula (Ia) with x = 1.

In a preferred embodiment the CNP prodrug of the present invention is of formula (IIe) 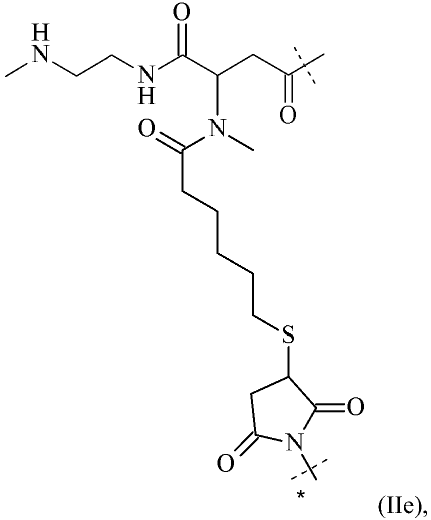 wherein
the unmarked dashed line indicates the attachment to a nitrogen of -D which is a CNP moiety by forming an amide bond; and
the dashed line marked with the asterisk indicates attachment to a moiety 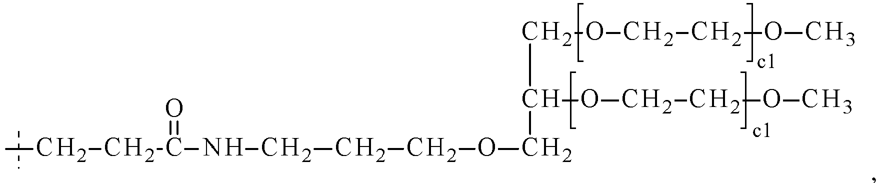 wherein
   each c1 is an integer independently ranging from 400 to 500.

Preferably, c1 of formula (IIe) is about 450.

In an equally preferred embodiment the CNP prodrug of the present invention is of formula (IIe-i) 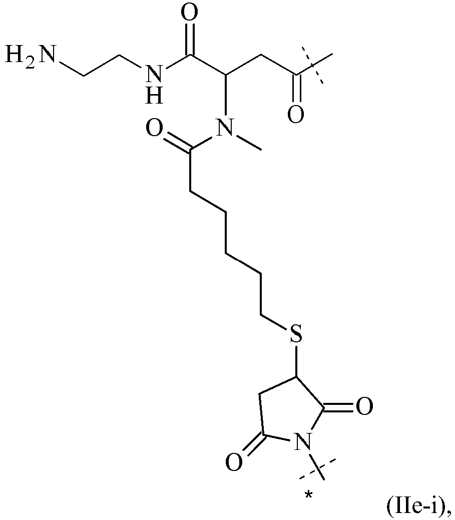 wherein
the unmarked dashed line indicates the attachment to a nitrogen of -D which is a CNP moiety by forming an amide bond; and
the dashed line marked with the asterisk indicates attachment to a moiety 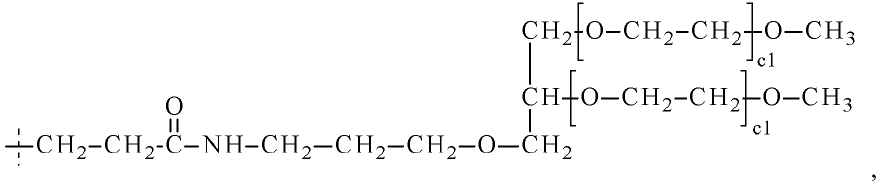 wherein
   each c1 is an integer independently ranging from 400 to 500.

Preferably, c1 of formula (IIe-i) is about 450.

In another equally preferred embodiment the CNP prodrug of the present invention is of formula (IIe-ii) 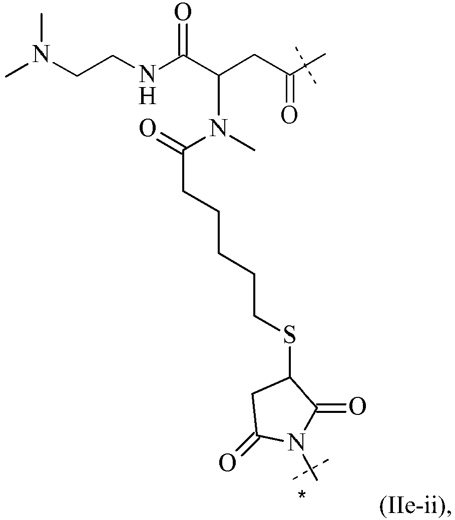 wherein
the unmarked dashed line indicates the attachment to a nitrogen of -D which is a CNP moiety by forming an amide bond; and
the dashed line marked with the asterisk indicates attachment to a moiety 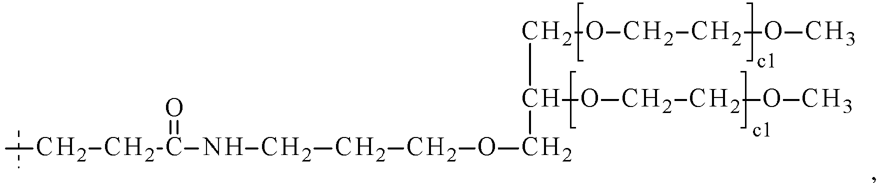 wherein
   each c1 is an integer independently ranging from 400 to 500.

Preferably, c1 of formula (IIe-ii) is about 450.

In one embodiment the CNP moiety of the CNP prodrug of formula (IIe), (IIe-i) and (IIe-ii) has the sequence of SEQ ID NO:25.

In another embodiment the CNP moiety of the CNP prodrug of formula (IIe), (IIe-i) and (IIe-ii) has the sequence of SEQ ID NO:30.

In a preferred embodiment the CNP moiety of the CNP prodrug of formula (IIe), (IIe-i) and (IIe-ii) has the sequence of SEQ ID NO:24.

In one embodiment the CNP moiety is attached to -L¹- in the CNP prodrug of formula (IIe), (IIe-i) and (IIe-ii) through the nitrogen of the N-terminal amine functional group of CNP.

In a preferred embodiment the CNP moiety is attached to -L¹- in the CNP prodrug of formula (IIe), (IIe-i) and (IIe-ii) through a nitrogen provided by the amine functional group of a lysine side chain of CNP.

In one embodiment this lysine side chain is not part of the ring formed by the disulphide bridge between the cysteine residues at positions 22 and 38, if the CNP moiety is of SEQ ID NO:24.

Accordingly, in one embodiment the CNP moiety is connected to -L¹- in the CNP prodrug of formula (IIe), (IIe-i) and (IIe-ii) through the amine functional group provided by the side chain of the lysine at position 9, if the CNP has the sequence of SEQ ID NO:24.

In another embodiment the CNP moiety is connected to -L¹- in the CNP prodrug of formula (IIe), (IIe-i) and (IIe-ii) through the amine functional group provided by the side chain of the lysine at position 11, if the CNP has the sequence of SEQ ID NO:24.

In another embodiment the CNP moiety is connected to -L¹- in the CNP prodrug of formula (IIe), (IIe-i) and (IIe-ii) through the amine functional group provided by the side chain of the lysine at position 15, if the CNP has the sequence of SEQ ID NO:24.

In another embodiment the CNP moiety is connected to -L¹- in the CNP prodrug of formula (IIe), (IIe-i) and (IIe-ii) through the amine functional group provided by the side chain of the lysine at position 16, if the CNP has the sequence of SEQ ID NO:24.

In another embodiment the CNP moiety is connected to -L¹- in the CNP prodrug of formula (IIe), (IIe-i) and (IIe-ii) through the amine functional group provided by the side chain of the lysine at position 20, if the CNP has the sequence of SEQ ID NO:24.

In a preferred embodiment the lysine side chain for attachment to the rest of the CNP prodrug of formula (IIe), (IIe-i) and (IIe-ii) is part of the ring formed by the disulphide bridge between the cysteine residues at positions 22 and 38, if the CNP moiety is of SEQ ID NO:24.

Accordingly, in a preferred embodiment the CNP moiety is connected to -L¹- in the CNP prodrug of formula (IIe), (IIe-i) and (IIe-ii) through the amine functional group provided by the side chain of the lysine at position 26, if the CNP has the sequence of SEQ ID NO:24.

It is understood that the positions of the cysteines and lysines mentioned above vary depending on the lengths of the CNP moiety and that the person skilled in the art will have no difficulty identifying the corresponding cysteines and lysines in longer or shorter versions of the CNP moiety and also understands that for example some lysines may not be present in shorter CNP moieties. It is further understood that as a result of for example site-directed mutagenesis there might be more lysine residues in the non-ring forming part and/or ring forming part of the CNP moiety.

In a preferred embodiment the CNP prodrug of the present invention is of formula (IIe), wherein c1 is about 450, the CNP moiety has the sequence of SEQ ID NO:24 and is attached to -L¹- through the amine functional group provided by the side chain of the lysine at position 26.

In another preferred embodiment the CNP prodrug of the present invention is of formula (IIe-i), wherein c1 is about 450, the CNP moiety has the sequence of SEQ ID NO:24 and is attached to -L¹- through the amine functional group provided by the side chain of the lysine at position 26.

In another preferred embodiment the CNP prodrug of the present invention is of formula (IIe-ii), wherein c1 is about 450, the CNP moiety has the sequence of SEQ ID NO:24 and is attached to -L¹- through the amine functional group provided by the side chain of the lysine at position 26.

Accordingly, in a preferred embodiment the CNP prodrug of the present invention is of formula (IIe') 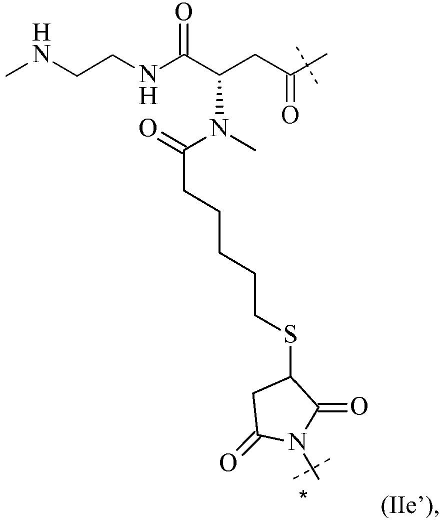 wherein
the unmarked dashed line indicates the attachment to a nitrogen provided by the side chain of the lysine at position 26 of the CNP moiety of SEQ ID NO:24 by forming an amide bond; and
the dashed line marked with the asterisk indicates attachment to a moiety 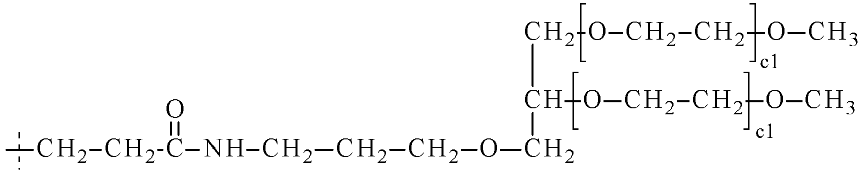
wherein
each c1 is an integer independently ranging from 400 to 500.

Preferably, each c1 of formula (IIe') is about 450.

In another preferred embodiment the CNP prodrug of the present invention is of formula (IIe-i') 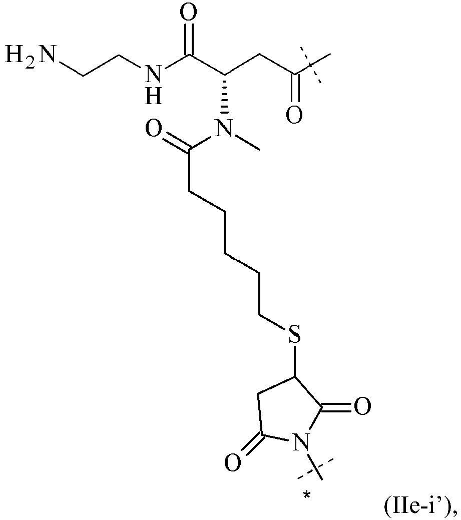 wherein
the unmarked dashed line indicates the attachment to a nitrogen provided by the side chain of the lysine at position 26 of the CNP moiety of SEQ ID NO:24 by forming an amide bond; and
the dashed line marked with the asterisk indicates attachment to a moiety 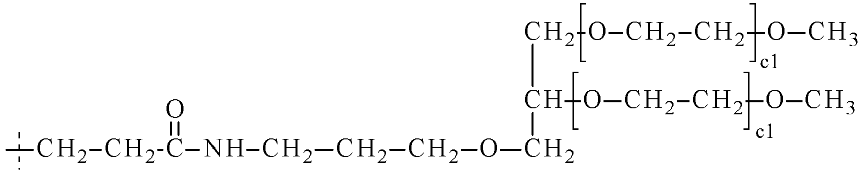
wherein
each c1 is an integer independently ranging from 400 to 500.

Preferably, each c1 of formula (IIe-i') is about 450.

In another preferred embodiment the CNP prodrug of the present invention is of formula (IIe-ii') 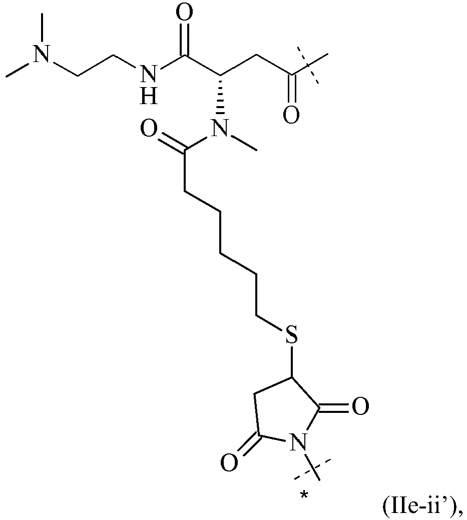 wherein
the unmarked dashed line indicates the attachment to a nitrogen provided by the side chain of the lysine at position 26 of the CNP moiety of SEQ ID NO:24 by forming an amide bond; and
the dashed line marked with the asterisk indicates attachment to a moiety 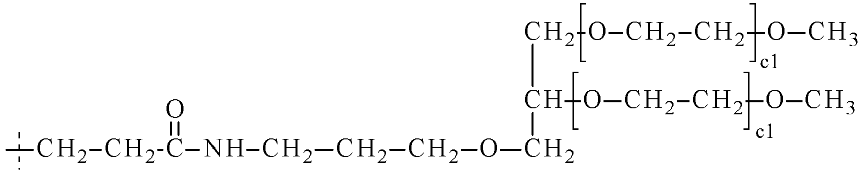
wherein
each c1 is an integer independently ranging from 400 to 500.

Preferably, each c1 of formula (IIe-ii') is about 450.

In another preferred embodiment the CNP prodrug of the present invention is of formula (IIf) 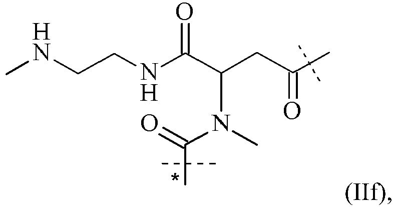 wherein
the unmarked dashed line indicates the attachment to a nitrogen of -D which is a CNP moiety by forming an amide bond; and
the dashed line marked with the asterisk indicates attachment to -Z having the structure 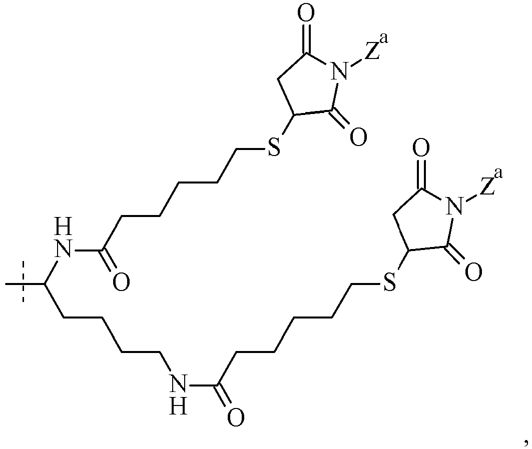 wherein
   each -Z^{a} is 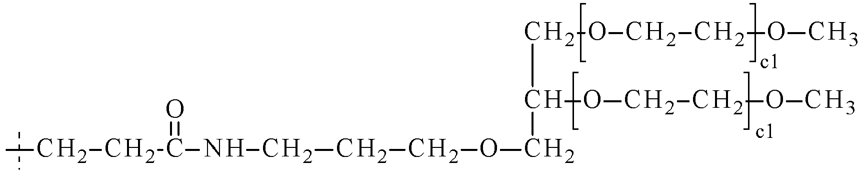 wherein
   each c1 is an integer independently ranging from 200 to 250; preferably each n is about 225.

Preferably, each c1 of formula (IIf) is about 225.

In another preferred embodiment the CNP prodrug of the present invention is of formula (IIf-i) 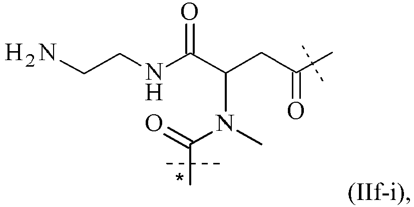 wherein
the unmarked dashed line indicates the attachment to a nitrogen of -D which is a CNP moiety by forming an amide bond; and
the dashed line marked with the asterisk indicates attachment to -Z having the structure 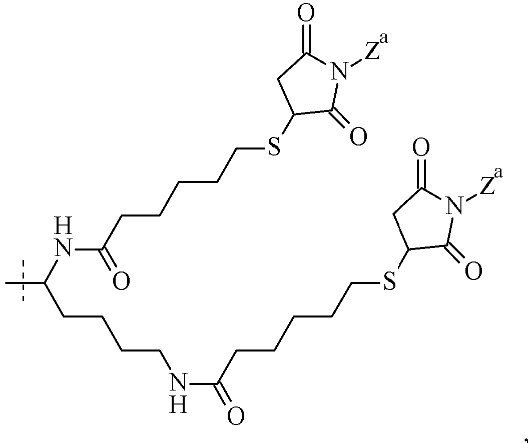 wherein
   each -Z^{a} is 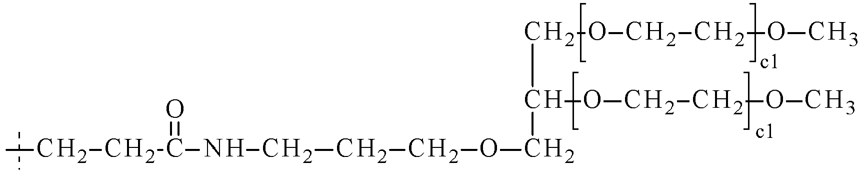 wherein
   each c1 is an integer independently ranging from 200 to 250; preferably each n is about 225.

Preferably, each c1 of formula (IIf-i) is about 225.

In another preferred embodiment the CNP prodrug of the present invention is of formula (IIf-ii) 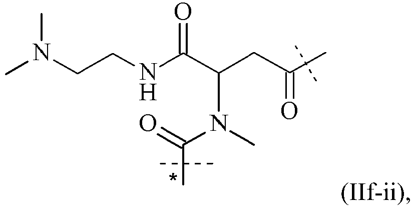 wherein
the unmarked dashed line indicates the attachment to a nitrogen of -D which is a CNP moiety by forming an amide bond; and
the dashed line marked with the asterisk indicates attachment to -Z having the structure 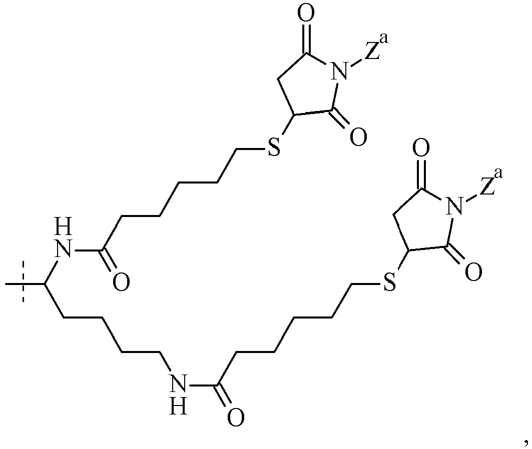 wherein
   each -Z^{a} is 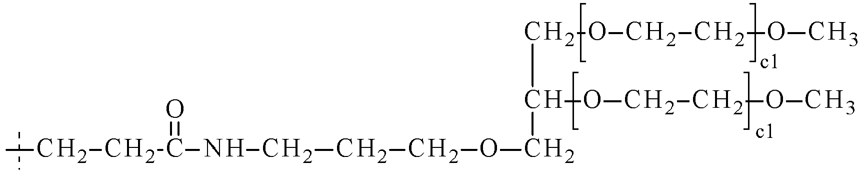 wherein
   each c1 is an integer independently ranging from 200 to 250; preferably each n is about 225.

Preferably, each c1 of formula (IIf-ii) is about 225.

In one embodiment the CNP moiety of the CNP prodrug of formula (IIf), (IIf-i) and (IIf-ii) has the sequence of SEQ ID NO:25.

In a preferred embodiment the CNP moiety of the CNP prodrug of formula (IIf), (IIf-i) and (IIf-ii) has the sequence of SEQ ID NO:24.

In one embodiment the CNP moiety is attached to -L¹- in the CNP prodrug of formula (IIf), (IIf-i) and (IIf-ii) through the nitrogen of the N-terminal amine functional group of CNP.

In a preferred embodiment the CNP moiety is attached to -L¹- in the CNP prodrug of formula (IIf), (IIf-i) and (IIf-ii) through a nitrogen provided by the amine functional group of a lysine side chain of CNP.

In one embodiment this lysine side chain is not part of the ring formed by the disulphide bridge between the cysteine residues at positions 22 and 38, if the CNP moiety is of SEQ ID NO:24.

Accordingly, in one embodiment the CNP moiety is connected to -L¹- in the CNP prodrug of formula (IIf), (IIf-i) and (IIf-ii) through the amine functional group provided by the side chain of the lysine at position 9, if the CNP has the sequence of SEQ ID NO:24.

In another embodiment the CNP moiety is connected to -L¹- in the CNP prodrug of formula (IIf), (IIf-i) and (IIf-ii) through the amine functional group provided by the side chain of the lysine at position 11, if the CNP has the sequence of SEQ ID NO:24.

In another embodiment the CNP moiety is connected to -L¹- in the CNP prodrug of formula (IIf), (IIf-i) and (IIf-ii) through the amine functional group provided by the side chain of the lysine at position 15, if the CNP has the sequence of SEQ ID NO:24.

In another embodiment the CNP moiety is connected to -L¹- in the CNP prodrug of formula (IIf), (IIf-i) and (IIf-ii) through the amine functional group provided by the side chain of the lysine at position 16, if the CNP has the sequence of SEQ ID NO:24.

In another embodiment the CNP moiety is connected to -L¹- in the CNP prodrug of formula (IIf), (IIf-i) and (IIf-ii) through the amine functional group provided by the side chain of the lysine at position 20, if the CNP has the sequence of SEQ ID NO:24.

In a preferred embodiment the lysine side chain for attachment to the rest of the CNP prodrug of formula (IIf), (IIf-i) and (IIf-ii) is part of the ring formed by the disulphide bridge between the cysteine residues at positions 22 and 38, if the CNP moiety is of SEQ ID NO:24.

Accordingly, in a preferred embodiment the CNP moiety is connected to -L¹- in the CNP prodrug of formula (IIf), (IIf-i) and (IIf-ii) through the amine functional group provided by the side chain of the lysine at position 26, if the CNP has the sequence of SEQ ID NO:24.

It is understood that the positions of the cysteines and lysines mentioned above vary depending on the lengths of the CNP moiety and that the person skilled in the art will have no difficulty identifying the corresponding cysteines and lysines in longer or shorter versions of the CNP moiety and also understands that for example some lysines may not be present in shorter CNP moieties. It is further understood that as a result of for example site-directed mutagenesis there might be more lysine residues in the non-ring forming part and/or ring forming part of the CNP moiety.

In a preferred embodiment the CNP prodrug of the present invention is of formula (IIf), wherein c1 is about 225, the CNP moiety has the sequence of SEQ ID NO:24 and is attached to -L¹- through the amine functional group provided by the side chain of the lysine at position 26.

In another preferred embodiment the CNP prodrug of the present invention is of formula (IIf-i), wherein c1 is about 225, the CNP moiety has the sequence of SEQ ID NO:24 and is attached to -L¹- through the amine functional group provided by the side chain of the lysine at position 26.

In another preferred embodiment the CNP prodrug of the present invention is of formula (IIf-ii), wherein c1 is about 225, the CNP moiety has the sequence of SEQ ID NO:24 and is attached to -L¹- through the amine functional group provided by the side chain of the lysine at position 26.

In another preferred embodiment the CNP prodrug of the present invention is of formula (IIf ') 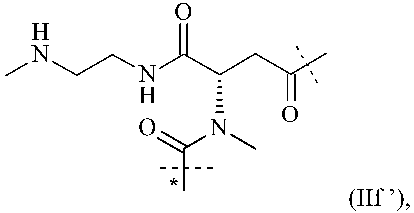 wherein
the unmarked dashed line indicates the attachment to a nitrogen provided by the side chain of the lysine at position 26 of the CNP moiety of SEQ ID NO:24 by forming an amide bond; and
the dashed line marked with the asterisk indicates attachment to -Z having the structure 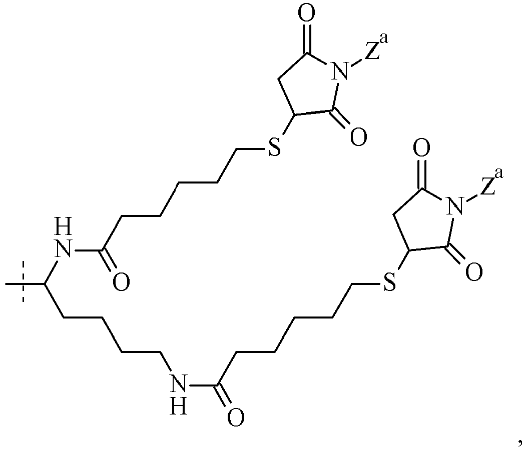 wherein
   each Z^{a} is 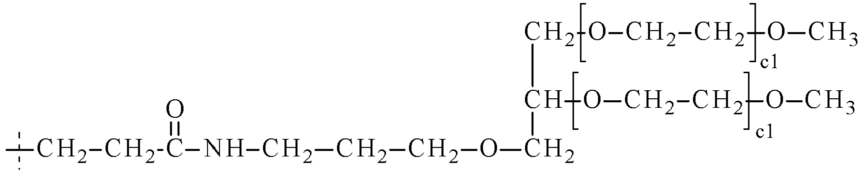 wherein

   each c1 is an integer independently ranging from 200 to 250.

Preferably, each c1 of formula (IIf ') is about 225.

In another preferred embodiment the CNP prodrug of the present invention is of formula (IIf-i') 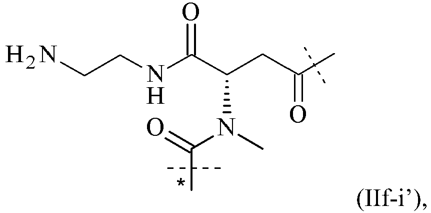 wherein
the unmarked dashed line indicates the attachment to a nitrogen provided by the side chain of the lysine at position 26 of the CNP moiety of SEQ ID NO:24 by forming an amide bond; and
the dashed line marked with the asterisk indicates attachment to -Z having the structure 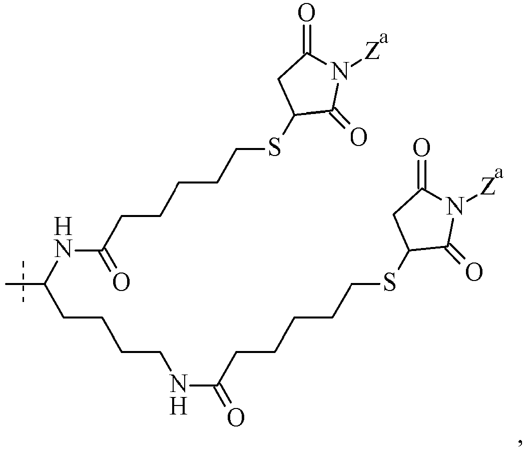 wherein
   each Z^{a} is 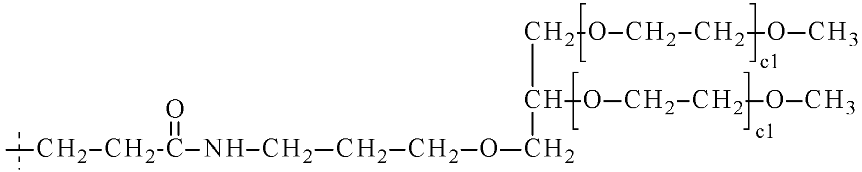 wherein
   each c1 is an integer independently ranging from 200 to 250.

Preferably, each c1 of formula (IIf-i') is about 225.

In another preferred embodiment the CNP prodrug of the present invention is of formula (IIf-ii') 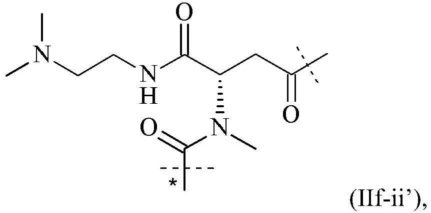 wherein
the unmarked dashed line indicates the attachment to a nitrogen provided by the side chain of the lysine at position 26 of the CNP moiety of SEQ ID NO:24 by forming an amide bond; and
the dashed line marked with the asterisk indicates attachment to -Z having the structure 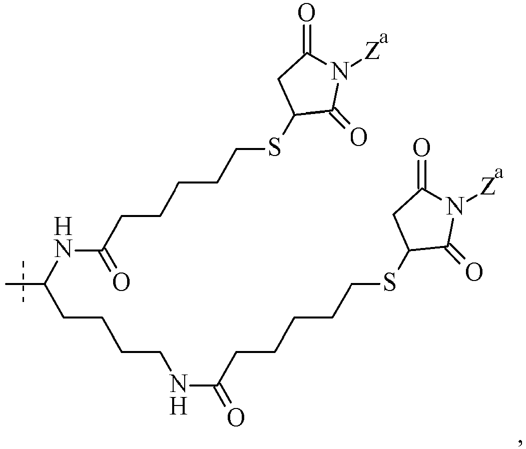 wherein
   each Z^{a} is 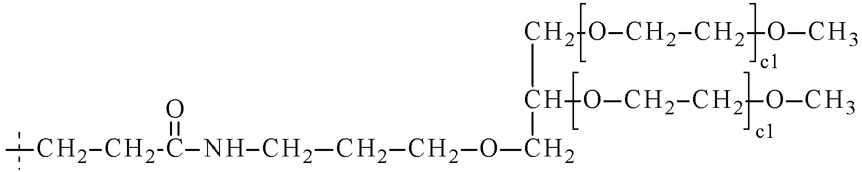 wherein
   each c1 is an integer independently ranging from 200 to 250.

Preferably, each c1 of formula (IIf-ii') is about 225.

In summary it was found that the combination of a reversible attachment of -L¹- to the CNP moiety via a side chain of an amino acid located in the ring moiety of CNP, the use of a branched moiety -Z having a molecular weight of at least 10 kDa and the use of a CNP moiety larger than CNP-22 leads to a CNP prodrug with an unexpected long *in vivo* half-life.

The carrier -Z' is a water-insoluble polymer, even more preferably a hydrogel. Preferably, such hydrogel comprises a polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.

If the carrier -Z' is a hydrogel, it is preferably a hydrogel comprising PEG or hyaluronic acid. Most preferably such hydrogel comprises PEG.

Even more preferably, the carrier -Z' is a hydrogel as described in WO 2006/003014 A2, WO 2011/012715 A1 or WO 2014/056926 A1, which are herewith incorporated by reference in their entirety.

In another embodiment -Z' is a polymer network formed through the physical aggregation of polymer chains, which physical aggregation is preferably caused by hydrogen bonds, crystallization, helix formation or complexation. In one embodiment such polymer network is a thermogelling polymer.

Preferably, the total mass of the CNP prodrug of the present invention is at least 10 kDa, such as at least 12 kDa, such as at least 15 kDa, such as at least 20 kDa or such as at least 30 kDa. It is preferred that the total mass of the CNP prodrug of the present invention is at most 250 kDa, such as at most 200 kDa, 180 kDa, 150 kDa or 100 kDa.

In a preferred embodiment the residual activity of the CNP prodrug of the present invention is less than 10%, more preferably less than 1%, even more preferably less than 0.1%, even more preferably less than 0.01%, even more preferably less than 0.001% and most preferably less than 0.0001%.

As used herein the term "residual activity" refers to the activity exhibited by the CNP prodrug of the present invention with the CNP moiety bound to a carrier in relation to the activity exhibited by the corresponding free CNP. In this context the term "activity" refers to NPR-B binding. It is understood that measuring the residual activity of the CNP prodrug of the present invention takes time during which a certain amount of CNP will be released the CNP prodrug of the present invention and that such released CNP will distort the results measured for the CNP prodrug. It is thus accepted practice to test the residual activity of a prodrug with a conjugate in which the drug moiety, in this case CNP, is non-reversibly, i.e. stably, bound to a carrier, which as closely as possible resembles the structure of the CNP prodrug for which residual activity is to be measured.

A suitable assay for measuring CNP activity and the residual activity of the CNP prodrug of the present invention, preferably in the form of a stable analog, is described in WO 2010/135541 A1, example 4, page 143/144.

Another aspect of the present invention is a pharmaceutical composition comprising at least one CNP prodrug of the present invention and at least one excipient.

In one embodiment the pharmaceutical composition comprising CNP prodrug molecules of the present invention comprises a mixture of CNP prodrugs in which the CNP moieties are attached to -L¹- through different functional groups, preferably through amine functional groups, provided by CNP, i.e. through the N-terminal amine functional group, through the amine functional group provided by the side chain of the lysine at position 4 and/or by the side chain of the lysine at position 10, if the CNP has the sequence of SEQ ID NO:1; through the N-terminal amine functional group, through the amine functional group provided by the side of the lysine at position 8, 10, 14, 15, 19 and/or 25, if the CNP has the sequence of SEQ ID NO:25; or through the N-terminal amine functional group, through the amine functional group provided by the side of the lysine at position 9, 11, 15, 16, 20 and/or 26, if the CNP has the sequence of SEQ ID NO:24.

In a preferred embodiment the CNP moieties of all CNP prodrug molecules comprised in the pharmaceutical composition are attached to -L¹- through the same amine functional group provided by CNP, i.e. either through the N-terminal amine functional group or through the amine functional group provided by the side chain of the lysine at position 4 or by the side chain of the lysine at position 10, if the CNP has the sequence of SEQ ID NO:1; through the amine functional group provided by the side chain of the lysine at position 8, 10, 14, 15, 19 or 25, if the CNP has the sequence of SEQ ID NO:25; or through the amine functional group provided by the side chain of the lysine at position 9, 11, 15, 16, 20 or 26, if the CNP has the sequence of SEQ ID NO:24. Most preferably the CNP moieties of all CNP prodrug molecules comprised in the pharmaceutical composition are attached to -L¹- through the same amine functional group, which is the amine functional group provided by the side chain of lysine 26, if the CNP moiety has the sequence of SEQ ID:NO 24.

Preferably, the pharmaceutical composition comprising at least one CNP prodrug of the present invention has a pH ranging from and including pH 3 to pH 8. More preferably, the pharmaceutical composition has a pH ranging from and including pH 4 to pH 6. Most preferably, the pharmaceutical composition has a pH ranging from and including pH 4 to pH 5.

In one embodiment the pharmaceutical composition comprising at least one CNP prodrug of the present invention and at least one excipient is a liquid or suspension formulation. It is understood that the pharmaceutical composition is a suspension formulation if the CNP prodrug of the present invention comprises a water-insoluble carrier -Z'.

In another embodiment the pharmaceutical composition comprising at least one CNP prodrug of the present invention and at least one excipient is a dry formulation.

Such liquid, suspension or dry pharmaceutical composition comprises at least one excipient. Excipients used in parenteral formulations may be categorized as, for example, buffering agents, isotonicity modifiers, preservatives, stabilizers, anti-adsorption agents, oxidation protection agents, viscosifiers/viscosity enhancing agents, or other auxiliary agents. However, in some cases, one excipient may have dual or triple functions. Preferably, the at least one excipient comprised in the pharmaceutical composition of the present invention is selected from the group consisting of
(i) Buffering agents: physiologically tolerated buffers to maintain pH in a desired range, such as sodium phosphate, bicarbonate, succinate, histidine, citrate and acetate, sulphate, nitrate, chloride, pyruvate; antacids such as Mg(OH)₂ or ZnCO₃ may be also used;
(ii) Isotonicity modifiers: to minimize pain that can result from cell damage due to osmotic pressure differences at the injection depot; glycerin and sodium chloride are examples; effective concentrations can be determined by osmometry using an assumed osmolality of 285-315 mOsmol/kg for serum;
(iii) Preservatives and/or antimicrobials: multidose parenteral formulations require the addition of preservatives at a sufficient concentration to minimize risk of patients becoming infected upon injection and corresponding regulatory requirements have been established; typical preservatives include m-cresol, phenol, methylparaben, ethylparaben, propylparaben, butylparaben, chlorobutanol, benzyl alcohol, phenylmercuric nitrate, thimerosol, sorbic acid, potassium sorbate, benzoic acid, chlorocresol, and benzalkonium chloride;
(iv) Stabilizers: Stabilisation is achieved by strengthening of the protein-stabilising forces, by destabilisation of the denatured state, or by direct binding of excipients to the protein; stabilizers may be amino acids such as alanine, arginine, aspartic acid, glycine, histidine, lysine, proline, sugars such as glucose, sucrose, trehalose, polyols such as glycerol, mannitol, sorbitol, salts such as potassium phosphate, sodium sulphate, chelating agents such as EDTA, hexaphosphate, ligands such as divalent metal ions (zinc, calcium, etc.), other salts or organic molecules such as phenolic derivatives; in addition, oligomers or polymers such as cyclodextrins, dextran, dendrimers, PEG or PVP or protamine or HSA may be used;
(v) Anti-adsorption agents: Mainly ionic or non-ionic surfactants or other proteins or soluble polymers are used to coat or adsorb competitively to the inner surface of the formulation´s container; e.g., poloxamer (Pluronic F-68), PEG dodecyl ether (Brij 35), polysorbate 20 and 80, dextran, polyethylene glycol, PEG-polyhistidine, BSA and HSA and gelatins; chosen concentration and type of excipient depends on the effect to be avoided but typically a monolayer of surfactant is formed at the interface just above the CMC value;
(vi) Oxidation protection agents: antioxidants such as ascorbic acid, ectoine, methionine, glutathione, monothioglycerol, morin, polyethylenimine (PEI), propyl gallate, and vitamin E; chelating agents such as citric acid, EDTA, hexaphosphate, and thioglycolic acid may also be used;
(vii) Viscosifiers or viscosity enhancers: retard settling of the particles in the vial and syringe and are used in order to facilitate mixing and resuspension of the particles and to make the suspension easier to inject (i.e., low force on the syringe plunger); suitable viscosifiers or viscosity enhancers are, for example, carbomer viscosifiers like Carbopol 940, Carbopol Ultrez 10, cellulose derivatives like hydroxypropylmethylcellulose (hypromellose, HPMC) or diethylaminoethyl cellulose (DEAE or DEAE-C), colloidal magnesium silicate (Veegum) or sodium silicate, hydroxyapatite gel, tricalcium phosphate gel, xanthans, carrageenans like Satia gum UTC 30, aliphatic poly(hydroxy acids), such as poly(D,L- or L-lactic acid) (PLA) and poly(glycolic acid) (PGA) and their copolymers (PLGA), terpolymers of D,L-lactide, glycolide and caprolactone, poloxamers, hydrophilic poly(oxyethylene) blocks and hydrophobic poly(oxypropylene) blocks to make up a triblock of poly(oxyethylene)-poly(oxypropylene)-poly(oxyethylene) (e.g. Pluronic^{®}), polyetherester copolymer, such as a polyethylene glycol terephthalate/polybutylene terephthalate copolymer, sucrose acetate isobutyrate (SAIB), dextran or derivatives thereof, combinations of dextrans and PEG, polydimethylsiloxane, collagen, chitosan, polyvinyl alcohol (PVA) and derivatives, polyalkylimides, poly (acrylamide-co-diallyldimethyl ammonium (DADMA)), polyvinylpyrrolidone (PVP), glycosaminoglycans (GAGs) such as dermatan sulfate, chondroitin sulfate, keratan sulfate, heparin, heparan sulfate, hyaluronan, ABA triblock or AB block copolymers composed of hydrophobic A-blocks, such as polylactide (PLA) or poly(lactide-co-glycolide) (PLGA), and hydrophilic B-blocks, such as polyethylene glycol (PEG) or polyvinyl pyrrolidone; such block copolymers as well as the abovementioned poloxamers may exhibit reverse thermal gelation behavior (fluid state at room temperature to facilitate administration and gel state above sol-gel transition temperature at body temperature after injection);
(viii) Spreading or diffusing agent: modifies the permeability of connective tissue through the hydrolysis of components of the extracellular matrix in the intrastitial space such as but not limited to hyaluronic acid, a polysaccharide found in the intercellular space of connective tissue; a spreading agent such as but not limited to hyaluronidase temporarily decreases the viscosity of the extracellular matrix and promotes diffusion of injected drugs; and
(ix) Other auxiliary agents: such as wetting agents, viscosity modifiers, antibiotics, hyaluronidase; acids and bases such as hydrochloric acid and sodium hydroxide are auxiliary agents necessary for pH adjustment during manufacture.

Another aspect of the present invention is the use of the CNP prodrug or a pharmaceutically acceptable salt thereof or a pharmaceutical composition comprising at least one CNP prodrug of the present invention as a medicament.

Another aspect of the present invention is the CNP prodrug or a pharmaceutically acceptable salt thereof or the pharmaceutical composition comprising at least one CNP prodrug of the present invention for use in a method of treatment of a disease which can be treated with CNP.

Preferably, said disease is selected from the group consisting of achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, camptomelic dysplasia, congenital lethal hypophosphatasia, perinatal lethal type of osteogenesis imperfecta, short-rib polydactyly syndromes, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, spondyloepimetaphyseal dysplasia, neurofibromatosis, Legius syndrome, LEOPARD syndrome, Noonan syndrome, hereditary gingival fibromatosis, neurofibromatosis type 1, Legius syndrome, cardiofaciocutaneous syndrome, Costello syndrome, SHOX deficiency, idiopathic short stature, growth hormone deficiency, osteoarthritis, cleidocranial dysostosis, craniosynostosis (e.g., Muenke syndrome, Crouzon syndrome, Apert syndrome, Jackson-Weiss syndrome, Pfeiffer syndrome, or Crouzonodermoskeletal syndrome), dactyly, brachydactyly, camptodactyly, polydactyly, syndactyly, dyssegmental dysplasia, enchondromatosis, fibrous dysplasia, hereditary multiple exostoses, hypophosphatemic rickets, Jaffe-Lichtenstein syndrome, Marfan syndrome, McCune-Albright syndrome, osteopetrosis and osteopoikilosis.

Preferably said disease is selected from the group consisting of achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, camptomelic dysplasia, congenital lethal hypophosphatasia, perinatal lethal type of osteogenesis imperfecta, short-rib polydactyly syndromes, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, spondyloepimetaphyseal dysplasia, neurofibromatosis, Legius syndrome, LEOPARD syndrome, Noonan syndrome, hereditary gingival fibromatosis, neurofibromatosis type 1, Legius syndrome, cardiofaciocutaneous syndrome, Costello syndrome, SHOX deficiency, idiopathic short stature, growth hormone deficiency, and osteoarthritis.

In another embodiment the disease is an ophthalmic disorder, such as glaucoma and/or elevated intraocular pressure.

In another embodiment said disease is associated with overactivation of FGFR3 in cancer, e.g., multiple myeloma, myeloproliferative syndrome, leukemia, plasma cell leukemia, lymphoma, glioblastoma, prostate cancer, bladder cancer, or mammary cancer.

In another embodiment said disease is a vascular smooth muscle disorder, preferably selected from the group consisting of hypertension, restenosis, arteriosclerosis, acute decompensated heart failure, congestive heart failure, cardiac edema, nephredema, hepatic edema, acute renal insufficiency, and chronic renal insufficiency.

Preferably said disease is an achondroplasia phenotype selected from the group consisting of growth retardation, skull deformities, orthodontic defects, cervical cord compression, spinal stenosis, hydrocephalus, hearing loss due to chronic otitis, cardiovascular disease, neurological disease, and obesity.

Most preferably said disease is achondroplasia.

In one embodiment the patient undergoing the method of treatment of the present invention is a mammalian patient, preferably a human patient. In one embodiment this human patient is an adult. In a preferred embodiment the human patient is a pediatric patient.

Another aspect of the present invention is the use of the CNP prodrug or a pharmaceutically acceptable salt thereof or the pharmaceutical composition comprising at least one CNP prodrug of the present invention for the manufacture of a medicament for treating a disease which can be treated with CNP.

Preferably, said disease is selected from the group consisting of achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, camptomelic dysplasia, congenital lethal hypophosphatasia, perinatal lethal type of osteogenesis imperfecta, short-rib polydactyly syndromes, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, spondyloepimetaphyseal dysplasia, neurofibromatosis, Legius syndrome, LEOPARD syndrome, Noonan syndrome, hereditary gingival fibromatosis, neurofibromatosis type 1, Legius syndrome, cardiofaciocutaneous syndrome, Costello syndrome, SHOX deficiency, idiopathic short stature, growth hormone deficiency, osteoarthritis, cleidocranial dysostosis, craniosynostosis (e.g., Muenke syndrome, Crouzon syndrome, Apert syndrome, Jackson-Weiss syndrome, Pfeiffer syndrome, or Crouzonodermoskeletal syndrome), dactyly, brachydactyly, camptodactyly, polydactyly, syndactyly, dyssegmental dysplasia, enchondromatosis, fibrous dysplasia, hereditary multiple exostoses, hypophosphatemic rickets, Jaffe-Lichtenstein syndrome, Marfan syndrome, McCune-Albright syndrome, osteopetrosis and osteopoikilosis.

Preferably said disease is selected from the group consisting of achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, camptomelic dysplasia, congenital lethal hypophosphatasia, perinatal lethal type of osteogenesis imperfecta, short-rib polydactyly syndromes, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, spondyloepimetaphyseal dysplasia, neurofibromatosis, Legius syndrome, LEOPARD syndrome, Noonan syndrome, hereditary gingival fibromatosis, neurofibromatosis type 1, Legius syndrome, cardiofaciocutaneous syndrome, Costello syndrome, SHOX deficiency, idiopathic short stature, growth hormone deficiency, and osteoarthritis.

In another embodiment the disease is an ophthalmic disorder, such as glaucoma and/or elevated intraocular pressure.

In another embodiment said disease is associated with overactivation of FGFR3 in cancer, e.g., multiple myeloma, myeloproliferative syndrome, leukemia, plasma cell leukemia, lymphoma, glioblastoma, prostate cancer, bladder cancer, or mammary cancer.

In another embodiment said disease is a vascular smooth muscle disorder, preferably selected from the group consisting of hypertension, restenosis, arteriosclerosis, acute decompensated heart failure, congestive heart failure, cardiac edema, nephredema, hepatic edema, acute renal insufficiency, and chronic renal insufficiency.

Preferably said disease is an achondroplasia phenotype selected from the group consisting of growth retardation, skull deformities, orthodontic defects, cervical cord compression, spinal stenosis, hydrocephalus, hearing loss due to chronic otitis, cardiovascular disease, neurological disease, and obesity.

Most preferably said disease is achondroplasia.

In one embodiment the disease to be treated with the CNP prodrug or a pharmaceutically acceptable salt thereof or the pharmaceutical composition comprising at least one CNP prodrug of the present invention occurs in a mammalian patient, preferably in a human patient. In one embodiment this human patient is an adult. In a preferred embodiment the human patient is a pediatric patient.

A further aspect of the present invention is a method of treating, controlling, delaying or preventing in a mammalian patient, preferably a human patient, in need of the treatment of one or more diseases which can be treated with CNP, comprising the step of administering to said patient in need thereof a therapeutically effective amount of CNP prodrug or a pharmaceutically acceptable salt thereof or a pharmaceutical composition comprising CNP prodrug of the present invention. In one embodiment the human patient is an adult. In a preferred embodiment the human patient is a pediatric patient.

Preferably, the one or more diseases which can be treated with CNP is selected from the group consisting of achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, camptomelic dysplasia, congenital lethal hypophosphatasia, perinatal lethal type of osteogenesis imperfecta, short-rib polydactyly syndromes, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, spondyloepimetaphyseal dysplasia, neurofibromatosis, Legius syndrome, LEOPARD syndrome, Noonan syndrome, hereditary gingival fibromatosis, neurofibromatosis type 1, Legius syndrome, cardiofaciocutaneous syndrome, Costello syndrome, SHOX deficiency, idiopathic short stature, growth hormone deficiency, osteoarthritis, cleidocranial dysostosis, craniosynostosis (e.g., Muenke syndrome, Crouzon syndrome, Apert syndrome, Jackson-Weiss syndrome, Pfeiffer syndrome, or Crouzonodermoskeletal syndrome), dactyly, brachydactyly, camptodactyly, polydactyly, syndactyly, dyssegmental dysplasia, enchondromatosis, fibrous dysplasia, hereditary multiple exostoses, hypophosphatemic rickets, Jaffe-Lichtenstein syndrome, Marfan syndrome, McCune-Albright syndrome, osteopetrosis and osteopoikilosis.

In another embodiment the one or more diseases which can be treated with CNP is an ophthalmic disorder, such as glaucoma and/or elevated intraocular pressure.

In another embodiment the one or more diseases which can be treated with CNP is associated with overactivation of FGFR3 in cancer, e.g., multiple myeloma, myeloproliferative syndrome, leukemia, plasma cell leukemia, lymphoma, glioblastoma, prostate cancer, bladder cancer, or mammary cancer.

In another embodiment the one or more diseases which can be treated with CNP is a vascular smooth muscle disorder, preferably selected from the group consisting of hypertension, restenosis, arteriosclerosis, acute decompensated heart failure, congestive heart failure, cardiac edema, nephredema, hepatic edema, acute renal insufficiency, and chronic renal insufficiency.

Preferably the one or more diseases which can be treated with CNP is an achondroplasia phenotype selected from the group consisting of growth retardation, skull deformities, orthodontic defects, cervical cord compression, spinal stenosis, hydrocephalus, hearing loss due to chronic otitis, cardiovascular disease, neurological disease, and obesity.

Most preferably the one or more diseases which can be treated with CNP is achondroplasia.

An additional aspect of the present invention is a method of administering the CNP prodrug, a pharmaceutically acceptable salt thereof or the pharmaceutical composition of the present invention, wherein the method comprises the step of administering the CNP prodrug, a pharmaceutically acceptable salt thereof or the pharmaceutical composition of the present invention via topical, enteral or parenteral administration and by methods of external application, injection or infusion, including intraarticular, periarticular, intradermal, subcutaneous, intramuscular, intravenous, intraosseous, intraperitoneal, intrathecal, intracapsular, intraorbital, intravitreal, intratympanic, intravesical, intracardiac, transtracheal, subcuticular, subcapsular, subarachnoid, intraspinal, intraventricular, intrasternal injection and infusion, direct delivery to the brain via implanted device allowing delivery of the invention or the like to brain tissue or brain fluids (e.g., Ommaya Reservoir), direct intracerebroventricular injection or infusion, injection or infusion into brain or brain associated regions, injection into the subchoroidal space, retro-orbital injection and ocular instillation, preferably via subcutaneous injection.

In a preferred embodiment, the present invention relates to a CNP prodrug or pharmaceutically acceptable salt thereof or a pharmaceutical composition of the present invention, for use in the treatment of achondroplasia via subcutaneous injection.

Another aspect of the present invention are non-reversible conjugates of formula (IVa) and (IVb): 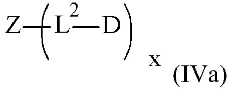 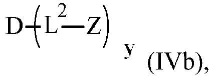 wherein
-D is a CNP moiety;
-L²- is a single chemical bond or a spacer moiety;
-Z is a water-soluble carrier moiety;
x is an integer selected from the group consisting of 1, 2, 3, 4, 5, 6, 7, 8, 9, 10, 11, 12, 13, 14, 15 or 16; and
y is an integer selected from the group consisting of 1, 2, 3, 4 and 5.

Preferred embodiments f -D, -L²-, -Z, x and y are as described above.

The invention is further described by the following non-limiting items.
1. A CNP prodrug or a pharmaceutically acceptable salt thereof, wherein the prodrug is of formula (Ia) or (Ib) 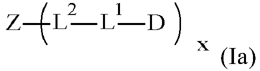 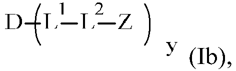 wherein
   -D is a CNP moiety;
   -L¹- is a reversible prodrug linker moiety;
   -L²- is a single chemical bond or a spacer moiety;
   -Z is a water-soluble carrier moiety;
   x is an integer selected from the group consisting of 1, 2, 3, 4, 5, 6, 7, 8, 9, 10, 11, 12, 13, 14, 15 or 16; and
   y is an integer selected from the group consisting of 1, 2, 3, 4 and 5.
2. A CNP prodrug or a pharmaceutically acceptable salt thereof comprising a conjugate D-L, wherein
   -D is a CNP moiety; and
   -L comprises a reversible prodrug linker moiety -L¹-;
   wherein -L¹- is substituted with -L²-Z' and is optionally further substituted; wherein
   -L²- is a single chemical bond or a spacer moiety; and
   -Z' is a water-insoluble carrier moiety.
3. The CNP prodrug or a pharmaceutically acceptable salt thereof of item 2, wherein -Z' is a hydrogel.
4. The CNP prodrug or a pharmaceutically acceptable salt thereof of item 1, wherein the CNP prodrug is of formula (Ia).
5. The CNP prodrug or a pharmaceutically acceptable salt thereof of item 1 or 4, wherein x is 1.
6. The CNP prodrug or a pharmaceutically acceptable salt thereof of any one of items 1 to 5, wherein CNP moiety has the sequence of SEQ ID NO:25 or SEQ ID NO:24.
7. The CNP prodrug or a pharmaceutically acceptable salt thereof of any one of items 1 to 6, wherein the CNP moiety has the sequence of SEQ ID NO:24.
8. The CNP prodrug or a pharmaceutically acceptable salt thereof of any one of items 1 to 7, wherein -L¹- is conjugated to the side chain of an amino acid residue of the ring moiety of -D or to the backbone of the ring moiety of -D.
9. The CNP prodrug or a pharmaceutically acceptable salt thereof of any one of items 1 to 8, wherein -L¹- is conjugated to the side chain of an amino acid residue of the ring moiety of -D selected from the group consisting of histidine, lysine, tryptophan, serine, threonine, tyrosine, aspartic acid, glutamic acid and arginine.
10. The CNP prodrug or a pharmaceutically acceptable salt thereof of any one of items 1 to 9, wherein -D has the sequence of SEQ ID NO:24 and -L¹- is conjugated to the lysine at position 26 of -D.
11. The CNP prodrug or a pharmaceutically acceptable salt thereof of any one of items 1 to 10, wherein the moiety -L¹- is of formula (II): 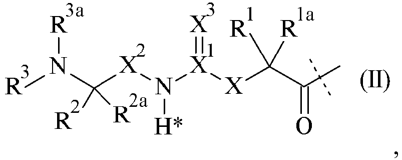
   wherein the dashed line indicates the attachment to a nitrogen of -D which is a CNP moiety by forming an amide bond;
   -X- is -C(R⁴R^{4a})-; -N(R⁴)-; -O-; -C(R⁴R^{4a})-C(R⁵R^{5a})-; -C(R⁵R^{5a})-C(R⁴R^{4a})-; -C(R⁴R^{4a})-N(R⁶)-; -N(R⁶)-C(R⁴R^{4a})-; -C(R⁴R^{4a})-O-; -O-C(R⁴R^{4a})-; or -C(R⁷R^{7a})-;
   X¹ is C; or S(O);
   -X²- is -C(R⁸R^{8a})-; or -C(R⁸R^{8a})-C(R⁹R^{9a})-;
   =X³ is =O; =S; or =N-CN;
   -R¹, -R^{1a}, -R², -R^{2a}, -R⁴, -R^{4a}, -R⁵, -R^{5a}, -R⁶, -R⁸, -R^{8a}, -R⁹, -R^{9a} are independently selected from the group consisting of -H; and C₁₋₆ alkyl;
   -R³, -R^{3a} are independently selected from the group consisting of -H; and C₁₋₆ alkyl, provided that in case one of -R³, -R^{3a} or both are other than -H they are connected to N to which they are attached through an SP³-hybridized carbon atom;
   -R⁷ is -N(R¹⁰R^{10a}); or -NR¹⁰-(C=O)-R¹¹;
   -R^{7a}, -R¹⁰, -R^{10a}, -R¹¹ are independently of each other -H; or C₁₋₆ alkyl;
   optionally, one or more of the pairs -R^{1a}/-R^{4a}, -R^{1a}/-R^{5a}, -R^{1a}/-R^{7a}, -R^{4a}/-R^{5a}, -R^{8a}/-R^{9a} form a chemical bond;
   optionally, one or more of the pairs -R¹/-R^{1a}, -R²/-R^{2a}, -R⁴/-R^{4a}, -R⁵/-R^{5a}, -R⁸/-R^{8a}, -R⁹/-R^{9a} are joined together with the atom to which they are attached to form a C₃₋₁₀ cycloalkyl; or 3- to 10-membered heterocyclyl;
   optionally, one or more of the pairs -R¹/-R⁴, -R¹/-R⁵, -R¹/-R⁶, -R¹/-R^{7a}, -R⁴/-R⁵, -R⁴/-R⁶, -R⁸/-R⁹, -R²/-R³ are joined together with the atoms to which they are attached to form a ring A;
   optionally, R³/R^{3a} are joined together with the nitrogen atom to which they are attached to form a 3- to 10-membered heterocycle;
   A is selected from the group consisting of phenyl; naphthyl; indenyl; indanyl; tetralinyl; C₃₋₁₀ cycloalkyl; 3- to 10-membered heterocyclyl; and 8- to 11-membered heterobicyclyl; and
   wherein -L¹- is substituted with -L²-Z or -L²-Z' and wherein -L¹- is optionally further substituted, provided that the hydrogen marked with the asterisk in formula (II) is not replaced by -L²-Z or -L²-Z' or a substituent;
      wherein
      - -L²-: is a single chemical bond or a spacer;
      - -Z: is a water-soluble carrier; and
      - -Z': is a water-insoluble carrier.
12. The CNP prodrug or a pharmaceutically acceptable salt thereof of item 11, wherein -X- is -C(R⁴R^{4a})- or -N(R⁴)-.
13. The CNP prodrug or a pharmaceutically acceptable salt thereof of item 11 or 12, wherein -R⁴ is substituted with -L²-Z or -L²-Z'.
14. The CNP prodrug or a pharmaceutically acceptable salt thereof of any one of items 11 to 13, wherein X¹ is C.
15. The CNP prodrug or a pharmaceutically acceptable salt thereof of any one of items 11 to 14, wherein =X³ is =O.
16. The CNP prodrug or a pharmaceutically acceptable salt thereof of any one of items 11 to 15, wherein -X²- is -C(R⁸R^{8a})-.
17. The CNP prodrug or a pharmaceutically acceptable salt thereof of any one of items 11 to 16, wherein -R¹ and -R^{1a} are -H.
18. The CNP prodrug or a pharmaceutically acceptable salt thereof of any one of items 11 to 17, wherein -R² and -R^{2a} are -H.
19. The CNP prodrug or a pharmaceutically acceptable salt thereof of any one of items 11 to 18, wherein -R³ is -H and -R^{3a} is methyl.
20. The CNP prodrug or a pharmaceutically acceptable salt thereof of any one of items 11 to 19, wherein -R⁴ and -R^{4a} are -H.
21. The CNP prodrug or a pharmaceutically acceptable salt thereof of any one of items 11 to 20, wherein -R⁸ and -R^{8a} are -H.
22. The CNP prodrug or a pharmaceutically acceptable salt thereof of any one of items 1 to 21, wherein -L²- is selected from the group consisting of -T-, -C(O)O-, -O-, -C(O)-, -C(O)N(R^{y1})-, -S(O)₂N(R^{y1})-, -S(O)N(R^{y1})-, -S(O)₂-, -S(O)-; -N(R^{y1})S(O)₂N(R^{y1a})-, -S-, -N(R^{y1})-, -OC(OR^{y1})(R^{y1a})-, -N(R^{y1})C(O)N(R^{y1a})-, -OC(O)N(R^{y1})-, C₁₋₅₀ alkyl, C₂₋₅₀ alkenyl, and C₂₋₅₀ alkynyl; wherein -T-, C₁₋₅₀ alkyl, C₂₋₅₀ alkenyl, and C₂₋₅₀ alkynyl are optionally substituted with one or more -R^{y2}, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y3})-, -S(O)₂N(R^{y3})-, -S(O)N(R^{y3})-, -S(O)₂-, -S(O)-, -N(R^{y3})S(O)₂N(R^{y3a})-, -S-, -N(R^{y3})-, -OC(OR^{y3})(R^{y3a})-, -N(R^{y3})C(O)N(R^{y3a})-, and -OC(O)N(R^{y3})-;
   -R^{y1} and -R^{y1a} are independently of each other selected from the group consisting of -H, -T, C₁₋₅₀ alkyl, C₂₋₅₀ alkenyl, and C₂₋₅₀ alkynyl; wherein -T, C₁₋₅₀ alkyl, C₂₋₅₀ alkenyl, and C₂₋₅₀ alkynyl are optionally substituted with one or more -R^{y2}, which are the same or different, and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R^{y4})-, -S(O)₂N(R^{y4})-, -S(O)N(R^{y4})-, -S(O)₂-, -S(O)-, -N(R^{y4})S(O)₂N(R^{y4a})-, -S-, -N(R^{y4})-, -OC(OR^{y4})(R^{y4a})-, -N(R^{y4})C(O)N(R^{y4a})-, and -OC(O)N(R^{y4})-;
   each T is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each T is independently optionally substituted with one or more -R^{y2}, which are the same or different;
   each -R^{y2} is independently selected from the group consisting of halogen, -CN, oxo (=O), -COOR^{y5}, -OR^{y5}, -C(O)R^{y5}, -C(O)N(R^{y5}R^{y5a}), -S(O)₂N(R^{y5}R^{y5a}), -S(O)N(R^{y5}R^{y5a}), -S(O)₂R^{y5}, -S(O)R^{y5}, -N(R^{y5})S(O)₂N(R^{y5a}R^{y5b}), -SR^{y5}, -N(R^{y5}R^{y5a}), -NO₂, -OC(O)R^{y5}, -N(R^{y5})C(O)R^{y5a}, -N(R^{y5})S(O)₂R^{y5a}, -N(R^{y5})S(O)R^{y5a}, -N(R^{y5})C(O)OR^{y5a}, -N(R^{y5})C(O)N(R^{y5a}R^{y5b}), -OC(O)N(R^{y5}R^{y5a}), and C₁₋₆ alkyl; wherein C₁₋₆ alkyl is optionally substituted with one or more halogen, which are the same or different; and
   each -R^{y3}, -R^{y3a}, -R^{y4}, -R^{y4a}, -R^{y5}, -R^{y5a} and -R^{y5b} is independently selected from the group consisting of -H, and C₁₋₆ alkyl, wherein C₁₋₆ alkyl is optionally substituted with one or more halogen, which are the same or different.
23. The CNP prodrug or a pharmaceutically acceptable salt thereof of any one of items 1 to 22, wherein -L²- is a C₁₋₂₀ alkyl chain, which is optionally interrupted by one or more groups independently selected from -O-, -T- and -C(O)N(R^{y1})-; and which C₁₋₂₀ alkyl chain is optionally substituted with one or more groups independently selected from -OH, -T and -C(O)N(R^{y6}R^{y6a}); wherein -R^{y1}, -R^{y6}, -R^{y6a} are independently selected from the group consisting of H and C₁₋₄ alkyl and wherein T is selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8- to 30-membered carbopolycyclyl, and 8- to 30-membered heteropolycyclyl.
24. The CNP prodrug or a pharmaceutical acceptable salt thereof of any one of items 1 to 23, wherein -L²- is of formula (i) 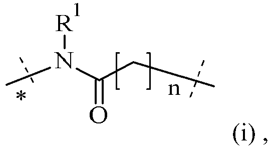 wherein
   the dashed line marked with the asterisk indicates attachment to -L¹-;
   the unmarked dashed line indicates attachment to -Z or -Z';
   -R¹ is selected from the group consisting of -H, C₁₋₆ alkyl, C₂₋₆ alkenyl and C₂₋₆ alkynyl; n is selected from the group consisting of 0, 1, 2, 3, 4, 5, 6, 7, 8, 9, 10, 11, 12, 13, 14, 15, 16, 17 and 18; and
   wherein the moiety of formula (i) is optionally further substituted.
25. The prodrug or pharmaceutically acceptable salt thereof of any one of items 1 or 4 to 24, wherein -Z has a molecular weight ranging from 5 to 200 kDa.
26. The prodrug or a pharmaceutically acceptable salt thereof of any one of items 1 or 4 to 25, wherein the carrier -Z comprises a C₈₋₂₄ alkyl or a polymer.
27. The prodrug or a pharmaceutically acceptable salt thereof of any one of items 1 or 4 to 26, wherein -Z comprises a polymer selected from the group consisting of 2-methacryloyl-oxyethyl phosphoyl cholins, poly(acrylic acids), poly(acrylates), poly(acrylamides), poly(alkyloxy) polymers, poly(amides), poly(amidoamines), poly(amino acids), poly(anhydrides), poly(aspartamides), poly(butyric acids), poly(glycolic acids), polybutylene terephthalates, poly(caprolactones), poly(carbonates), poly(cyanoacrylates), poly(dimethylacrylamides), poly(esters), poly(ethylenes), poly(ethyleneglycols), poly(ethylene oxides), poly(ethyl phosphates), poly(ethyloxazolines), poly(glycolic acids), poly(hydroxyethyl acrylates), poly(hydroxyethyl-oxazolines), poly(hydroxymethacrylates), poly(hydroxypropylmethacrylamides), poly(hydroxypropyl methacrylates), poly(hydroxypropyloxazolines), poly(iminocarbonates), poly(lactic acids), poly(lactic-co-glycolic acids), poly(methacrylamides), poly(methacrylates), poly(methyloxazolines), poly(organophosphazenes), poly(ortho esters), poly(oxazolines), poly(propylene glycols), poly(siloxanes), poly(urethanes), poly(vinyl alcohols), poly(vinyl amines), poly(vinylmethylethers), poly(vinylpyrrolidones), silicones, celluloses, carbomethyl celluloses, hydroxypropyl methylcelluloses, chitins, chitosans, dextrans, dextrins, gelatins, hyaluronic acids and derivatives, functionalized hyaluronic acids, mannans, pectins, rhamnogalacturonans, starches, hydroxyalkyl starches, hydroxyethyl starches and other carbohydrate-based polymers, xylans, and copolymers thereof.
28. The CNP prodrug or a pharmaceutically acceptable salt thereof of any one of items 1 or 4 to 27, wherein -Z is a branched polymer.
29. The CNP prodrug or a pharmaceutically acceptable salt thereof of any one of items 1 or 4 to 28, wherein -Z has a molecular weight of at least 10 kDa.
30. The CNP prodrug or a pharmaceutically acceptable salt thereof of any one of items 1 to 29, wherein -Z or -Z' comprises a moiety 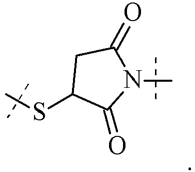
31. The CNP prodrug or a pharmaceutically acceptable salt thereof of any one of items 1 or 4 to 30, wherein -Z comprises a moiety of formula (a) 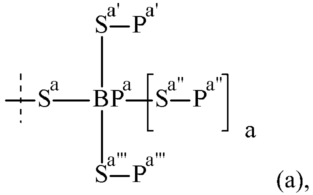 wherein
   the dashed line indicates attachment to -L²- or to the remainder of -Z;
   BP^{a} is a branching point selected from the group consisting of -N<, -CR< and >C<;
   -R is selected from the group consisting of -H and C₁₋₆ alkyl;
   a is 0 if BP^{a} is -N< or -CR< and n is 1 if BP^{a} is >C<;
   -S^{a}-, -S^{a'}-, -S^{a"}- and -S^{a‴}- are independently of each other a chemical bond or are selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
   each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each -T- is independently optionally substituted with one or more -R¹, which are the same or different;
   each -R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}, -OC(O)N(R³R^{3a}), and C₁₋₆ alkyl; wherein C₁₋₆ alkyl is optionally substituted with one or more halogen, which are the same or different;
   each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different; and
   -P^{a'}, -P^{a"} and -P^{a‴} are independently a polymeric moiety.
32. The CNP prodrug or a pharmaceutically acceptable salt thereof of any one of items 1 or 4 to 31, wherein -Z is of formula (d) 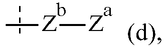 wherein
   the dashed line indicates attachment to -L²-;
   -Z^{b}- is selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
      each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8-to 30-membered carbopolycyclyl, and 8- to 30-membered heteropolycyclyl; wherein each -T- is independently optionally substituted with one or more -R¹, which are the same or different;
      each -R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}), -OC(O)N(R³R^{3a}), and C₁₋₆ alkyl; wherein C₁₋₆ alkyl is optionally substituted with one or more halogen, which are the same or different;
      each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different;
         and
   -Z^{a} is 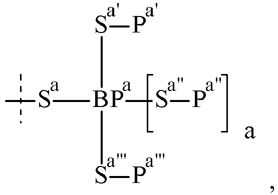
   wherein
   BP^{a}, -S^{a}-, -S^{a'}-, -S^{a"}-, -S^{a‴}-, -P^{a'}, -P^{a"}, -P^{a‴} and a are used as defined in item 31.
33. The CNP prodrug or a pharmaceutically acceptable salt thereof of any one of items 1 or 4 to 32, wherein -Z is of formula (e) 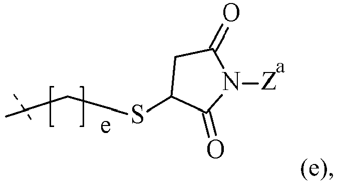 wherein
   the dashed line indicates attachment to -L²-;
   e is selected from the group consisting of 1, 2, 3, 4, 5, 6, 7, 8, 9, 10, 11, 12, 13, 14 and 15; and
   -Z^{a} is 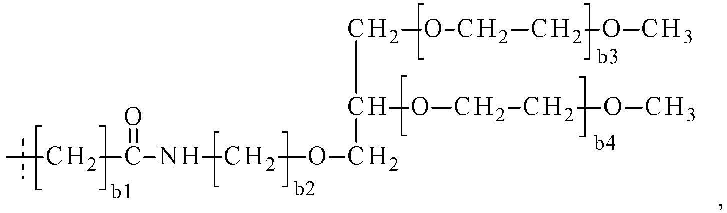 wherein
   b1 is selected from the group consisting of 0, 1, 2, 3, 4, 5, 6, 7 and 8;
   b2 is selected from the group consisting of 1, 2, 3, 4, 5, 6, 7 and 8;
   b3 is an integer ranging from and including 150 to 1000; and
   b4 is an integer ranging from and including 150 to 1000.
34. The CNP prodrug or a pharmaceutically acceptable salt thereof of item 33, wherein e is 5, b1 is 2, b2 is 3 and b3 and b4 are both about 450.
35. The CNP prodrug or a pharmaceutically acceptable salt thereof of any one of items 1 or 4 to 31, wherein -Z is of formula (f) 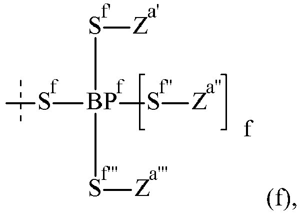 wherein
   the dashed line indicates attachment to -L²-;
   BP^{f} is a branching point selected from the group consisting of -N<, -CR< and >C<;
   -R is selected from the group consisting of -H and C₁₋₆ alkyl;
   f is 0 if BP^{f} is -N< or -CR< and f is 1 if BP^{f} is >C<;
   -S^{f}-, -S^{f'}-, -S^{f"}- and -S^{f‴}- are independently either a chemical bond or are independently selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
      each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8- to 30-membered carbopolycyclyl, and 8- to 30-membered heteropolycyclyl; wherein each -T- is independently optionally substituted with one or more -R¹, which are the same or different;
      each R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}, -OC(O)N(R³R^{3a}), and C₁₋₆ alkyl; wherein C₁₋₆ alkyl is optionally substituted with one or more halogen, which are the same or different;
      each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different;
         and
   -Z^{a'}, -Z^{a"} and -Z^{a‴} are independently 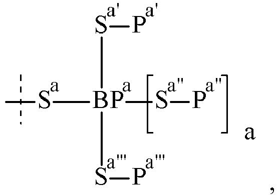 wherein
      BP^{a}, -S^{a}-, -S^{a'}-, -S^{a"}-, -S^{a‴}-, -P^{a'}, -P^{a"}, -P^{a‴} and a are used as defined in item 31.
36. The CNP prodrug or a pharmaceutically acceptable salt thereof of any one of items 1, 4 to 31 or 35, wherein -Z is of formula (g) 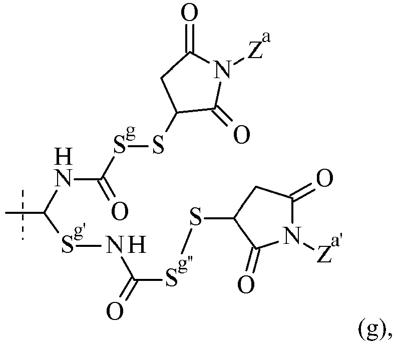 wherein
   the dashed line indicates attachment to -L²-;
   -S^{g}-, -S^{g'}- and -S^{g"}- are independently selected from the group consisting of C₁₋₅₀ alkyl, C₂₋₅₀ alkenyl, and C₂₋₅₀ alkynyl; wherein C₁₋₅₀ alkyl, C₂₋₅₀ alkenyl, and C₂₋₅₀ alkynyl are optionally substituted with one or more -R¹, which are the same or different and wherein C₁₋₅₀ alkyl, C₂₋₅₀ alkenyl, and C₂₋₅₀ alkynyl are optionally interrupted by one or more groups selected from the group consisting of -T-, -C(O)O-, -O-, -C(O)-, -C(O)N(R²)-, -S(O)₂N(R²)-, -S(O)N(R²)-, -S(O)₂-, -S(O)-, -N(R²)S(O)₂N(R^{2a})-, -S-, -N(R²)-, -OC(OR²)(R^{2a})-, -N(R²)C(O)N(R^{2a})-, and -OC(O)N(R²)-;
      each -T- is independently selected from the group consisting of phenyl, naphthyl, indenyl, indanyl, tetralinyl, C₃₋₁₀ cycloalkyl, 3- to 10-membered heterocyclyl, 8- to 11-membered heterobicyclyl, 8- to 30-membered carbopolycyclyl, and 8- to 30-membered heteropolycyclyl; wherein each -T- is independently optionally substituted with one or more -R¹, which are the same or different;
      each R¹ is independently selected from the group consisting of halogen, -CN, oxo (=O), -COOR³, -OR³, -C(O)R³, -C(O)N(R³R^{3a}), -S(O)₂N(R³R^{3a}), -S(O)N(R³R^{3a}), -S(O)₂R³, -S(O)R³, -N(R³)S(O)₂N(R^{3a}R^{3b}), -SR³, -N(R³R^{3a}), -NO₂, -OC(O)R³, -N(R³)C(O)R^{3a}, -N(R³)S(O)₂R^{3a}, -N(R³)S(O)R^{3a}, -N(R³)C(O)OR^{3a}, -N(R³)C(O)N(R^{3a}R^{3b}, -OC(O)N(R³R^{3a}), and C₁₋₆ alkyl; wherein C₁₋₆ alkyl is optionally substituted with one or more halogen, which are the same or different;
      each -R², -R^{2a}, -R³, -R^{3a} and -R^{3b} is independently selected from the group consisting of -H, and C₁₋₆ alkyl, wherein C₁₋₆ alkyl is optionally substituted with one or more halogen, which are the same or different;
         and
   -Z^{a} and -Z^{a'} are independently 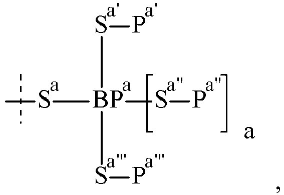 wherein BP^{a}, -S^{a}-, -S^{a'}-, -S^{a"}-, -S^{a‴}-, -P^{a'}, -P^{a"}, -P^{a‴} and a are used as defined in item 31.
37. The CNP prodrug or a pharmaceutically acceptable salt thereof of any one of items 1, 4 to 31, 35 or 36, wherein -Z is of formula (h) 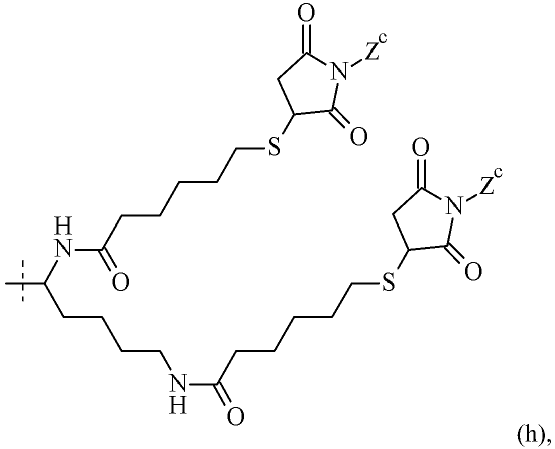 wherein
   the dashed line indicates attachment to -L²-; and
   each -Z^{c} is a moiety 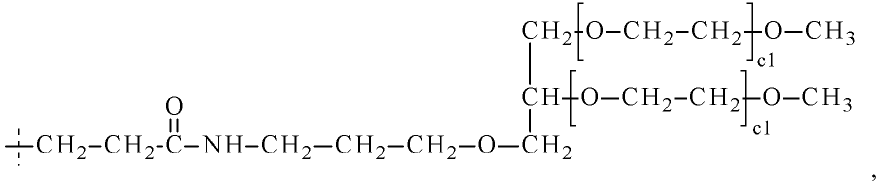 wherein
      each c1 is an integer independently ranging from about 200 to 250.
38. The CNP prodrug or a pharmaceutically acceptable salt thereof of any one of items 1 or 4 to 34, wherein the CNP prodrug is of formula (IIe) 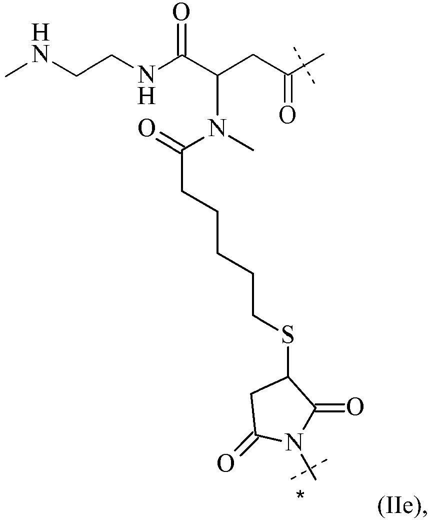 wherein
   the unmarked dashed line indicates the attachment to a nitrogen of -D which is a CNP moiety by forming an amide bond; and
   the dashed line marked with the asterisk indicates attachment to a moiety 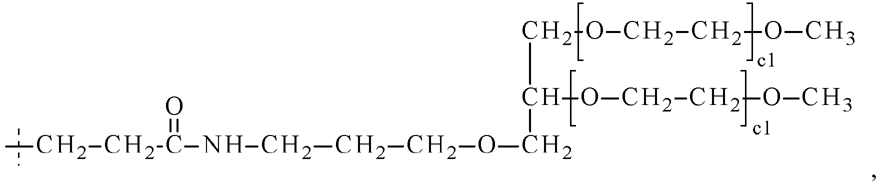 wherein
      each c1 is an integer independently ranging from 400 to 500.
39. The CNP prodrug or a pharmaceutically acceptable salt thereof of any one of items 1, 4 to 34 or 38, wherein the CNP prodrug is of formula (IIe') 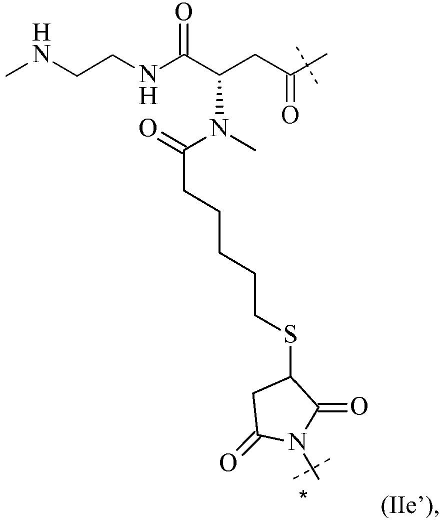 wherein
   the unmarked dashed line indicates the attachment to a nitrogen provided by the side chain of the lysine at position 26 of the CNP moiety of SEQ ID NO:24 by forming an amide bond; and
   the dashed line marked with the asterisk indicates attachment to a moiety 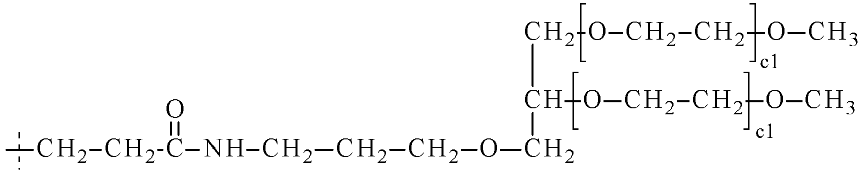
   wherein
   each c1 is an integer independently ranging from 400 to 500.
40. The CNP prodrug or a pharmaceutically acceptable salt thereof of any one of items 1, 4 to 34, wherein the CNP prodrug is of formula (IIe-i') 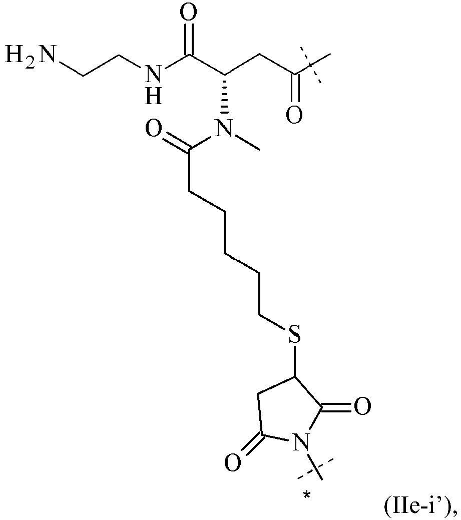 wherein
   the unmarked dashed line indicates the attachment to a nitrogen provided by the side chain of the lysine at position 26 of the CNP moiety of SEQ ID NO:24 by forming an amide bond; and
   the dashed line marked with the asterisk indicates attachment to a moiety 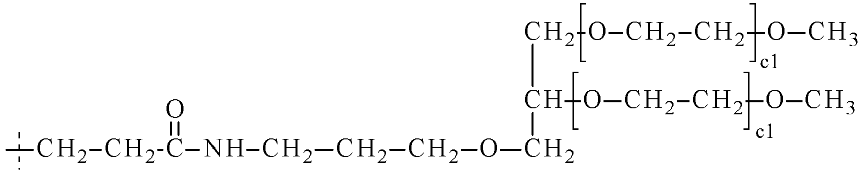
   wherein
   each c1 is an integer independently ranging from 400 to 500.
41. The CNP prodrug or a pharmaceutically acceptable salt thereof of any one of items 1, 4 to 34, wherein the CNP prodrug is of formula (IIe-ii') 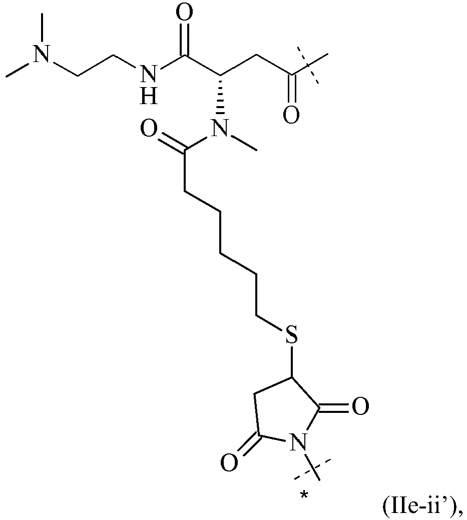 wherein
   the unmarked dashed line indicates the attachment to a nitrogen provided by the side chain of the lysine at position 26 of the CNP moiety of SEQ ID NO:24 by forming an amide bond; and
   the dashed line marked with the asterisk indicates attachment to a moiety 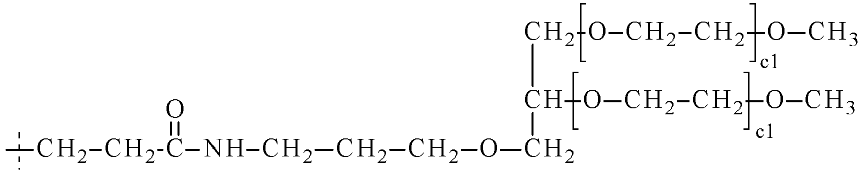
   wherein
   each c1 is an integer independently ranging from 400 to 500.
42. The CNP prodrug or a pharmaceutically acceptable salt thereof of any one of items 1, 4 to 31 or 35 to 37, wherein the CNP prodrug is of formula (IIf) 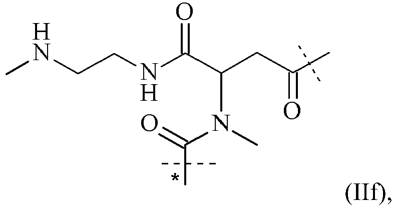 wherein
   the unmarked dashed line indicates the attachment to a nitrogen of -D which is a CNP moiety by forming an amide bond; and
   the dashed line marked with the asterisk indicates attachment to -Z having the structure 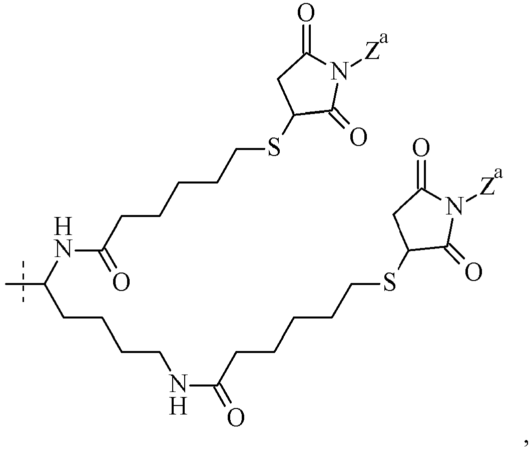 wherein
      each -Z^{a} is 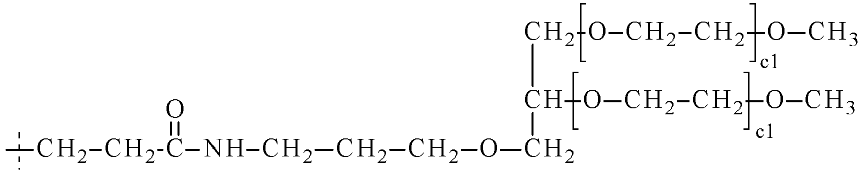 wherein
      each c1 is an integer independently ranging from 200 to 250.
43. The CNP prodrug or a pharmaceutically acceptable salt thereof of any one of items 1, 4 to 31, 35 to 37 or 42, wherein the CNP prodrug is of formula (IIf') 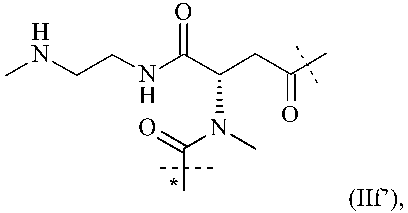 wherein
   the unmarked dashed line indicates the attachment to a nitrogen provided by the side chain of the lysine at position 26 of the CNP moiety of SEQ ID NO:24 by forming an amide bond; and
   the dashed line marked with the asterisk indicates attachment to -Z having the structure 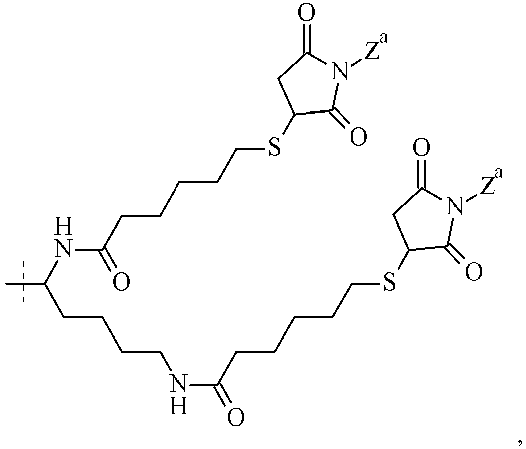 wherein
      each Z^{a} is 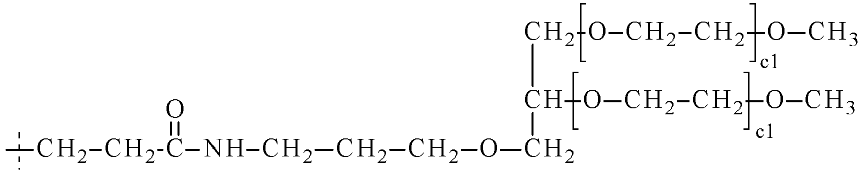 wherein
      each c1 is an integer independently ranging from 200 to 250.
44. The CNP prodrug or a pharmaceutically acceptable salt thereof of any one of items 1, 4 to 31 or 35 to 37, wherein the CNP prodrug is of formula (IIf-i') 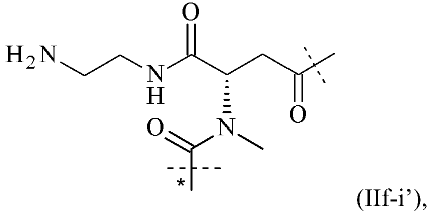 wherein
   the unmarked dashed line indicates the attachment to a nitrogen provided by the side chain of the lysine at position 26 of the CNP moiety of SEQ ID NO:24 by forming an amide bond; and
   the dashed line marked with the asterisk indicates attachment to -Z having the structure 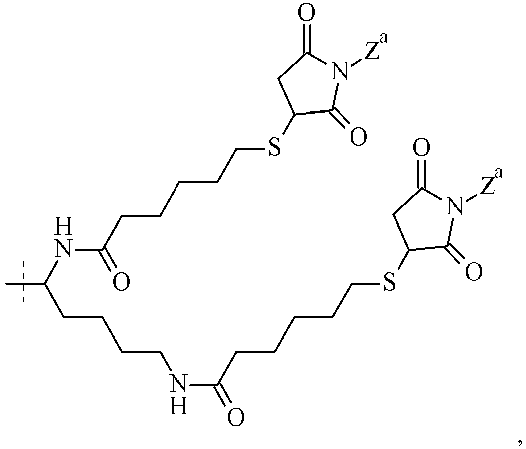 wherein
      each Z^{a} is 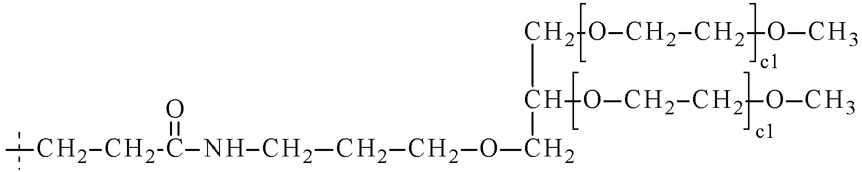 wherein
   each c1 is an integer independently ranging from 200 to 250.
45. The CNP prodrug or a pharmaceutically acceptable salt thereof of any one of items 1, 4 to 31 or 35 to 37, wherein the CNP prodrug is of formula (IIf-ii') 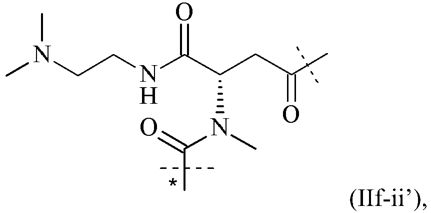 wherein
   the unmarked dashed line indicates the attachment to a nitrogen provided by the side chain of the lysine at position 26 of the CNP moiety of SEQ ID NO:24 by forming an amide bond; and
   the dashed line marked with the asterisk indicates attachment to -Z having the structure 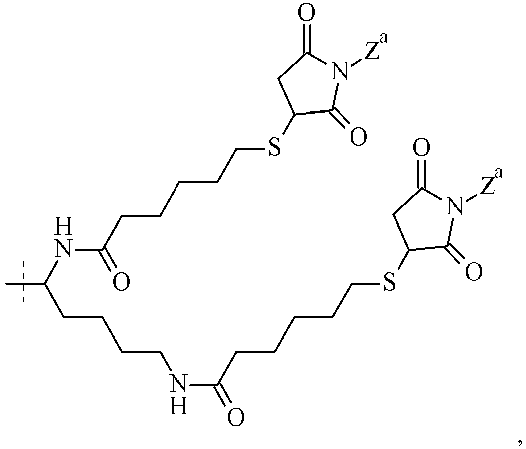 wherein
      each Z^{a} is 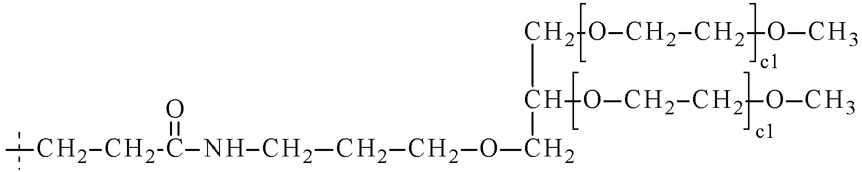 wherein
      each c1 is an integer independently ranging from 200 to 250.
46. The prodrug or pharmaceutically acceptable salt thereof of any one of items 1 to 45, wherein the residual activity of the CNP prodrug is less than 10%.
47. A pharmaceutical composition comprising at least one CNP prodrug or pharmaceutically acceptable salt thereof of any one of items 1 to 46 and at least one excipient.
48. The pharmaceutical composition of item 47, wherein the pharmaceutical composition has a pH ranging from and including pH 4 to pH 6.
49. Use of the prodrug or pharmaceutically acceptable salt thereof of any one of items 1 to 46 or the pharmaceutical composition of item 47 or 48 as a medicament.
50. Use of the prodrug or pharmaceutically acceptable salt thereof of any one of items 1 to 46 or the pharmaceutical composition of item 47 or 48 in a method of treatment of a disease which can be treated with CNP.
51. The use of item 50, wherein the disease is selected from the group consisting of achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, camptomelic dysplasia, congenital lethal hypophosphatasia, perinatal lethal type of osteogenesis imperfecta, short-rib polydactyly syndromes, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, spondyloepimetaphyseal dysplasia, neurofibromatosis, Legius syndrome, LEOPARD syndrome, Noonan syndrome, hereditary gingival fibromatosis, neurofibromatosis type 1, Legius syndrome, cardiofaciocutaneous syndrome, Costello syndrome, SHOX deficiency, idiopathic short stature, growth hormone deficiency, osteoarthritis, cleidocranial dysostosis, craniosynostosis (e.g., Muenke syndrome, Crouzon syndrome, Apert syndrome, Jackson-Weiss syndrome, Pfeiffer syndrome, or Crouzonodermoskeletal syndrome), dactyly, brachydactyly, camptodactyly, polydactyly, syndactyly, dyssegmental dysplasia, enchondromatosis, fibrous dysplasia, hereditary multiple exostoses, hypophosphatemic rickets, Jaffe-Lichtenstein syndrome, Marfan syndrome, McCune-Albright syndrome, osteopetrosis and osteopoikilosis.
52. The use of item 50, wherein the disease is an ophthalmic disorder.
53. The use of item 50 or 51, wherein the disease is achondroplasia.
54. Use of the CNP prodrug or the pharmaceutically acceptable salt thereof of any one of items 1 to 46 or the pharmaceutical composition of item 47 or 48 for the manufacture of a medicament for treating a disease which can be treated with CNP.
55. The use of item 54, wherein the disease is selected from the group consisting of achondroplasia, hypochondroplasia, short stature, dwarfism, osteochondrodysplasias, thanatophoric dysplasia, osteogenesis imperfecta, achondrogenesis, chondrodysplasia punctata, homozygous achondroplasia, camptomelic dysplasia, congenital lethal hypophosphatasia, perinatal lethal type of osteogenesis imperfecta, short-rib polydactyly syndromes, rhizomelic type of chondrodysplasia punctata, Jansen-type metaphyseal dysplasia, spondyloepiphyseal dysplasia congenita, atelosteogenesis, diastrophic dysplasia, congenital short femur, Langer-type mesomelic dysplasia, Nievergelt-type mesomelic dysplasia, Robinow syndrome, Reinhardt syndrome, acrodysostosis, peripheral dysostosis, Kniest dysplasia, fibrochondrogenesis, Roberts syndrome, acromesomelic dysplasia, micromelia, Morquio syndrome, Kniest syndrome, metatrophic dysplasia, spondyloepimetaphyseal dysplasia, neurofibromatosis, Legius syndrome, LEOPARD syndrome, Noonan syndrome, hereditary gingival fibromatosis, neurofibromatosis type 1, Legius syndrome, cardiofaciocutaneous syndrome, Costello syndrome, SHOX deficiency, idiopathic short stature, growth hormone deficiency, osteoarthritis, cleidocranial dysostosis, craniosynostosis (e.g., Muenke syndrome, Crouzon syndrome, Apert syndrome, Jackson-Weiss syndrome, Pfeiffer syndrome, or Crouzonodermoskeletal syndrome), dactyly, brachydactyly, camptodactyly, polydactyly, syndactyly, dyssegmental dysplasia, enchondromatosis, fibrous dysplasia, hereditary multiple exostoses, hypophosphatemic rickets, Jaffe-Lichtenstein syndrome, Marfan syndrome, McCune-Albright syndrome, osteopetrosis and osteopoikilosis.
56. The use of item 54, wherein the disease is an ophthalmic disorder.
57. The use of item 54 or 55, wherein the disease is achondroplasia.
58. A method of administering the CNP prodrug or pharmaceutically acceptable salt thereof of any one of items 1 to 46 or the pharmaceutical composition of item 47 or 48, wherein the method comprises the step of administering the CNP prodrug or pharmaceutically acceptable salt thereof or the pharmaceutical composition via topical, enteral or parenteral administration or by methods of external application, injection or infusion, including intraarticular, periarticular, intradermal, subcutaneous, intramuscular, intravenous, intraosseous, intraperitoneal, intrathecal, intracapsular, intraorbital, intravitreal, intratympanic, intravesical, intracardiac, transtracheal, subcuticular, subcapsular, subarachnoid, intraspinal, intraventricular, intrasternal injection and infusion, direct delivery to the brain via implanted device allowing delivery of the invention or the like to brain tissue or brain fluid, direct intracerebroventricular injection or infusion, injection or infusion into brain or brain associated regions, injection into the subchoroidal space, retro-orbital injection or ocular instillation.
59. The CNP prodrug or the pharmaceutically acceptable salt thereof of any one of items 1 to 46 or the pharmaceutical composition of item 47 or 48 for use in the treatment of achondroplasia via subcutaneous injection.

**Fig. 1****:** Structure of CNP according to SEQ ID NO:1.

### Examples

### Materials and Methods

CNP SEQ ID No:1 was obtained from Bachem AG, Bubendorf, Switzerland (CNP-22, human, catalogue no. H-1296). CNP-34 SEQ ID No:40 and CNP-38 SEQ ID No:24 were obtained from CASLO ApS, Kongens Lyngby, Denmark.

Side chain protected CNP-38 on TCP resin having Boc protected N-terminus and ivDde protected side chain of Lys26 (synthesized by Fmoc-strategy) was obtained from CASLO ApS, Kongens Lyngby, Denmark.

Side chain protected CNP-34 on TCP Tentagel resin having Boc protected N-terminus and ivDde protected side chain of either Lys12, Lys16 or Lys22 (synthesized by Fmoc-strategy) was obtained from Peptide Specialty Laboratories GmbH, Heidelberg, Germany. Side chain protected CNP-38 on TCP tentagel resin having free N-terminus (synthesized by Fmoc-strategy) was obtained from Peptide Specialty Laboratories GmbH, Heidelberg, Germany. Methoxy PEG amine 5 kDa was obtained from Rapp Rapp Polymere GmbH, Tuebingen, Germany. All other PEGs used in this work were acquired from NOF Europe N.V., Grobbendonk, Belgium.

FmocN-Me-Asp(OtBu)-OH was obtained from Bachem AG, Bubendorf, Switzerland. S-Trityl-6-mercaptohexanoic acid was purchased from Polypeptide, Strasbourg, France. HATU was obtained from Merck Biosciences GmbH, Schwalbach/Ts, Germany.

2,4-Dimethylbenzyl alcohol was obtained from aber GmbH, Karlsruhe, Germany. Fmoc-N-Me-Asp(OBn)-OH was obtained from Peptide International Inc., Louisville, KY, USA.

Neutral Endopeptidase (NEP) was obtained from Enzo Life Sciences GmbH, Lörrach, Germany All other chemicals and reagents were purchased from Sigma Aldrich GmbH, Taufkirchen, Germany.

Syringes equipped with polyethylenene frits (MultiSynTech GmbH, Witten, Germany) were used as reaction vessels or for washing steps for peptide resins.

General procedure for the removal of ivDde protecting group from side chain protected CNPs on resin

The resin was pre-swollen in DMF for 30 min and the solvent was discarded. The ivDde group was removed by incubating the resin with DMF/hydrazine hydrate 4/1 (v/v, 2.5 mL/g resin) for 8 x 15 min. For each step fresh DMF/hydrazine hydrate solution was used. Finally, the resin was washed with DMF (10 x), DCM (10 x) and dried *in vacuo.*

### RP-HPLC purification:

For preparative RP-HPLC a Waters 600 controller and a 2487 Dual Absorbance Detector was used, equipped with the following columns: Waters XBridge^{™} BEH300 Prep C18 5 µm, 150 x 10 mm, flow rate 6 mL/min, or Waters XBridge^{™} BEH300 Prep C18 10 µm, 150 x 30 mm, flow rate 40 mL/min. Linear gradients of solvent system A (water containing 0.1 % TFA v/v or 0.01 % conc. HCl v/v) and solvent system B (acetonitrile containing 0.1 % TFA v/v or 0.01 % conc. HCl v/v) were used.

HPLC fractions containing product were pooled and lyophilized if not stated otherwise.

### Flash Chromatography

Flash chromatography purifications were performed on an Isolera One system from Biotage AB, Sweden, using Biotage KP-Sil silica cartridges and n-heptane and ethyl acetate as eluents. Products were detected at 254 nm.

### Analytical methods

Analytical ultra-performance LC (UPLC)-MS was performed on a Waters Acquity system equipped with a Waters BEH300 C18 column (2.1 x 50 mm, 1.7 µm particle size, flow: 0.25 mL/min; solvent A: water containing 0.04% TFA (v/v), solvent B: acetonitrile containing 0.05% TFA (v/v)) coupled to a LTQ Orbitrap Discovery mass spectrometer from Thermo Scientific or coupled to a Waters Micromass ZQ.

Size exclusion chromatography (SEC) was performed using an Amersham Bioscience AEKTAbasic system equipped with a Superdex 200 5/150 GL column (Amersham Bioscience/GE Healthcare) equipped with a 0.45 µm inlet filter, if not stated otherwise. 20 mM sodium phosphate, 140 mM NaCl, pH 7.4, was used as mobile phase.

Due to the reversible nature of the attachment of -L¹- to -D measurements for NEP-stability and receptor affinity were made using stable analogs of the CNP prodrugs of the present invention, i.e. they were made using similar structures to those of the CNP prodrugs of the present invention which instead of a reversible attachment of -Z to -D have a stable attachment.

This was necessary, because the CNP prodrugs of the present invention would release CNP in the course of the experiment and said released CNP would have influenced the result.

### Quantification of plasma total CNP-38 concentrations

Plasma total CNP-38 concentrations were determined by quantification of the N-terminal signature peptide (sequence: LQEHPNAR) and C-terminal signature peptide (sequence: IGSMSGLGC) after tryptic digestion.

LC-MS analysis was carried out by using an Agilent 1290 UPLC coupled to an Agilent 6550 iFunnel Q-TOF mass spectrometer via an ESI probe. Chromatography was performed on a Waters Acquity BEH300 C18 analytical column (50 x 2.1 mm I.D., 1.7 µm particle size) with pre-filter at a flow rate of 0.25 mL/min (T = 25 °C). Water (UPLC grade) containing 0.2 % formic acid (v/v) was used as mobile phase A and acetonitrile (UPLC grade) with 0.2 % formic acid as mobile phase B. The gradient system comprised a short isocratic step at the initial parameters of 0.1 % B for 3.0 min followed by a linear increase from 0.1 % B to 16 % B in 17 min. Mass analysis was performed in the single ion monitoring (SIM) mode, monitoring the ions m/z 482.75 [M+2H]²⁺ (N-terminal) and m/z 824.36 [M+H]¹⁺ (C-terminal). As internal standard deuterated CNP-38 peptide was used.

Calibration standards of CNP-38 conjugate in blank plasma were prepared as follows: The thawed Li-heparin cynomolgous plasma was first homogenized, then centrifuged for 5 minutes. The CNP-38 conjugate formulation was diluted to a working solution of 10 µg/mL (conjugate CNP-38 eq.) in DMSO and spiked into blank plasma at concentrations between 9.3 ng/100 µL (conjugate CNP-38 eq.) and 139.5 ng/100 µL (conjugate CNP-38 eq.). These solutions were used for the generation of a calibration curve. Calibration curves were weighted 1/x² for both signature peptides (N- and C-Terminal). For quality control, three quality control samples were prepared accordingly with contents of 116.2 ng/100 µL (high QC, conjugate CNP-38 eq.), 69.75 ng/100 µL (mid QC, conjugate CNP-38 eq.) and 23.25 ng /100 µL (low QC, conjugate CNP-38 eq.).

For sample preparation, protein precipitation was carried out by addition of 300 µL of precooled (0 °C) methanol to 100 µL of the plasma sample. 200 µL of the supernatant were transferred into a new well-plate and evaporated to dryness (under a gentle nitrogen stream at 35 °C). 100 µL of reconstitution solvent (Thermo digestion buffer, order number 60109-101, Thermo Fisher Scientific GmbH, Dreieich, Germany) were used to dissolve the residue. 20 µg of trypsin (order number V5111, Promega GmbH, Mannheim, Germany) were dissolved in 20 µL of 10 mM acetic acid. 2 µL of the trypsin solution were added to each cavity.

After 4 hours incubation at 37 °C (water bath), 5 µL of a 0.5 M TCEP solution were added to each cavity and incubated again for 5 min at 96 °C. After the samples had cooled to room temperature, 3 µL acetonitrile were added. The eluates were transferred into vials. 10 µL were injected into the UPLC-MS system.

### Example 1

### Synthesis of linker reagent 1f

Linker reagent **1f** was synthesized according to the following scheme:

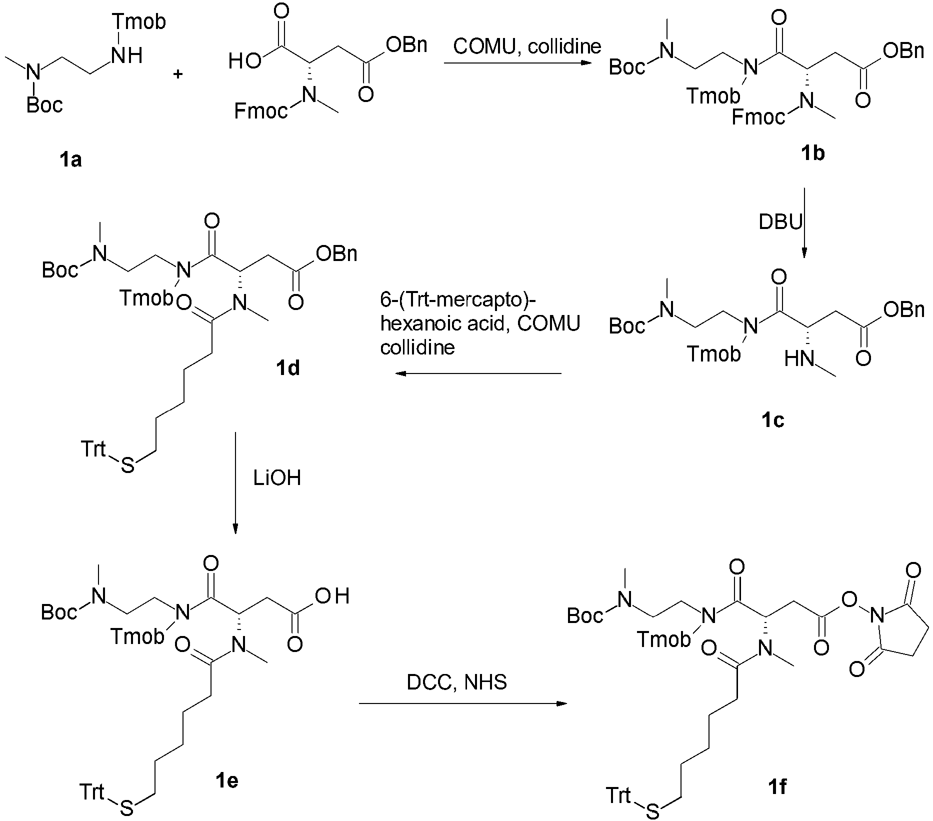

To a solution of N-methyl-N-Boc-ethylenediamine (2 g, 11.48 mmol) and NaCNBH₃ (819 mg, 12.63 mmol) in MeOH (20 mL) was added 2,4,6-trimethoxybenzaldehyde (2.08 g, 10.61 mmol) portion wise. The mixture was stirred at rt for 90 min, acidified with 3 M HCl (4 mL) and stirred further 15 min. The reaction mixture was added to saturated NaHCO₃ solution (200 mL) and extracted 5 x with CH₂Cl₂. The combined organic phases were dried over Na₂SO₄ and the solvents were evaporated *in vacuo.* The resulting N-methyl-N-Boc-N'-Tmob-ethylenediamine **1a** was dried in high vacuum and used in the next reaction step without further purification. Yield: 3.76 g (11.48 mmol, 89 % purity, **1a** : double Tmob protected product = 8 :1) MS: m/z 355.22 = [M+H]⁺, (calculated monoisotopic mass =354.21.

To a solution of **1a** (2 g, 5.65 mmol) in CH₂Cl₂ (24 mL) COMU (4.84 g, 11.3 mmol), N-Fmoc-N-Me-Asp(OBn)-OH (2.08 g, 4.52 mmol) and 2,4,6-collidine (2.65 mL, 20.34 mmol) were added. The reaction mixture was stirred for 3 h at rt, diluted with CH₂Cl₂ (250 mL) and washed 3 x with 0.1 M H₂SO₄ (100 mL) and 3 x with brine (100 mL). The aqueous phases were re-extracted with CH₂Cl₂ (100 mL). The combined organic phases were dried over Na₂SO₄, filtrated and the residue concentrated to a volume of 24 mL. **1b** was purified using flash chromatography.

| | |
|---|---|
| Yield: | 5.31 g (148 %, 6.66 mmol) |
| MS: | m/z 796.38 = [M+H]⁺, (calculated monoisotopic mass =795.37). |

To a solution of **1b** (5.31 g, max. 4.52 mmol ref. to N-Fmoc-N-Me-Asp(OBn)-OH) in THF (60 mL) DBU (1.8 mL, 3 % v/v) was added. The solution was stirred for 12 min at rt, diluted with CH₂Cl₂ (400 mL) and washed 3 x with 0.1 M H₂SO₄ (150 mL) and 3 x with brine (150 mL). The aqueous phases were re-extracted with CH₂Cl₂ (100 mL). The combined organic phases were dried over Na₂SO₄ and filtrated. **1c** was isolated upon evaporation of the solvent and used in the next reaction without further purification.

| | |
|---|---|
| MS: | m/z 574.31 = [M+H]⁺, (calculated monoisotopic mass = 573.30). |

**1c** (5.31 g, 4.52 mmol, crude) was dissolved in acetonitrile (26 mL) and COMU (3.87 g, 9.04 mmol), 6-tritylmercaptohexanoic acid (2.12 g, 5.42 mmol) and 2,4,6-collidine (2.35 mL, 18.08 mmol) were added. The reaction mixture was stirred for 4 h at rt, diluted with CH₂Cl₂ (400 mL) and washed 3 x with 0.1 M H₂SO₄ (100 mL) and 3 x with brine (100 mL). The aqueous phases were re-extracted with CH₂Cl₂ (100 mL). The combined organic phases were dried over Na₂SO₄, filtrated and **1d** was isolated upon evaporation of the solvent. Product **1d** was purified using flash chromatography.

| | |
|---|---|
| Yield: | 2.63 g (62 %, 94 % purity) |
| MS: | m/z 856.41 = [M+H]⁺, (calculated monoisotopic mass = 855.41). |

To a solution of **1d** (2.63 g, 2.78 mmol) in i-PrOH (33 mL) and H₂O (11 mL) was added LiOH (267 mg, 11.12 mmol) and the reaction mixture was stirred for 70 min at rt. The mixture was diluted with CH₂Cl₂ (200 mL) and washed 3 x with 0.1 M H₂SO₄ (50 mL) and 3 x with brine (50 mL). The aqueous phases were re-extracted with CH₂Cl₂ (100 mL). The combined organic phases were dried over Na₂SO₄, filtrated and **1e** was isolated upon evaporation of the solvent. **1e** was purified using flash chromatography.

| | |
|---|---|
| Yield: | 2.1 g (88 %) |
| MS: | m/z 878.4 = [M+Na]⁺, (calculated monoisotopic mass = 837.40). |

To a solution of **1e** (170 mg, 0.198 mmol) in anhydrous DCM (4 mL) were added DCC (123 mg, 0.59 mmol), and a catalytic amount of DMAP. After 5 min N-hydroxy-succinimide (114 mg, 0.99 mmol) was added and the reaction mixture was stirred at rt for 1 h. The reaction mixture was filtered, the solvent was removed *in vacuo* and the residue was taken up in 90 % acetonitrile plus 0.1 % TFA (3.4 mL). The crude mixture was purified by RP-HPLC. Product fractions were neutralized with 0.5 M pH 7.4 phosphate buffer and concentrated. The remaining aqueous phase was extracted with DCM and **1f** was isolated upon evaporation of the solvent.

| | |
|---|---|
| Yield: | 154 mg (81%) |
| MS: | m/z 953.4 = [M+H]⁺, (calculated monoisotopic mass = 952.43). |

### Example 2

### Synthesis of N^{εK4/εK10}-CNP mono-linker thiol 2, N^{εK4}-CNP mono-linker thiol 2c and N^{εK10}-CNP mono-linker thiol 2d

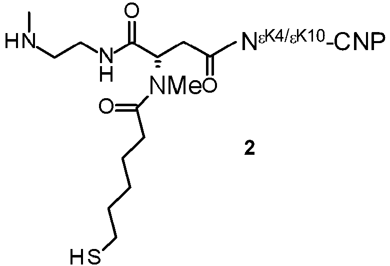

N^{εK4/εK10}-CNP mono-linker thiol (mixture of regioisomers with linker conjugated at side chain amino group of Lys4 or Lys10) 2 is prepared by dissolving CNP-22 (5.2 µmol) in 0.6 mL DMSO. 0.15 mL 0.375 M borate buffer, adjusted to pH 8.5 with tetrabutyl-ammoniumhydroxide hydrate, 60 µL DIPEA and 1f (6.1 mg, 7.1 µmol) in 0.34 mL of DMSO are added and the mixture is stirred for 30 min at rt. Reaction mixture is diluted with 2 mL acetonitrile/ water 1/1 (v/v) and 200 µL AcOH and the protected N^{εK4/εK10}-CNP mono-linker conjugate is isolated from the reaction mixture by RP-HPLC.

Optimized RP-HPLC gradients can be used for isolation of N^{εK4}-CNP mono-linker thiol **2a** and N^{εK10}-CNP mono-linker thiol **2b.**

Removal of protecting groups is affected by treatment of lyophilized product fractions with 0.6 mL of 90/10/2/2 (v/v/v/v) HFIP/TFA/TES/water for 1h at rt. The deprotected N^{εK4/εK10}-CNP mono-linker thiol **2** is purified by RP-HPLC. Identity and purity of the product is determined by ESI-LCMS.

Deprotected N^{εK4}-CNP mono-linker thiol **2c** and N^{εK10}-CNP mono-linker thiol **2d** can be obtainend likewise from **2a** and **2b**, respectively.

### Example 3

### Synthesis of N^{αG1}-CNP mono-linker thiol 3

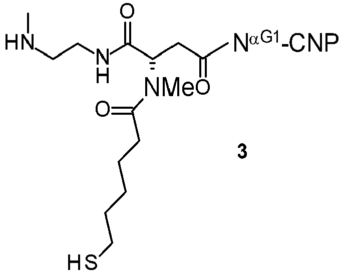

N^{αG1}-CNP mono-linker thiol **3** is prepared by dissolving CNP-22 (5.2 µmol) in 0.6 mL DMSO. 0.25 mL 0.5 M phosphate buffer pH 7.4 and **1f** (6.1 mg, 7.1 µmol) in 0.34 mL of DMSO are added and the mixture is stirred for several hours at rt. Reaction mixture is diluted with 2 mL acetonitrile/ water 1/1 (v/v) and 200 µL AcOH and the protected N^{αG1}-CNP mono-linker thiol is isolated from the reaction mixture by RP-HPLC.

Removal of protecting groups is affected by treatment of lyophilized product fractions with 0.6 mL of 90/10/2/2 (v/v/v/v) HFIP/TFA/TES/water for 1h at rt. The deprotected N^{αG1}-CNP mono-linker thiol **3** is purified by RP-HPLC. Identity and purity of the product is determined by ESI-LCMS.

### Example 4

### PEGylation of CNP mono-linker thiols 2c, 2d and 3

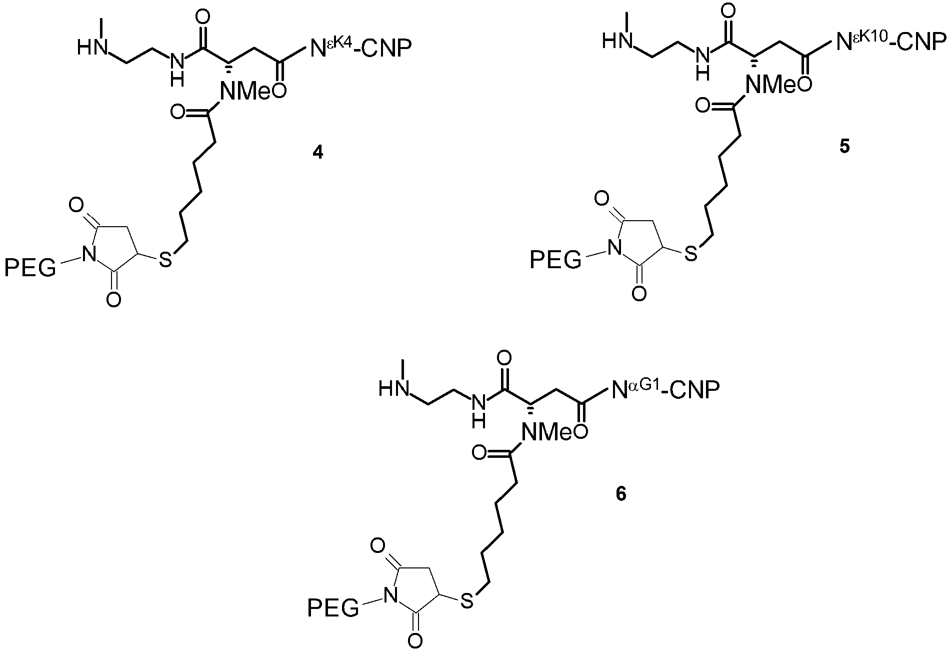

1 µmol CNP mono-linker thiol **2c** is dissolved in 0.5 mL acetonitrile / 0.2 M succinate buffer pH 3.8 1/1 (v/v) 1.2 µmol of linear 40 kDa PEG-maleimide is added and the mixture is stirred at rt. The reaction is quenched by addition of 20 µL AcOH and CNP conjugate **4** is purified by preparative RP-HPLC.

CNP conjugates **5** and **6** are prepared likewise from 1 µmol CNP mono-linker thiols **2d** and **3.**

CNP content is determined by quantitative amino acid analysis after total hydrolysis under acidic conditions.

### Example 5

### Release kinetics in vitro

CNP conjugates **4, 5** and **6** are dissolved in 60 mM sodium phosphate, 3 mM EDTA, 0.01% Tween-20, pH 7.4 at a concentration of approximately 2 mg/mL and filtered sterile. Mixtures are incubated at 37 °C. At time points aliquots are withdrawn and analysed by RP-HPLC and ESI-MS. UV-signals correlating to liberated CNP are integrated and plotted against incubation time.

Curve-fitting software is applied to estimate the corresponding halftime of release.

### Example 6

### Pharmacokinetics and cGMP production in rats

Equimolar doses of CNP-22, CNP conjugates **4, 5** or **6** are injected iv and sc in normal rats. Plasma CNP and cGMP levels over time are determined as described in the literature (US patent 8,377,884 B2).

### Example 7

### Synthesis of Dmb protected 6-mercaptohexanoic acid 7

Compound 7 was synthesized according to the following scheme:

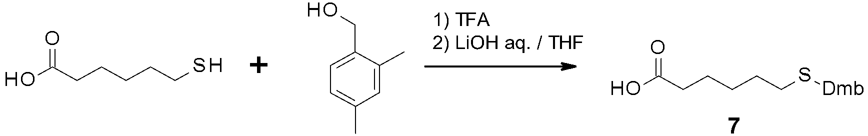

To a solution of 6-mercaptohexanoic acid (7.10 g, 47.90 mmol) in trifluoroacetic acid (20 mL), 2,4-dimethylbenzyl alcohol (13.5 g, 95.80 mmol) was added. The mixture was stirred at RT for 60 min and then the trifluoroacetic acid was removed *in vacuo.* The residue was dissolved in a mixture of 95.8 mL LiOH (3 M) and THF (81 mL) and stirred at rt for 60 min. The solvent was removed *in vacuo* and the aqueous residue was extracted 3x with EtOAc (200 mL). The combined organic phases were dried over MgSO₄, and the solvent was removed *in vacuo.* **7** was purified by RP-HPLC.

| | |
|---|---|
| Yield: | 2.27 g (8.52 mmol, 18 %) |
| MS: | m/z 267.01 = [M+H]⁺, (calculated monoisotopic mass = 266.13). |

### Example 8

### Synthesis of linker reagent 8c

Linker reagent **8c** was synthesized according to the following scheme:

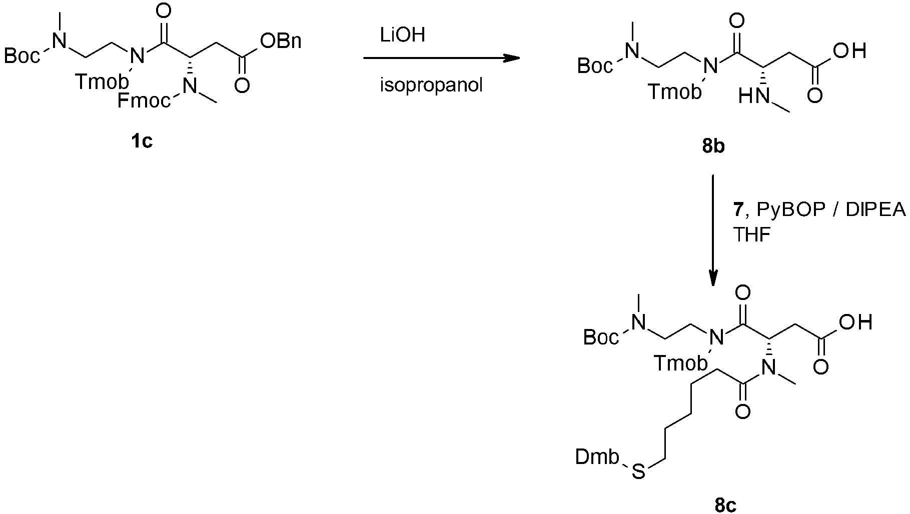

To a solution of **1c** (21.6 g, 27.18 mmol) in isopropanol (401 mL) were added water (130 mL) and LiOH (3.90 g, 163.06 mmol). The reaction mixture was stirred for 3 h at rt, then it was diluted with toluene (300 mL) and washed 3 x with 0.1 M HCl (200 mL). The combined aqueous phases were washed 3 x with toluene (100 mL). The aqueous phase was basified with 4 M NaOH (4 mL) to a pH of 8.5 and extracted 8 x with CH₂Cl₂ (200 mL). The combined CH₂Cl₂ phases were washed with brine (50 mL), dried over Na₂SO₄. **8b** was isolated upon evaporation of the solvent and used in the next reaction without further purification.

| | |
|---|---|
| Yield: | 11.89 g (24.59 mmol, 90 %) |
| MS: | m/z 484.16 = [M+H]⁺, (calculated monoisotopic mass = 483.26). |

To a solution of 7 (293 mg, 1.10 mmol) and PyBOP (572 mg, 1.10 mmol) in THF (10 mL) was added DIEA (0.52 mL, 3.00 mmol) under a N₂-atmosphere. The reaction mixture was stirred for 60 min at rt. A solution of **8b** (484 mg, 1.00 mmol) in THF (2 mL) was added and the reaction was stirred for a further 60 min. The reaction was quenched with 2 M citric acid solution (10 mL) and the THF was removed *in vacuo.* The resulting aqueous phase was then extracted 2 x with EtOAc (15 mL) and the combined organic layers were washed with water (10 mL) and brine (10 mL), and dried over MgSO₄. The solvent was removed *in vacuo* and **8c** was purified by RP HPLC.

| | |
|---|---|
| Yield: | 330 mg ( 0.451 mmol, 45 %) |
| MS: | m/z 732.34 = [M+H]⁺, (calculated monoisotopic mass = 731.38). |

### Example 9

### Synthesis of linker reagent 9

Linker reagent **9** was synthesized according to the following scheme:

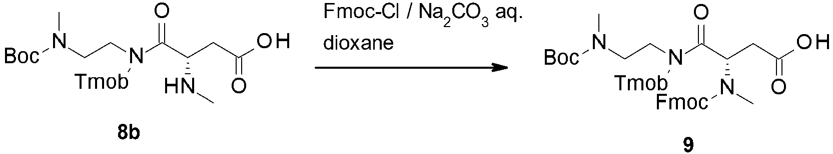

To a solution of **8b** (2.00 g, 4.14 mmol) and Fmoc-Cl (1.07 g, 4.14 mmol) in dioxane (20 mL) was added 1 M Na₂CO₃ solution (20 mL). The reaction mixture was stirred for 40 min at rt. Water (100 mL) and diethyl ether (100 mL) were added and the aqueous phase was extracted 2 x with diethyl ether (100 mL). The aqueous phase was acidified with conc. HCl until pH 1 and again extracted 3 x with diethyl ether. The combined organic phases were dried over Na₂SO₄ and the solvent was removed *in vacuo.* 9 was used in the next step without further purification.

| | |
|---|---|
| Yield: | 2.63 g (3.73 mmol, 90 %) |
| MS: | m/z 728.32 = [M+Na]⁺, (calculated monoisotopic mass = 705.33). |

### Example 10

### Synthesis of reversible Lys26 CNP-38 PEG2x20 kDa conjugate 10f

Conjugate **10f** was synthesized according to the following scheme:

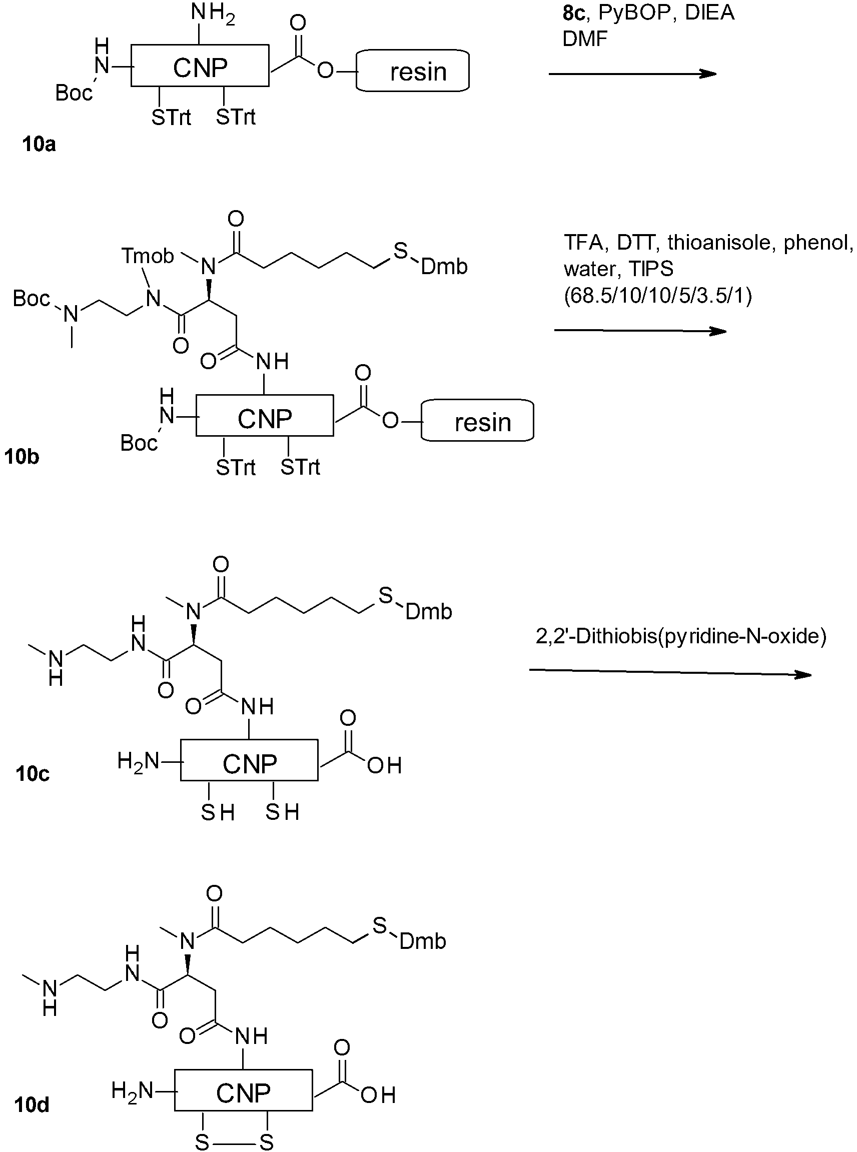

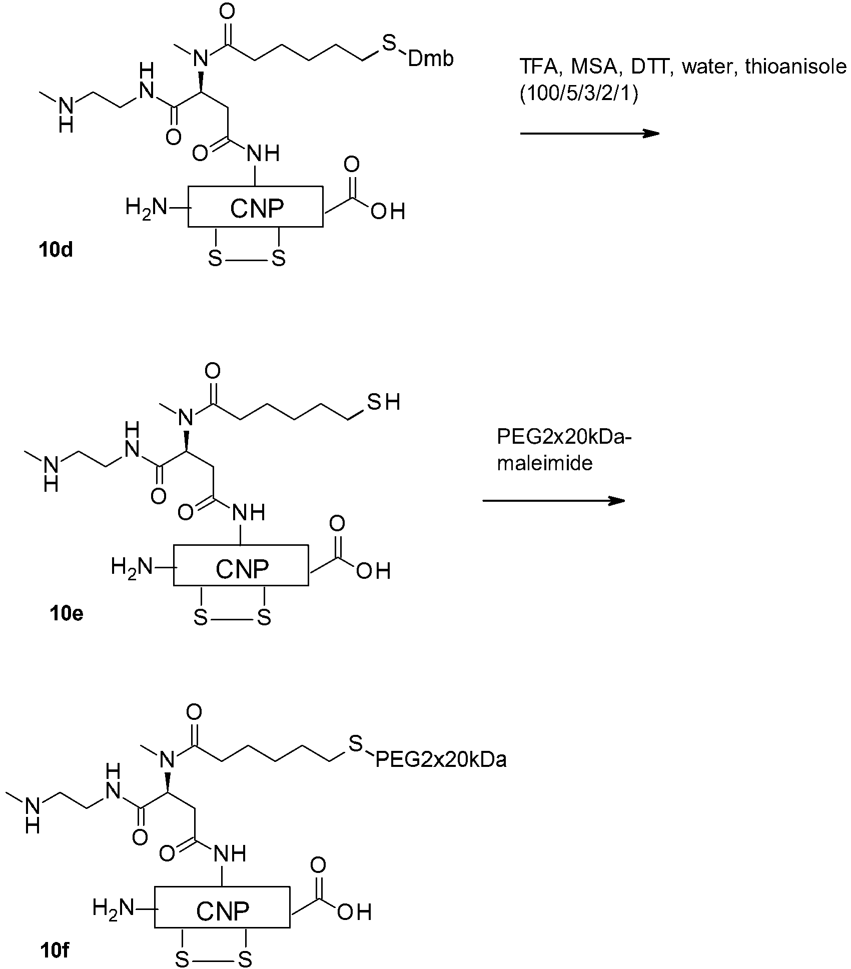

2.00 g (0.21 mmol) of side chain protected CNP-38 on TCP resin having Boc protected N-terminus and ivDde protected side chain of Lys26 was ivDde deprotected according to the procedure given in Materials and Methods to obtain **10a.** A solution of linker reagent **8c** (336 mg, 0.46 mmol), PyBOP (239 mg, 0.46 mmol) and DIEA (182 µL, 1.04 mmol) in DMF (5 mL) was incubated for 10 min at rt, then added to the resin **10a.** The suspension was shaken for 2 h at rt. The resin was washed 10 x with DMF (10 mL) and 10 x with DCM (10 mL) and dried *in vacuo* for 15 min. Cleavage of the peptide from resin and removal of protecting groups was achieved by treatment of the resin with 15 mL pre-cooled (-18 °C) cleavage cocktail 68.5/10/10/5/3.5/1 (v/w/v/v/v/v) TFA/DTT/thioanisole/phenol/water/TIPS. The mixture was allowed to warm to rt and was agitated for 60 min. Crude **10c** was precipitated in pre-cooled diethyl ether (-18 °C). The precipitate was dissolved in ACN/water and purified by RP-HPLC. The combined HPLC fractions were used directly in the next step.

| | |
|---|---|
| MS: | m/z 1124.60 = [M+4H]⁴⁺, (calculated monoisotopic mass for [M+4H]⁴⁺ = 1124.59). |

To the combined HPLC fractions of **10c** (250 mL) 40 mL of 0.5 M citric acid buffer (pH = 5.00) and 7 mL of a 0.01 M solution of 2,2'-dithiobis(pyridine-N-oxide) solution in 1/1 (v/v) acetonitrile/water were added. After incubation for 5 min at rt the reaction was complete. The mixture was diluted with 500 mL water containing 0.1 % TFA (v/v)and acidified with AcOH (20 mL) to a pH of approx. 2. **10d** was purified by RP-HPLC.

| | |
|---|---|
| Yield: | 101 mg (17.3 µmol, 9 % ) CNP-38-linker-Dmb * 10 TFA |
| MS: | m/z 1124.10 = [M+4H]⁴⁺, (calculated monoisotopic mass for [M+4H]⁴⁺ =1124.09). |

Cleavage of the Dmb protecting group was achieved by adding 30 mL pre-cooled (-18 °C) cleavage cocktail 100/5/3/2/1 (v/v/w/v/v) TFA/MSA/DTT/water/thioanisole to **10d** (101 mg, 17.3 µmol) and stirring for 3 h at 0 °C. Crude **10e** was precipitated in pre-cooled (-18 °C) diethyl ether. The precipitate was dissolved in water containing 0.1 % TFA (v/v) and incubated for 10 min in order to hydrolyze any TFA esters. **10e** was purified by RP-HPLC.

| | |
|---|---|
| Yield: | 46 mg (8.34 µmol, 48 %) CNP-38-linker-thiol * 10 TFA |
| MS: | m/z 1094.58 = [M+4H]⁴⁺, (calculated monoisotopic mass for [M+4H]⁴⁺ =1094.57). |

To a solution of **10e** (46 mg, 8.43 µmol) in 1.15 mL water containing 0.1 % TFA (v/v) was added a solution of PEG 2x20 kDa maleimide (Sunbright GL2-400MA, 870 mg, 21.75 µmol) in 4.35 mL water containing 0.1 % TFA (v/v), followed by 0.5 M lactic acid buffer (1.07 mL, pH = 4.20). The mixture was stirred at rt for 4 h. Conjugate **10f** was purified by RP-HPLC.

| | |
|---|---|
| Yield: | 233 mg (5.21 µmol, 62 %) conjugate **10f** * 10 HCl |

### Example 11

### Synthesis of reversible Lys26 CNP-38 PEG4x10 kDa conjugate conjugate 11i

Conjugate **11i** was synthesized according to the following scheme:

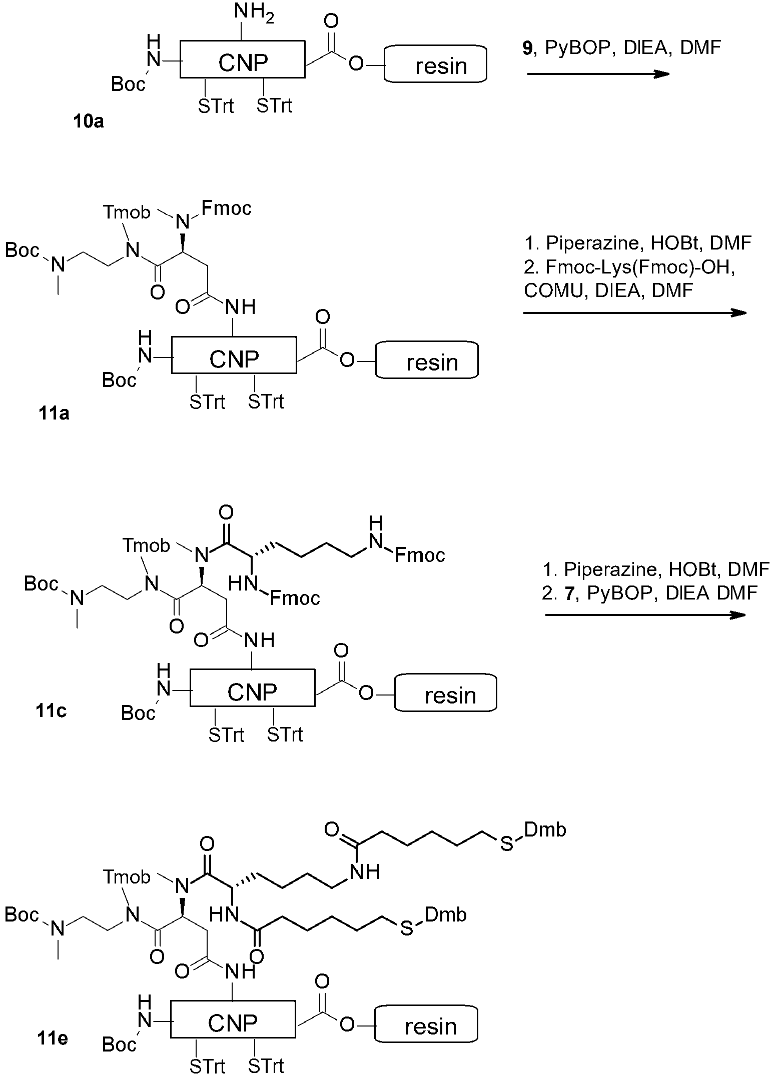

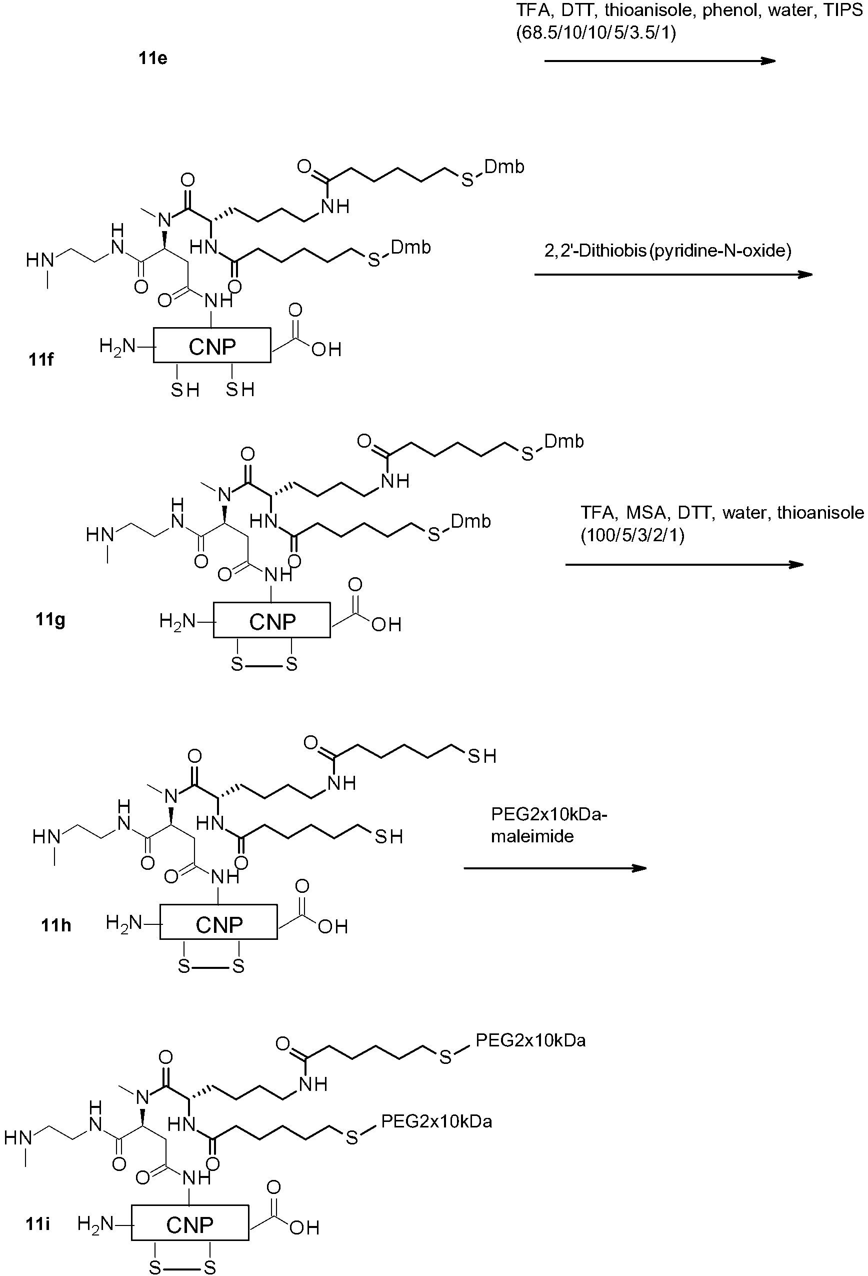

To a solution of **9** (353 mg, 0.50 mmol) and PyBOP (260 mg, 0.50 mmol) in DMF (9 mL) was added DIEA (105 µL, 0.60 mmol).This mixture was drawn onto Lys26-side-chain deprotected CNP-38 resin **10a** (2.00 g, 0.21 mmol) and the suspension was shaken for 2 h at RT in order to afford resin **11a**. The resin was washed 10 x with DMF (7 mL). Cleavage of the Fmoc protecting group in **11a** was carried out with a solution of HOBt (0.68 g, 5.03 mmol) and piperazine (3.00 g, 34.83 mmol) in DMF (47 mL). Therefore, the resin was incubated 5 x with 10 mL of the cleavage mixture for 15 min at rt each time. Then, the resin was washed 7 x with DMF (7 mL). A solution of Fmoc-Lys(Fmoc)-OH (449 mg, 0.76 mmol), COMU (325 mg, 0.76 mmol) and DIEA (165 µL, 0.95 mmol) in DMF (9 mL) was prepared and drawn onto the resin. The mixture was shaken for 2 h at rt. The procedure was repeated twice, each for 1 h with freshly prepared coupling mixture. The resin was washed 10 x with DMF (7 mL) and the remaining free amino groups were capped with 8 mL 1/1/2 (v/v/v) Ac₂O/pyridine/DMF.

Cleavage of the Fmoc protecting groups in **11c** was carried out with a solution of HOBt (0.68 g, 5.03 mmol), piperazine (3.00 g, 34.83 mmol) in DMF (47 mL). Therefore, the resin was incubated 5 x with 10 mL of the cleavage mixture for 15 min at rt each time. The resin was washed 7 x with DMF (7 mL)

To a solution of **7** (266 mg, 1.00 mmol) and PyBOP (520 mg, 1.00 mmol) in DMF (9 mL) was added DIEA (209 µL, 1.20 mmol). This mixture was drawn onto the resin and was shaken for 2 h at rt. The resin was washed 7 x with DMF (7 mL) affording resin **11e**. Cleavage of the peptide from resin and removal of protecting groups was achieved by treatment of the resin with 15 mL pre-cooled (-18 °C) cleavage cocktail 68.5/10/10/5/3.5/1 (v/w/v/v/v/v) TFA/DTT/thioanisole/phenol/water/TIPS. The mixture was allowed to warm to rt and was agitated for 3 h at. Crude **11f** was precipitated in pre-cooled (- 18 °C) diethyl ether and purified by RP-HPLC. The combined HPLC fractions were used directly in the next step.

MS: m/z 1218.66 = [M+4H]⁴⁺, (calculated monoisotopic mass for [M+4H]⁴⁺= 1218.65).

To the combined HPLC product fractions of **11f** (1 L) 160 mL of 0.5 M citric acid buffer (pH = 5.00) and 100 mL of a 50 mM 2,2'-dithiobis(pyridine-N-oxide) solution in 9/1 (v/v) acetonitrile/water were added. The mixture was stirred for 4 h at rt and then diluted with 1 L of water containing 0.1 % TFA (v/v). **11g** was purified by RP-HPLC.

| | |
|---|---|
| Yield: | 64.3 mg (10.7 µmol, 6 % ) CNP-38-linker-DMB * 10 TFA |
| MS: | m/z 1218.15 = [M+4H]⁴⁺, (calculated monoisotopic mass for [M+4H]⁴⁺ =1218.14). |

Cleavage of the Dmb protecting group was achieved by adding 45 mL of pre-cooled (-18 °C) cleavage cocktail 100/5/3/2/1 (v/v/w/v/v) TFA/MSA/DTT/water/thioanisole to **11g** (61.8 mg, 10.3 µmol), and then stirring for 4 h at 0 °C. Crude **11h** was precipitated in pre-cooled (-18 °C) ether. The precipitate was dissolved in a solution of 1/1 (v/v) acetonitrile/water containing 0.1 % TFA (v/v) and incubated for 4 h at rt in order to hydrolyze any TFA esters. **11h** was purified by RP-HPLC.

| | |
|---|---|
| Yield: | 38.4 mg (6.65 µmol, 65 %) CNP-38-linker-thiol * 10 TFA |
| MS: | m/z 1159.11 = [M+4H]⁴⁺, (calculated monoisotopic mass for [M+4H]⁴⁺ =1159.10). |

To a solution of **11h** (34.6 mg, 5.99 µmol) in 1 mL water containing 0.1 % TFA (v/v) was added a solution of PEG 2x10 kDa maleimide (Sunbright GL2-200MA, 1.12 g, 56.03 µmol) in 6.1 mL water containing 0.1 % TFA (v/v), followed by 0.5 M lactic acid buffer (1.46 mL, pH = 4.00). The mixture was stirred at rt for 4 h. Conjugate **11i** was purified by RP-HPLC.

| | |
|---|---|
| Yield: | 227 mg (4.96 µmol, 83 %) conjugate **11i** * 10 HCl |

### Example 12

### Synthesis of permanent Lys26 CNP-38 PEG4x10 kDa conjugate 12g

Conjugate **12g** was synthesized according to the following scheme:

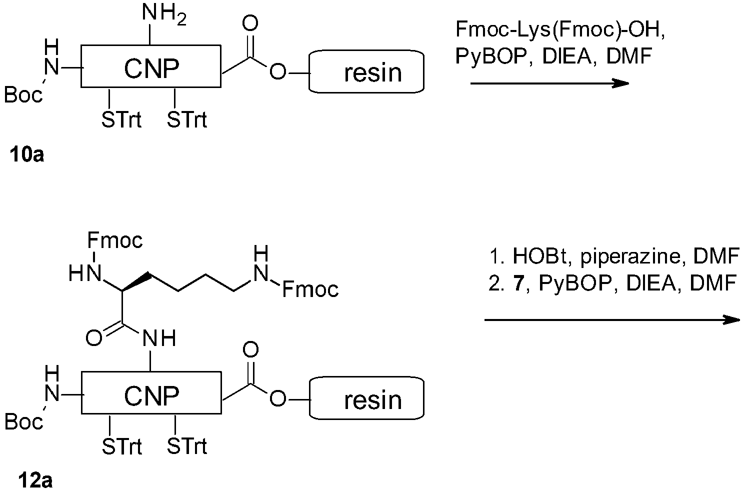

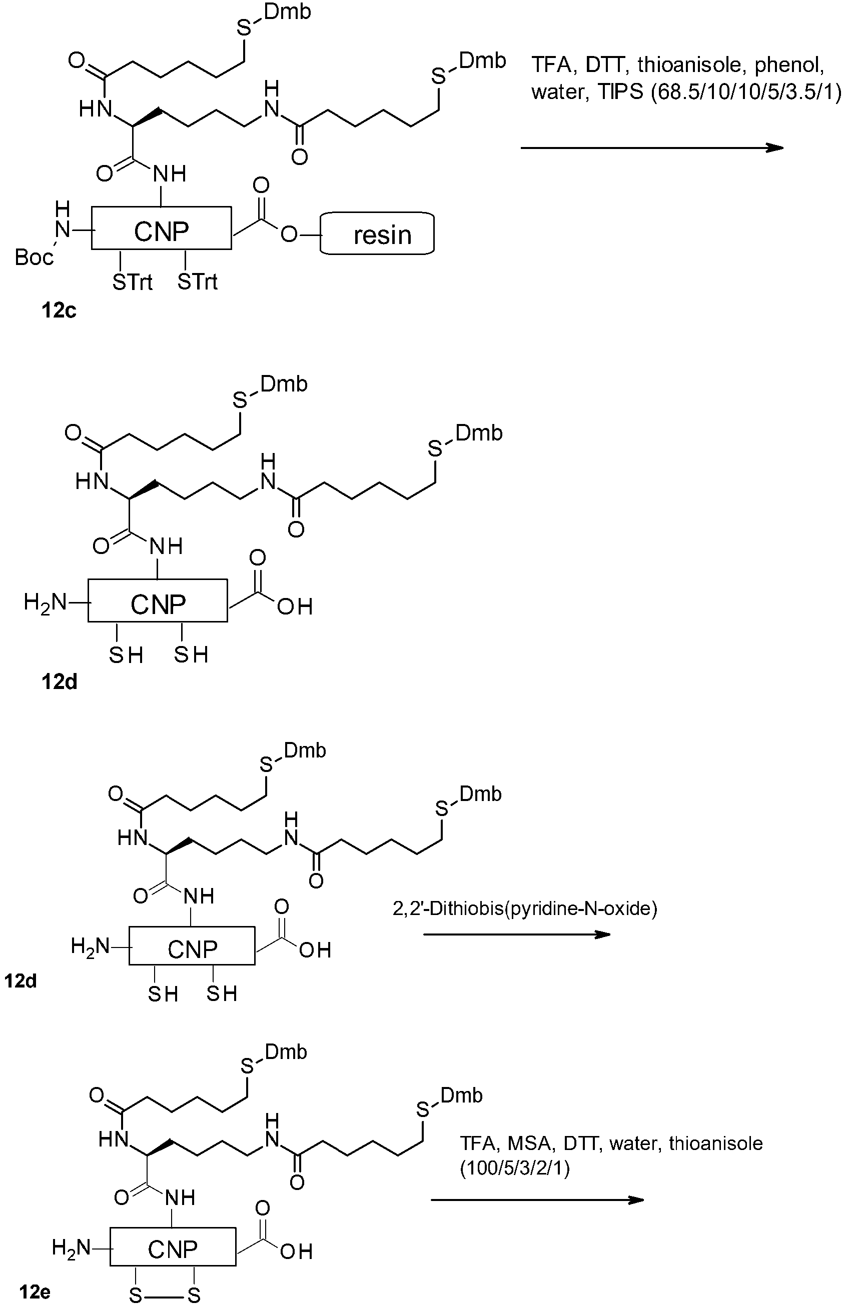

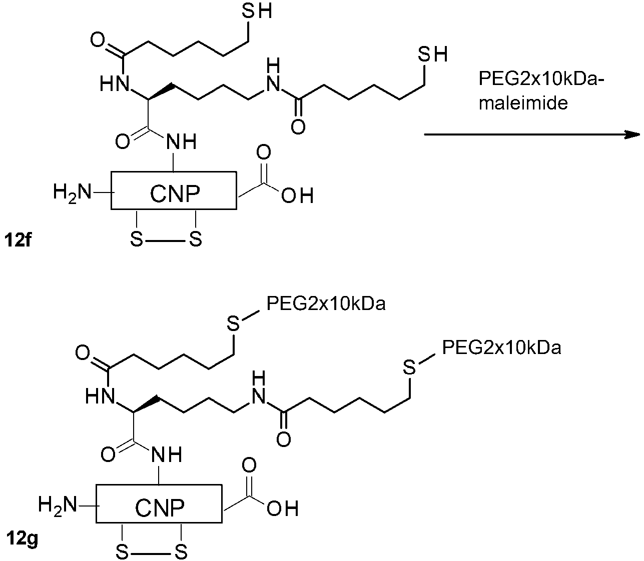

To a solution of Fmoc-Lys(Fmoc)-OH (365 mg, 0.62 mmol) and PyBOP (322 mg, 0.62 mmol) in DMF (4.6 mL) was added DIEA (0.11 mL, 0.62 mmol).The mixture was drawn onto resin **10a** (2.0 g, 0.21 mmol). The suspension was shaken for 2 h at rt. The resin was washed 10 x with DMF (7 mL). Cleavage of the Fmoc protecting groups in **12a** was carried out with a solution of HOBt (1.35 g, 9.99 mmol), piperazine (6.00 g, 69.66 mmol) in DMF (94 mL). Therefore, the resin was incubated 5 x with the cleavage mixture for 15 min at rt each time, affording resin **12b.** Then the resin was washed 7 x with DMF (7 mL)

To a solution of 7 (283 mg, 1.06 mmol) and PyBOP (552 mg, 1.06 mmol) in DMF (6.5 mL), DIEA (185 µL, 1.06 mmol) was added and drawn onto resin **12b** (2.07 g, 0.10 mmol/g, 0.21 mmol). The mixture was shaken for 2 h at rt. Then, the resin was washed 10 x each with DMF (7 mL) and CH₂Cl₂ (7 mL) and dried *in vacuo.*

Cleavage of the peptide from resin and removal of protecting groups was achieved by treatment of the resin with 15 mL pre-cooled (-18 °C) cleavage cocktail 68.5/10/10/5/3.5/1 (v/w/v/v/v/v) TFA/DTT/thioanisole/phenol/water/TIPS. The mixture was allowed to warm to rt and was agitated for 2.5 h. Crude **12d** was precipitated in pre-cooled diethyl ether (-18 °C) and purified by RP-HPLC. The combined HPLC fractions were used directly in the next step.

| | |
|---|---|
| MS: | m/z 1172.37 = [M+4H]⁴⁺, (calculated monoisotopic mass for [M+4H]⁴⁺ =1172.37). |

To the combined HPLC product fractions of **12d** (390 mL) 58.5 mL of 0.5 M citric acid buffer (pH = 5.00) and 8.9 mL of a 10 mM 2,2'-dithiobis(pyridine-N-oxide) solution in 1/1 (v/v) acetonitrile/water were added. The mixture was stirred for 10 min at rt then diluted with 400 mL of water containing 0.1 % TFA (v/v). **12e** was purified by RP-HPLC.

| | |
|---|---|
| Yield: | 100 mg (17.5 µmol, 8 % over 6 steps) CNP-38-linker-Dmb * 9 TFA |
| MS: | m/z 1171.87 = [M+4H]⁴⁺, (calculated monoisotopic mass for [M+4H]⁴⁺ =1171.86). |

Cleavage of the Dmb protecting group was achieved by adding 65 mL pre-cooled (-18 °C) cleavage cocktail 100/5/3/2/1 (v/v/w/v/v) TFA/MSA/DTT/water/thioanisole to **12e** (100 mg, 17.5 µmol) and stirring for 3.5 h at 0 °C. Crude **12f** was precipitated in pre-cooled (- 18 °C) diethyl ether. The precipitate was dissolved in water containing 0.1 % TFA (v/v) and incubated for 2 h at rt in order to hydrolyze any TFA esters. **12f** was purified by RP-HPLC.

| | |
|---|---|
| Yield: | 43.4 mg (7.92 µmol, 45 %) CNP-38-linker-thiol * 9TFA |
| MS: | m/z 1112.83 = [M+4H]⁴⁺, (calculated monoisotopic mass for [M+4H]⁴⁺ =1112.82). |

To a solution of **12f** (39.6 mg, 7.22 µmol) in 1 mL water containing 0.1 % TFA (v/v) was added a solution of PEG 2x10 kDa maleimide (Sunbright GL2-200MA, 1.22 g, 59.94 µmol) in 6.16 mL water containing 0.1 % TFA (v/v), followed by 0.5 M lactic acid buffer (1.41 mL, pH = 4.20). The mixture was stirred at rt for 4 h. Conjugate **12g** was purified by RP-HPLC.

| | |
|---|---|
| Yield: | 204 mg (4.48 µmol, 57 %) conjugate **12g** * 9 HCl |

### Example 13

### Synthesis of PEG5kDa thiol 13c

PEG5kDa thiol **13c** was synthesized according to the following scheme:

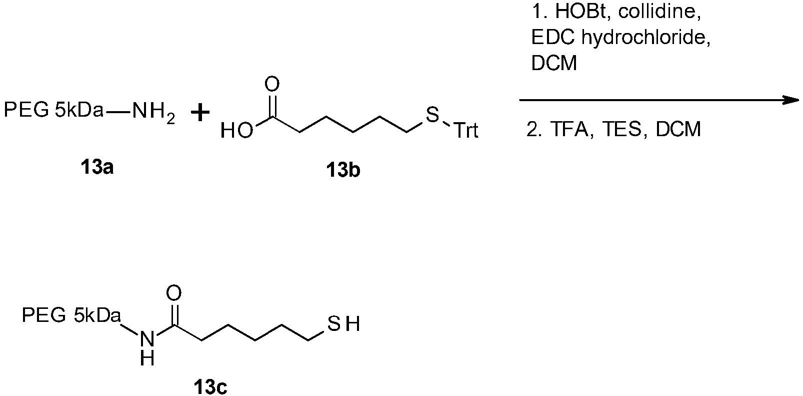

To a solution of **13b** (58.6 mg, 0.15 mmol), HOBt (22.9 mg, 0.15 mmol) and EDC hydrochloride (28.8 mg, 0.15 mmol) in DCM (1.00 mL) 2,4,6-collidine (121 mg, 1.00 mmol) was added. Then, a solution of methoxy PEG amine 5 kDa **13a** (500 mg, 0.10 mmol) in DCM (4.00 mL) was added and the mixture was stirred for 16 h at rt. The solvent was evaporated and the mixture was dissolved in ACN/water and purified by RP-HPLC. The amount of solvent was reduced *in vacuo* and the aqueous residue was extracted with DCM (1 x 100 mL, 2 x 50 mL). The combined organic layers were reduced *in vacuo* to 20 mL. TFA (1.6 mL) and TES (3.5 mL) were added and the mixture was stirred at rt for 4.5 h. **13c** was precipitated in diethyl ether, stored over night at - 20 °C, filtered and dried *in vacuo*.

| | |
|---|---|
| Yield: | 372 mg (72 µmol, 72 %) |

### Example 14

### Synthesis of permanent N-terminal CNP-34 PEG 5 kDa conjugate 14e

Conjugate **14e** was synthesized according to the following scheme:

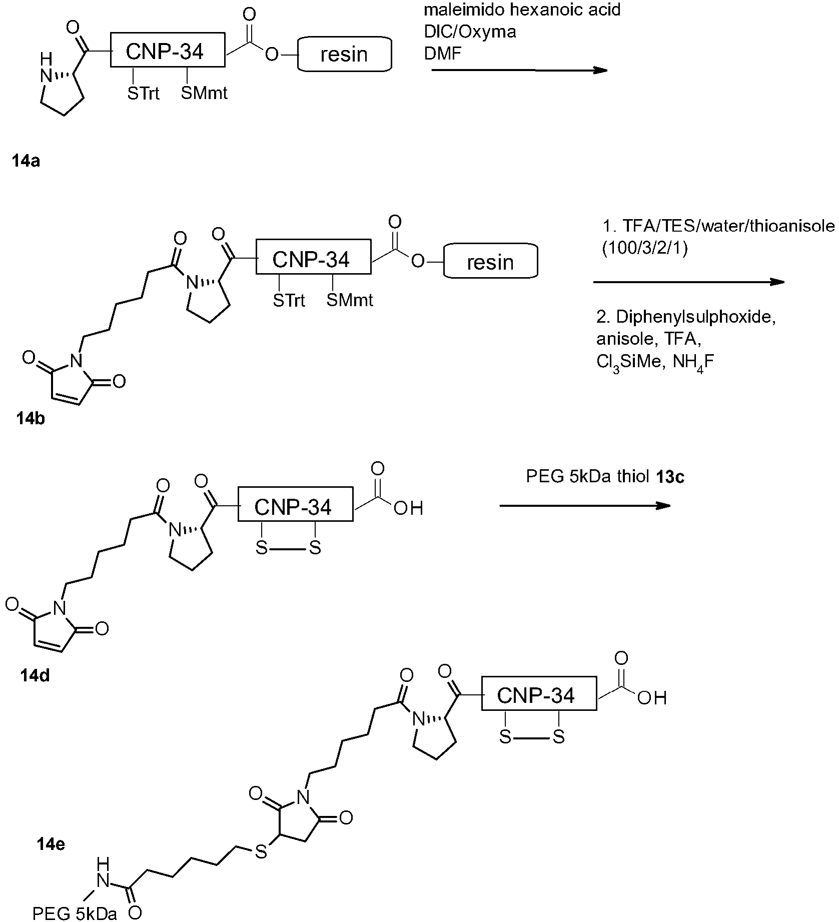

Side chain protected CNP-34 on TCP tentagel resin having free N-terminus **14a** (0.78 g, 70 µmol) was pre-swollen in DMF for 30 min. A solution of maleimido hexanoic acid (85.3 mg, 0.40 mmol), DIC (50.9 mg, 0.40 mmol) and Oxyma (57.4 mL, 0.40 mmol) in DMF (6 mL) was drawn onto the resin and the mixture was shaken for 30 min at rt. The coupling then was repeated once with freshly prepared coupling solution. The resin was washed 10 x each with DMF and CH₂Cl₂ and dried *in vacuo* affording **14b.**

Cleavage of the peptide from resin and removal of protecting groups was achieved by treatment of the resin with 6 mL cleavage cocktail 100/3/2/1 (v/v/v/v) TFA/TES/water/thioanisole for 1.5 h at rt. The crude peptide was precipitated in pre-cooled (-18 °C) diethyl ether.

MS: m/z 937.77 = [M+4H]⁴⁺, (calculated monoisotopic mass for [M+4H]⁴⁺ = 937.74).

The precipitate was dissolved in 15 mL TFA. A solution of diphenylsulfoxide (68.06 mg, 0.34 mmol) and anisole (0.18 mL, 1.68 mmol) in 5 mL TFA was added. Trichloromethylsilane (0.47 mL, 4.17 mmol) was added and the mixture was stirred for 15 min at rt. Ammonium fluoride (0.38 g, 10.3 mmol) was added and the solution was agitated for a further 2 min. The crude material was precipitated in pre-cooled (-18 °C ) diethyl ether and purified by RP-HPLC affording **14d**.

| | |
|---|---|
| Yield: | 8.30 mg (1.78 µmol, 82 % purity, 1.4 % over 3 steps) CNP-34-Malhx * 8 TFA |
| MS: | m/z 937.26 = [M+4H]⁴⁺, (calculated monoisotopic mass for [M+4H]⁴⁺ =937.23). |

To a solution of **14d** (7.34 mg, 1.57 µmol) in 200 µL 1/1 (v/v) acetonitrile/water containing 0.1 % TFA (v/v) was added a solution of **13c** (20 mg, 3.90 µmol) in 200 µL water containing 0.1 % TFA (v/v), followed by 200 µL 0.5 M acetate buffer (pH = 5.00). The mixture was incubated at rt for 30 min. Conjugate **14e** was purified by RP-HPLC.

| | |
|---|---|
| Yield: | 9.92 mg (1.01 µmol, 57 %) conjugate **14e** * 8 TFA |

### Example 15

### Synthesis of permanent N-terminal CNP-38 PEG 5kDa conjugate 15e

Conjugate **15e** was synthesized according to the following scheme:

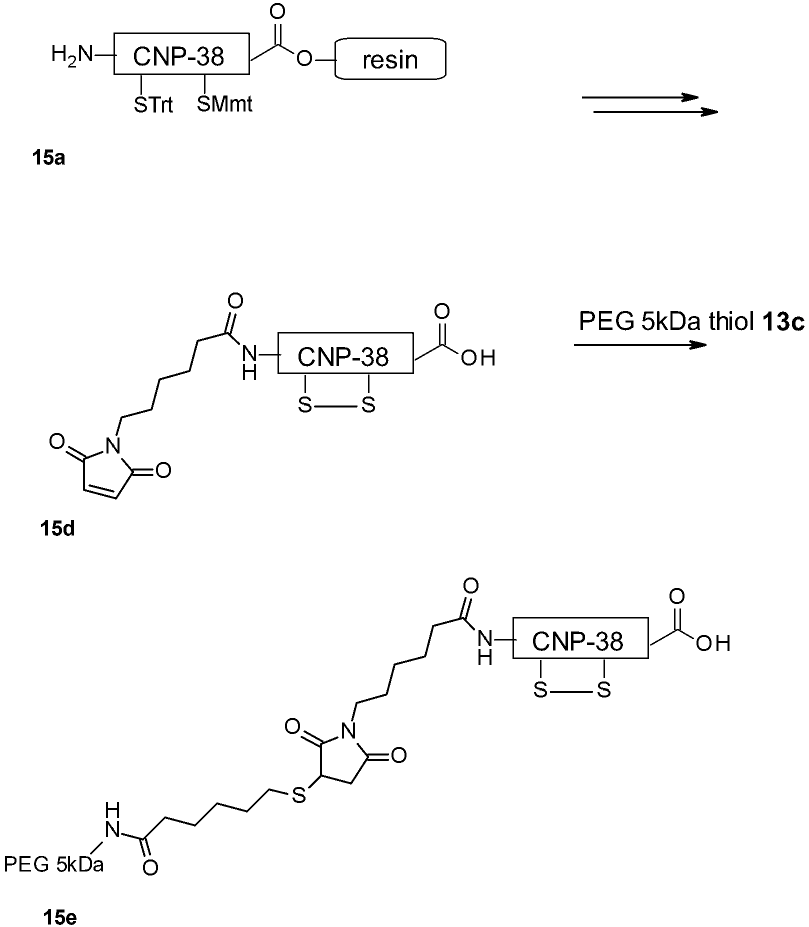

Compound **15d** was synthesized as described for **14d,** except that side chain protected CNP-38 on TCP tentagel resin having free N-terminus **15a** (1.34 g, 0.12 mmol) was used as starting material.

| | |
|---|---|
| Yield: | 15.6 mg (2.94 µmol, 6.6 %) CNP-38-Malhx * 9 TFA |
| MS: | m/z 1064.05 = [M+4H]⁴⁺, (calculated monoisotopic mass for [M+4H]⁴⁺ =1064.04). |

Conjugate **15e** was synthesized as described for **14e,** except that **15d** (8.34 g, 1.58 mmol) was used as starting material.

| | |
|---|---|
| Yield: | 9.47 mg (0.91 µmol, 31 %) conjugate **15e** * 9 TFA |

### Example 16

### Synthesis of permanent Lys12 CNP-34 PEG 5 kDa conjugate 16e

Conjugate **16e** was synthesized according to the following scheme:

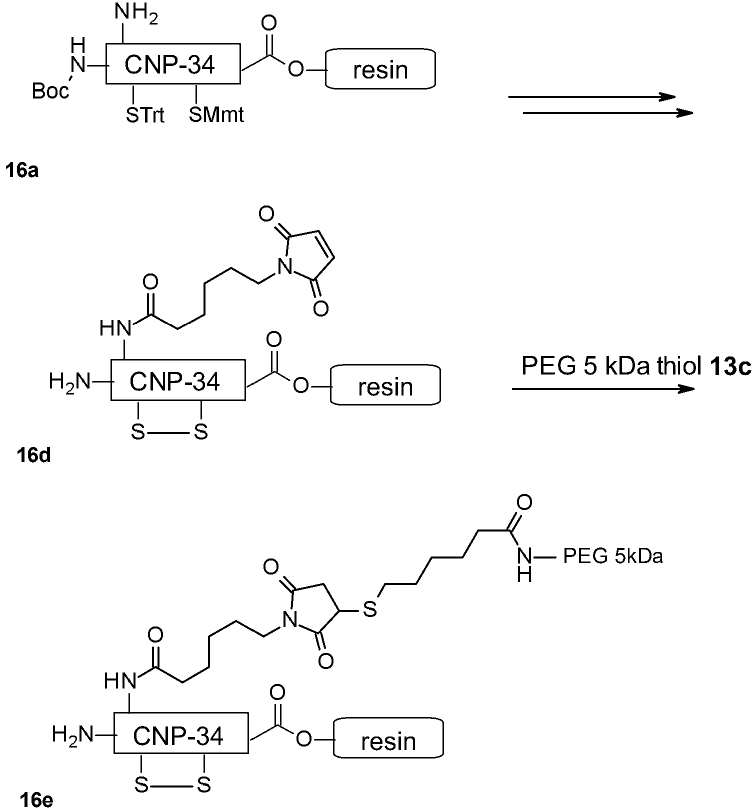

1.00 g (0.10 mmol) of side chain protected CNP-34 on TCP tentagel resin having Boc protected N-terminus and ivDde protected side chain of Lys12 was ivDde deprotected according to the procedure given in Materials and Methods to obtain **16a.**

Compound **16d** was synthesized as described for **14d,** except that resin **16a** (1.00 g, 0.10 mmol) was used as starting material.

| | |
|---|---|
| Yield: | 17.0 mg (3.65 µmol, 3.7 %) CNP-34-Lys12-Malhx * 8 TFA |
| MS: | m/z 937.25 = [M+4H]⁴⁺, (calculated monoisotopic mass for [M+4H]⁴⁺ =937.23). |

Conjugate **16e** was synthesized as described for **14e,** except that **16d** (17 mg, 3.65 µmol) was used as starting material.

| | |
|---|---|
| Yield: | 12.2 mg (1.25 µmol, 34 %) conjugate **16e** * 8 TFA |

### Example 17

### Synthesis of permanent Lys16 CNP-34 PEG 5 kDa conjugate 17e

Conjugate **17e** was synthesized according to the following scheme:

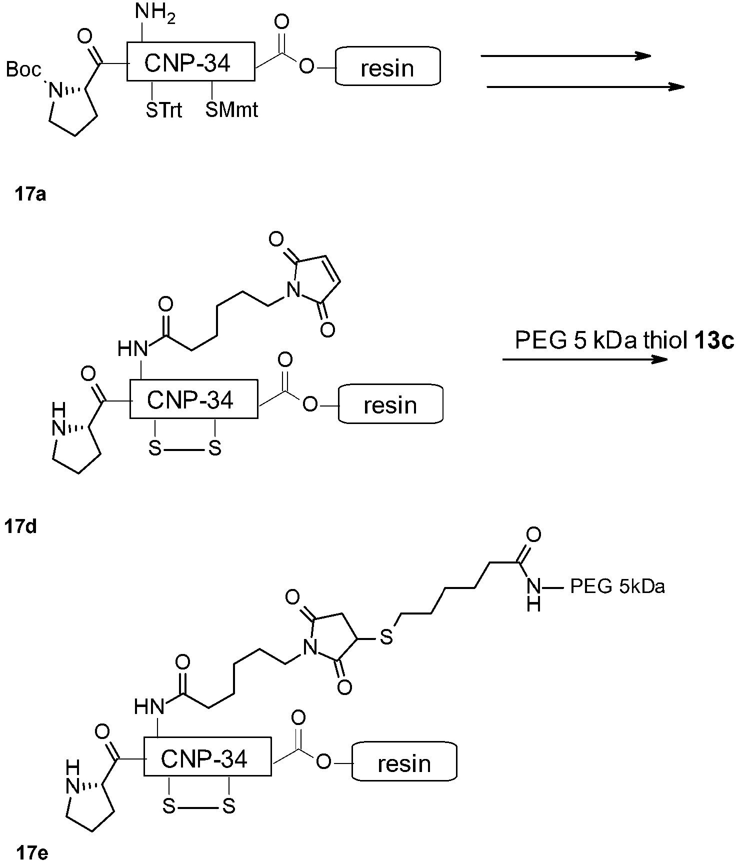

0.78 g (0.07 mmol) of side chain protected CNP- 34 on TCP tentagel resin having Boc protected N-terminus and ivDde protected side chain of Lys16 was ivDde deprotected according to the procedure given in Materials and Methods to obtain **17a.**

Compound **17d** was synthesized as described for **14d,** except that resin **17a** (0.78 g, 0.13 mmol) was used as starting material.

| | |
|---|---|
| Yield: | 5.39 mg (1.16 µmol, 1.7 %) CNP-34-Lys16-Malhx * 8 TFA |
| MS: | m/z 937.26 = [M+4H]⁴⁺, (calculated monoisotopic mass for [M+4H]⁴⁺ =937.23). |

Conjugate 17e was synthesized as described for **14e,** except that **17d** (5.39 mg, 1.16 µmol) was used as starting material.

| | |
|---|---|
| Yield: | 10.7 mg (1.09 µmol, 94 %) conjugate 17e * 8 TFA |

### Example 18

### Synthesis of permanent Lys22 CNP-34 PEG 5 kDa conjugate 18e

Conjugate **18e** was synthesized according to the following scheme:

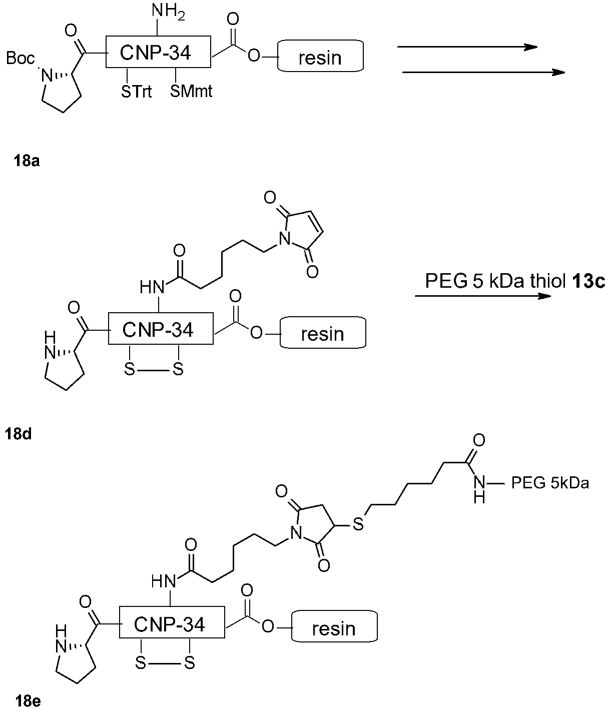

1.07 g (0.11 mmol) of side chain protected CNP- 34 on TCP tentagel resin having Boc protected N-terminus and ivDde protected side chain of Lys12 was ivDde deprotected according to the procedure given in Materials and Methods to obtain **18a.**

Compound **18d** was synthesized as described for **14d,** except that resin **18a** (1.07 g, 0.11 mmol) was used as starting material.

| | |
|---|---|
| Yield: | 5.20 mg (1.12 µmol, 1.0 %) CNP-34-Lys22-Malhx * 8 TFA |
| MS: | m/z 937.26 = [M+4H]⁴⁺, (calculated monoisotopic mass for [M+4H]⁴⁺ =937.23). |

Conjugate **18e** was synthesized as described for **14e,** except that **18d** (5.2 mg, 1.12 µmol) was used as starting material.

| | |
|---|---|
| Yield: | 4.20 mg (0.43 µmol, 38 %) conjugate **18e** * 8 TFA |

### Example 19

### Synthesis of permanent Lys26 CNP-38 PEG 5 kDa conjugate 19e

Conjugate 19e was synthesized according to the following scheme:

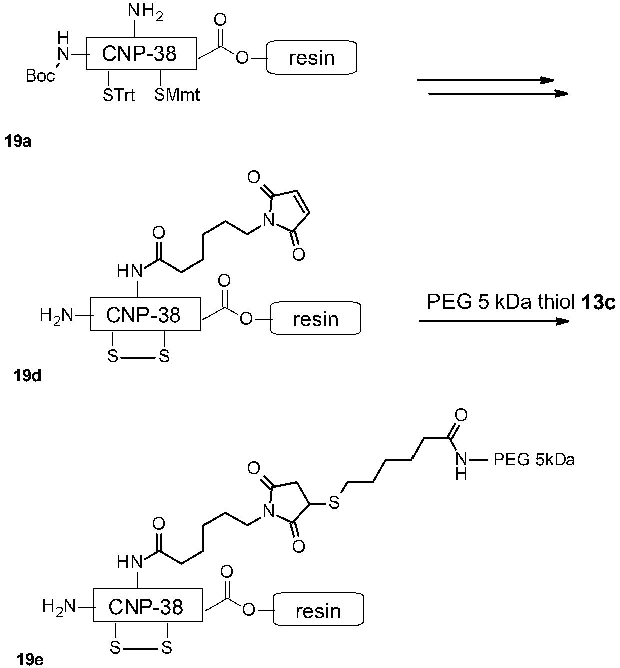

(0.865 g, 0.10 mmol) of side chain protected CNP-38 on TCP tentagel resin having Boc protected N-terminus and ivDde protected side chain of Lys26 was ivDde deprotected according to the procedure given in Materials and Methods to obtain **19a.**

Compound **19d** was synthesized as described for **14d**, except that resin **19a** (0.865 g, 0.10 mmol) was used as starting material.

| | |
|---|---|
| Yield: | 10.3 mg (1.95 µmol, 2.0 %) CNP-38-Lys26-Malhx * 9 TFA |
| MS: | m/z 1064.05 = [M+4H]⁴⁺, (calculated monoisotopic mass for [M+4H]⁴⁺ =1064.04). |

Conjugate **19e** was synthesized as described for **14e**, except that **19d** (4.70 mg, 1.10 µmol) was used as starting material.

| | |
|---|---|
| Yield: | 3.20 mg (0.31 µmol, 28 %) conjugate **19e** * 9 TFA |

### Example 20

### Release kinetics in vitro

CNP conjugates **10f** and **11i** were dissolved in a PBS buffer containing 3 mM EDTA and 10 mM methionine, pH 7.4 at a concentration of approximately 1 mg conjugate/mL. The solutions was filtered sterile and were incubated at 37 °C. At time points aliquots were withdrawn and analysed by RP-HPLC and ESI-MS. UV-signals correlating to liberated CNP were integrated and plotted against incubation time.

Curve-fitting software was applied to estimate the corresponding halftime of release.

### Results:

For conjugate **10f** a release half life time of 8.5 d (± 1 d) was obtained.

For conjugate **11i** a release half life time of 9.5 d (± 1.5 d) was obtained.

### Example 21

### Digest of CNP variants by Neutral Endopeptidase In Vitro

In order to determine the *in vitro* stability of various CNP variants including different peptide chain lengths and PEGylations using different PEGylation sites and PEG molecules in the presence of Neutral Endopeptidase (NEP), a NEP digest assay was established. This assay monitored the decrease of the non-digested CNP variant (normalized with the internal standard PFP) over time in reference to the t₀-time point.

In detail, recombinant human NEP (2.5 µg/mL final concentration) and the standard pentafluorophenol (PFP; 40 µg/mL final concentration) were added to the CNP variant (100 µg CNP equivalents/mL) in digest buffer (50 mM Tris-HCl, pH 7.4, 10 mM NaCl). The solution was incubated at 37 °C and 500 rpm for up to 4 days. Samples were taken at different time intervals. The reaction was stopped by a combined reduction and heat denaturation adding TCEP (tris(2-carboxyethyl)phosphine; 25 mM final concentration) and incubating the mixture at 95 °C, 500 rpm for 5 minutes. The resulting reaction products were assigned using HPLC-MS. The half life of each CNP variant was calculated via the ratio change in the HPLC-UV peak areas of CNP and PFP over time. To compensate for variations in the protease activity, a CNP-38 or CNP-34 digest was carried out in every batch measurement as reference.

**Table 1 lists the half-lives, based on the in vitro NEP cleavage assay, of various CNP variants of different lengths and having various PEG molecules attached to different side chains.**

| **Compound** | **CNP-variant** | **PEGylation** | **half life norm. [h]** |
|---|---|---|---|
| CNP-22¹ | CNP-22 | - | 0.32 |
| CNP-34¹ | CNP-34 | - | 4.15 |
| **14e**¹ | CNP-34 | 5 kDa PEG, N-Terminus | Almost no proteolysis after 4 days. |
| **17e**¹ | CNP 34 | 5 kDa PEG, Lys16 | 54.23 |
| **18e**¹ | CNP-34 | 5 kDa PEG, Lys22 | 38.87 |
| **16e**¹ | CNP-34 | 5 kDa PEG, Lys12 | No evaluation possible. |
| | | | |
| CNP-38² | CNP-38 | - | 12.10 |
| **19e**² | CNP-38 | 5 kDa PEG, Lys26 | 62.76 |
| **15e**² | CNP-38 | 5 kDa PEG, N-Terminus | Almost no proteolysis after 4 days. |
| **12g**² | CNP-38 | 4x10 kDa PEG, -Lys26 | Almost no proteolysis after 4 days. |

1) Due to variations in NEP catalytic activity between experiments, a mean was formed of all CNP-34 half life measurements (4.15h) and the CNP-34 conjugates' half life measurements were normalized to this mean using a coefficient to calculate the adjusted t_{1/2}.
2) Due to variations in NEP catalytic activity between experiments, a mean was formed of all CNP-38 half life measurements (12.10h) and the CNP-38 conjugates' half life measurements were normalized to this mean using a coefficient to calculate the adjusted t_{1/2} .

The rank order of resistance towards NEP is as follows: The longer CNP-variant (CNP-38) is more stable than the shorter CNP variant (CNP-34), which in turn is more stable than the shorter CNP-22. The order of the PEG-attachment sites is as follows: N-terminal > next-to-ring > ring. Therefore, an N-terminal PEG attachment confers the highest stability towards the proteolytic digest with NEP for the tested conjugates. The stability of CNP-38 PEGylated at Lys26 can be increased with increasing PEG size.

### Example 22

### Functional cGMP stimulation in NIH-3T3 cells with CNP variants

Functional activity of CNP variants were determined in a cell-based assay with NIH-3T3 cells (Murine Embryo Fibroblast cell line). These cells express endogenously NPR-B on the cell surface. Stimulation of NPR-B with CNP leads to intracellular production of the second messenger cGMP which is detected with a commercially available cGMP assay. NIH-3T3 cells were routinely cultured in DMEM F-12 medium with 5 % FBS and 5 mM glutamine at 37°C and 5 % CO₂. For each assay, 50,000 cells were resuspended in stimulation buffer (Dulbecco's PBS with IBMX) and incubated with the CNP variants in different concentrations. CNP (dilutions were made in PBS with 0.2 % BSA). After incubation of 30 min at 37°C and 5 % CO₂, the cells were lyzed and cGMP levels were determined with a commercially available cGMP TR-FRET assay (Cisbio, cGMP kit, Cat. No. 62GM2PEB). PEGylated CNP variants were always characterized in comparison with the non-PEGylated version in the same experiment batch. If possible, evaluation of the residual activity was done via the EC50-parameter of the resulting dose-response curve (restricted model with common slope).

**Table 2: Residual NPR-B activity of PEGylated CNP variants in a cell-based assay as determined against the non-PEGylated CNP variant**

| Compound | CNP Variant | PEGylation | Residual Activity [%] |
|---|---|---|---|
| **15e** | CNP-38 | 5 kDa PEG, N-Terminus | 14 |
| **19e** | CNP-38 | 5 kDa PEG, Lys26 | < 1 |
| **12g** | CNP-38 | 4x10 kDa PEG, Lys26 | << 1 |

Comparing the tested PEG attachment sites, the attachment at the Lys26 (ring-lysine) showed the highest functional activity reduction, whereas the N-terminal attachment showed relatively high residual functional activity values. Increasing the PEG size resulted in a better shielding of the CNP molecule and a lower residual functional activity.

### Example 23

### Growth study in FVB mice after 5 weeks treatment with CNP-38 by daily subcutaneous bolus injection or by continuous subcutaneous infusion

This study was performed in order to test the effect of daily subcutaneous bolus injection vs. continuous subcutaneous infusion of CNP-38 on animal growth. 21- to 22-days-old wild-type FVB male mice (n = 9/group) were given 50 nmol/kg/d CNP-38 or vehicle (30 mM acetate pH 4 containing 5 % sucrose and 1 % benzylic alcohol) either by daily subcutaneous bolus injection or by continuous subcutaneous infusion in the scapular region over 35 days. Continuous infusion was applied by Alzet osmotic pumps model 1002 for week 1-2, followed by model 1004 for week 3-5. CNP-38 concentrations in the pumps were adjusted for the mean animal weight at study day 7 (pump model 1002) or study day 25 (pump model 1004). Growth was determined at d 35 by total body length measurement and X-ray measurements of the right femur and tibia.

Results of animals treated by daily subcutaneous bolus injection: At d 35, total body lenght of CNP-38 treated animals was 110.2 %, right femur length was 105.6 % and right tibia length was 104.0 % compared to vehicle treated animals.

Results of animals treated by continuous subcutaneous infusion: At d 35, total body lenght of CNP-38 treated animals was 121.7 %, right femur length was 107.5 % and right tibia length was 112.2 % compared to vehicle treated animals.

It was concluded that continuous subcutaneous infusion or related slow release formulations of CNP-38 (e.g. a slow releasing CNP-38 prodrug) are more effective than daily subcutaneous bolus injection in eliciting growth in the appendicular and axial skeleton.

### Example 24

### Pharmacokinetic study of permanent Lys26 CNP-38 PEG4x10 kDa conjugate 12g in cynomolgus monkeys

This study was performed in order to show the suitability of **12g** as a model compound for a slow release CNP-38 prodrug in cynomolgus monkeys. Male cynomolgus monkeys (2-4 years old, 3.5-4.1 kg) received either a single intravenous (n = 3 animals) or a single subcutaneous (n = 2 animals) administration of **12g** at a dose of 0.146 mg CNP-38 eq/kg. Blood samples were collected up to 168 h post dose, and plasma was generated. Plasma CNP-38 concentrations were determined by quantification of the N-terminal signature peptide (sequence: LQEHPNAR) and C-terminal signature peptide (sequence: IGSMSGLGC) after tryptic digestion as described in Materials and Methods.

Results: Dose administrations were well tolerated with no visible signs of discomfort during administration and following administration. No dose site reactions were observed any time throughout the study. After intraveneous injection the CNP-38 tₘₐₓ was observed at 15 min (earliest time point analyzed), followed by a slow decay in CNP-38 content with a half life time of approx. 24 h. After subcutaneous injection the CNP-38 concentration peaked at a tₘₐₓ of 48 h. At 168 h the CNP-38 concentration was still as high as ca. 50 % of cₘₐₓ. The bioavailability was ca. 50 %.

Similar PK curves were obtained for the N- and the C-terminal signature peptide up to 168 h post dose, indicating the presence of intact CNP-38 in the conjugate.

The favourable long lasting PK over several days and the stability of CNP-38 in the conjugate indicates the suitability of the permanent model compound Lys26 CNP-38 PEG 4x10 kDa conjugate **12g** as a slow releasing CNP-38 prodrug after subcutaneous injection. It can be concluded that similar conjugates having a transiently Lys26 linked CNP-38 (like e.g. **11i**) are suitable CNP-38 prodrugs providing long lasting levels of released bioactive CNP-38 over several days.

### Example 25

### Pharmacokinetic study of transient Lys26 CNP-38 PEG4x10 kDa conjugate 11i in cynomolgus monkeys

This study is performed in order to show the suitability of **11i** as slow release CNP-38 prodrug in cynomolgus monkeys. The study is performed as described for example 24. Plasma levels of total CNP-38 content (conjugated and released CNP-38) are analyzed as described in example 24. In order to analyze the plasma content of free CNP-38, the blood samples have to be acidified after withdrawal (e.g. by adding 20 vol% of 0.5 M sodium citrate buffer pH 4) to stop further CNP-38 release from the conjugate. Free CNP-38 levels in plasma can e.g. be determined by ELISA using an CNP antibody that binds to the ring region of CNP, as described in the literature (US patent 8,377,884 B2), or by LC-MS/MS.

### Example 26

### Pharmacodynamic study of transient Lys26 CNP-38 PEG4x10 kDa conjugate 11i in cynomolgus monkeys

The effects of weekly treatment with the transient Lys26 CNP-38 PEG4x10 kDa conjugate **11i** on bone growth and the levels of bone growth-related biomarkers are evaluated in cynomolgus monkeys. Eight normal male juvenile cynomolgus monkeys (about 2 years of age at the start of the study) are subcutaneously injected once weekly with 16 or 56 nmol/kg/week. Four such monkeys are injected subcutaneously with a daily dose of 8 nmol/kg/day of CNP-38, resulting in a weekly accumulated dose of 56 nmol/kg/week. Four additional monkeys are administered vehicle as control. The total length of treatment is 6 months. Various measures of growth plate expansion and bone growth are made by digital X-ray and magnetic resonance imaging, and by measurement of limb and body lengths externally. Blood and urine samples are collected periodically for clinical pathology and measurement. At termination of the study, gross pathology is performed and tissue samples are evaluated histologically for assessment of efficacy and safety.

### Abbreviations:

- ACH: achondroplasia
- ACN: acetonitrile
- AcOH: acetic acid
- Bn: benzyl
- Boc: tert-butyloxycarbonyl
- BSA: bovine serum albumin
- cGMP: cyclic guanosine monophosphate
- CNP: C-type natriuretic peptide
- COMU: (1-cyano-2-ethoxy-2-oxoethylidenaminooxy)dimethylamino-morpholino-carbenium hexafluorophosphate
- conc.: Concentrated
- d: day
- DBU: 1,3-diazabicyclo[5.4.0]undecene
- DCC: N,N'*-*dicyclohexylcarbodiimide
- DCM: dichloromethane
- DIC: N,N'-diisopropylcarbodiimide
- DIEA: N,N-diisopropylethylamine
- DIPEA: N,N-diisopropylethylamine
- DMAP: dimethylamino-pyridine
- DMEM: Dulbecco's modified Eagle's medium
- Dmb: 2,4-dimethylbenzyl
- DMEM: Dulbecco's modified eagle medium
- DMF: N,N-dimethylformamide
- DMSO: dimethylsulfoxide
- DTT: dithiothreitol
- EC50: half maximal effective concentration
- EDC: 1-ethyl-3-(3-dimethylaminopropyl)carbodiimide
- EDTA: ethylenediaminetetraacetic acid
- ELISA: enzyme-linked immunosorbent assay
- eq: stoichiometric equivalent
- ESI-MS: electrospray ionization mass spectrometry

- Et: ethyl
- EtOAc: ethyl acetate
- EtOH: ethanol
- FBS: fetal bovine serum
- FGFR3: fibroblast-growth-factor-receptor 3
- Fmoc: 9-fluorenylmethyloxycarbonyl
- h: hour
- HATU: O-(7-azabenzotriazole-1-yl)-N,N,N',N'-tetramethyluronium hexafluorophosphate
- HCH: hypochondroplasia
- HFIP: hexafluoroisopropanol
- HPLC: high performance liquid chromatography
- HOBt: N-hydroxybenzotriazole
- IBMX: 3-isobutyl-1-methylxanthine
- iPrOH: 2-propanol
- iv: intravenous
- ivDde: 4,4-dimethyl-2,6-dioxocyclohex-1-ylidene)-3-methylbutyl
- LC: liquid chromatography
- LTQ: linear trap quadrupole
- Mal: 3-maleimido propyl
- Me: methyl
- MeOH: methanol
- min: minutes
- Mmt: monomethoxytrityl
- MS: mass spectrum / mass spectrometry
- MSA: methanesulfonic acid
- MW: molecular weight
- m/z: mass-to-charge ratio
- NEP: neutral endopeptidase
- NHS: N-hydroxy succinimide
- NPR: natriuretic peptide receptor
- OtBu: tert-butyloxy
- PBS: phosphate buffered saline

- PEG: poly(ethylene glycol)
- PFP: pentafluorophenol
- pH: *potentia Hydrogenii*
- Pr: propyl
- PyBOP: benzotriazol-1-yl-oxytripyrrolidinophosphonium hexafluorophosphate
- Q-TOF: quadrupole time-of-flight
- RP-HPLC: reversed-phase high performance liquid chromatography
- rpm: rounds per minute
- rt: room temperature
- SIM: single ion monitoring
- SEC: size exclusion chromatography
- sc: subcutaneous
- t_{1/2}: half life
- TCEP: tris(2-carboxyethyl)phosphine
- TCP: tritylchloride polystyrol
- TD: thanatophoric dysplasia
- TES: triethylsilane
- TFA: trifluoroacetic acid
- THF: tetrahydrofuran
- TIPS: triisoproylsilane
- TMEDA: N,N,N'N'-tetramethylethylene diamine
- Tmob: 2,4,6-trimethoxybenzyl
- TR-FRET: time-resolved fluorescence energy transfer
- Trt: triphenylmethyl, trityl
- UPLC: ultra performance liquid chromatography
- UV: ultraviolet
- vs.: versus
- ZQ: single quadrupole

## Claims

1. A CNP prodrug or a pharmaceutically acceptable salt thereof, wherein the prodrug is of formula (Ia) 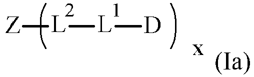 wherein
-D is a CNP moiety comprising a ring moiety of SEQ ID NO:96, wherein -D has the sequence of SEQ ID NO:24, SEQ ID NO:25 or SEQ ID NO:30;
-L¹- is a reversible prodrug linker moiety, wherein -L¹- is conjugated to the side chain of an amino acid residue of the ring moiety of -D or to the backbone of the ring moiety of -D and wherein -L¹- is of formula (III): 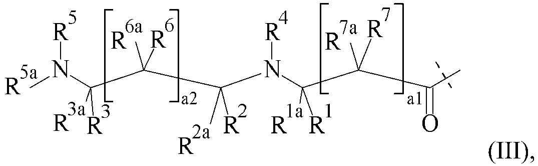 wherein
the dashed line indicates attachment to a primary or secondary amine or hydroxyl of -D by forming an amide or ester linkage, respectively;
-R¹, -R^{1a}, -R², -R^{2a}, -R³ and -R^{3a} are independently of each other selected from the group consisting of -H, -C(R⁸R^{8a}R^{8b}), -C(=O)R⁸, -C≡N, -C(=NR⁸)R^{8a},
-CR⁸(=CR^{8a}R^{8b}), -C≡CR⁸ and -T;
-R⁴, -R⁵ and -R^{5a} are independently of each other selected from the group consisting of -H, -C(R⁹R^{9a}R^{9b}) and -T;
a1 and a2 are independently of each other 0 or 1;
each -R⁶, -R^{6a}, -R⁷, -R^{7a}, -R⁸, -R^{8a}, -R^{8b}, -R⁹, -R^{9a}, -R^{9b} are independently of each other selected from the group consisting of -H, halogen, -CN, -COOR¹⁰, -OR¹⁰, -C(O)R¹⁰, -C(O)N(R¹⁰R^{10a}), -S(O)₂N(R¹⁰R^{10a}), -S(O)N(R¹⁰R^{10a}), -S(O)₂R¹⁰, -S(O)R¹⁰, -N(R¹⁰)S(O)₂N(R^{10a}R^{10b}), -SR¹⁰, -N(R¹⁰R^{10a}), -NO₂, -OC(O)R¹⁰, -N(R¹⁰)C(O)R^{10a}, -N(R¹⁰)S(O)₂R^{10a}, -N(R¹⁰)S(O)R^{10a}, -N(R¹⁰)C(O)OR^{10a}, -N(R¹⁰)C(O)N(R^{10a}R^{10b}), -OC(O)N(R¹⁰R^{10a}), -T, C₁₋₂₀ alkyl, C₂₋₂₀ alkenyl, and C₂₋₂₀ alkynyl; wherein -T, C₁₋₂₀ alkyl, C₂₋₂₀ alkenyl, and C₂₋₂₀ alkynyl are optionally substituted with one or more -R¹¹, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R¹²)-, -S(O)₂N(R¹²)-, -S(O)N(R¹²)-, -S(O)₂-, -S(O)-, -N(R¹²)S(O)₂N(R^{12a})-, -S-, -N(R¹²)-, -OC(OR¹²)(R^{12a})-, -N(R¹²)C(O)N(R^{12a})-, and -OC(O)N(R¹²)-;
each -R¹⁰, -R^{10a}, -R^{10b} is independently selected from the group consisting of -H, -T, C₁₋₂₀ alkyl, C₂₋₂₀ alkenyl, and C₂₋₂₀ alkynyl; wherein -T, C₁₋₂₀ alkyl, C₂₋₂₀ alkenyl, and C₂₋₂₀ alkynyl are optionally substituted with one or more -R¹¹, which are the same or different and wherein C₁₋₂₀ alkyl, C₂₋₂₀ alkenyl, and C₂₋₂₀ alkynyl are optionally interrupted by one or more groups selected from the group consisting of -T-, -C(O)O-, -O-, -C(O)-, -C(O)N(R¹²)-, -S(O)₂N(R¹²)-, -S(O)N(R¹²)-, -S(O)₂-, -S(O)-, -N(R¹²)S(O)₂N(R^{12a})-, -S-, -N(R¹²)-, -OC(OR¹²)(R^{12a})-, -N(R¹²)C(O)N(R^{12a})-, and -OC(O)N(R¹²)-;
each T is independently of each other selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T is independently optionally substituted with one or more -R¹¹, which are the same or different;
each -R¹¹ is independently of each other selected from halogen, -CN, oxo (=O), -COOR¹³, -OR¹³, -C(O)R¹³, -C(O)N(R¹³R^{13a}), -S(O)₂N(R¹³R^{13a}), -S(O)N(R¹³R^{13a}), -S(O)₂R¹³, -S(O)R¹³, -N(R¹³)S(O)₂N(R^{13a}R^{13b}), -SR¹³, -N(R¹³R^{13a}), -NO₂, -OC(O)R¹³, -N(R¹³)C(O)R^{13a}, -N(R¹³)S(O)₂R^{13a}, -N(R¹³)S(O)R^{13a}, -N(R¹³)C(O)OR^{13a}, -N(R¹³)C(O)N(R^{13a}R^{13b}), -OC(O)N(R¹³R^{13a}), and C₁₋₆ alkyl; wherein C₁₋₆ alkyl is optionally substituted with one or more halogen, which are the same or different;
each -R¹², -R^{12a}, -R¹³, -R^{13a}, -R^{13b} is independently selected from the group consisting of -H, and C₁₋₆ alkyl; wherein C₁₋₆ alkyl is optionally substituted with one or more halogen, which are the same or different; optionally, one or more of the pairs -R¹/-R^{1a}, -R²/-R^{2a}, -R³/-R^{3a}, -R⁶/-R^{6a}, -R⁷/-R^{7a} are joined together with the atom to which they are attached to form a C₃₋₁₀ cycloalkyl or a 3- to 10-membered heterocyclyl;
optionally, one or more of the pairs -R¹/-R², -R¹/-R³, -R¹/-R⁴, -R¹/-R⁵, -R¹/-R⁶, -R¹/-R⁷, -R²/-R³, -R²/-R⁴, -R²/-R⁵, -R²/-R⁶, -R²/-R⁷, -R³/-R⁴, -R³/-R⁵, -R³/-R⁶, -R³/-R⁷, -R⁴/-R⁵, -R⁴/-R⁶, -R⁴/-R⁷, -R⁵/-R⁶, -R⁵/-R⁷, -R⁶/-R⁷ are joined together with the atoms to which they are attached to form a ring A;
A is selected from the group consisting of phenyl; naphthyl; indenyl; indanyl; tetralinyl; C₃₋₁₀ cycloalkyl; 3- to 10-membered heterocyclyl; and 8- to 11-membered heterobicyclyl;
wherein -L¹- is substituted with -L²-Z;
-Z is a water-soluble carrier moiety comprising a C₈₋₂₄ alkyl; and
x is 1.

2. The CNP prodrug or a pharmaceutically acceptable salt thereof of claim 1, wherein the CNP moiety has the sequence of SEQ ID NO:24.

3. The CNP prodrug or a pharmaceutically acceptable salt thereof of claim 1, wherein the CNP moiety has the sequence of SEQ ID NO:30.

4. The CNP prodrug or a pharmaceutically acceptable salt thereof of any one of claims 1 to 3, wherein -L¹- is conjugated to the side chain of an amino acid residue of the ring moiety of -D.

5. The CNP prodrug or a pharmaceutically acceptable salt thereof of any one of claims 1 to 4, wherein -L¹- is conjugated to the side chain of a lysine residue of the ring moiety of -D.

6. The CNP prodrug or a pharmaceutically acceptable salt thereof of any one of claims 1, 2, 4 or 5, wherein -D has the sequence of SEQ ID NO:24 and -L¹- is conjugated to the lysine at position 26 of -D.

7. The CNP prodrug or a pharmaceutically acceptable salt thereof of any one of claims 1 to 6, wherein a1 and a2 are 0.

8. The CNP prodrug or a pharmaceutically acceptable salt thereof of any one of claims 1 to 7, wherein Z is an alkyl chain having 8 to 24 carbon atoms, wherein each hydrogen atom of the alkyl chain is optionally replaced by one or more substituents or optionally interrupted by one or more moieties selected from the group consisting of 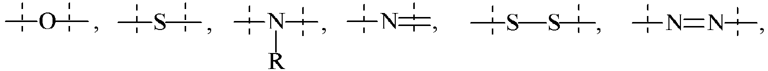 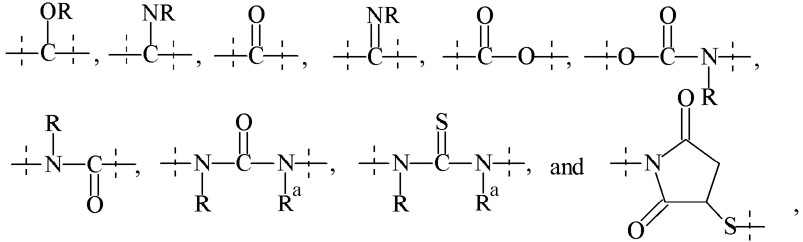 wherein
the dashed lines indicate attachment to the remainder of the moiety or reagent; and
-R and -R^{a} are independently of each other selected from the group consisting of -H, methyl, ethyl, propyl, butyl, pentyl and hexyl.

9. The CNP prodrug or a pharmaceutically acceptable salt thereof of claim 8, wherein the one or more substituents are independently of each other selected from the group consisting of halogen, -CN, -COOR^{x1}, -OR^{x1}, -C(O)R^{x1}, -C(O)N(R^{x1}R^{x1a}), -S(O)₂N(R^{x1}R^{x1a}), -S(O)N(R^{x1}R^{x1a}), -S(O)₂R^{x1}, -S(O)R^{x1}, -N(R^{x1})S(O)₂N(R^{x1a}R^{x1b}), -SR^{x1}, -N(R^{x1}R^{x1a}), -NO2, -OC(O)R^{x1}, -N(R^{x1})C(O)R^{x1a}, -N(R^{x1})S(O)₂R^{x1a}, -N(R^{x1})S(O)R^{x1a}, -N(R^{x1})C(O)OR^{x1a}, -N(R^{x1})C(O)N(R^{x1a}R^{x1b}), -OC(O)N(R^{x1}R^{x1a}), -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, _ S(O)₂N(R)-, -S(O)N(R^{x3})-, -S(O)-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
-R^{x1}, -R^{x1a}, -R^{x1b} are independently of each other selected from the group consisting of -H, -T⁰, C₁₋₅₀ alkyl, C₂₋₅₀ alkenyl, and C₂₋₅₀ alkynyl; wherein -T⁰, C₁₋₅₀ alkyl, C₂₋₅₀ alkenyl, and C₂₋₅₀ alkynyl are optionally substituted with one or more -R^{x2}, which are the same or different and wherein C₁₋₅₀ alkyl, C₂₋₅₀ alkenyl, and C₂₋₅₀ alkynyl are optionally interrupted by one or more groups selected from the group consisting of -T⁰-, -C(O)O-, -O-, -C(O)-, -C(O)N(R^{x3})-, -S(O)₂N(R^{x3})-, -S(O)N(R^{x3})-; -S(O)₂-, -S(O)-, -N(R^{x3})S(O)₂N(R^{x3a})-, -S-, -N(R^{x3})-, -OC(OR^{x3})(R^{x3a})-, -N(R^{x3})C(O)N(R^{x3a})-, and -OC(O)N(R^{x3})-;
each T⁰ is independently selected from the group consisting of phenyl, naphthyl, indenyl, indanyl, tetralinyl, C₃₋₁₀ cycloalkyl, 3- to 10-membered heterocyclyl, and 8- to 11-membered heterobicyclyl; wherein each T⁰ is independently optionally substituted with one or more -R^{x2}, which are the same or different;
each -R^{x2} is independently selected from the group consisting of halogen, -CN, oxo (=O), -COOR^{x4}, -OR^{x4}, -C(O)R^{x4}, -C(O)N(R^{x4}R^{x4a}), -S(O)₂N(R^{x4}R^{x4a}), -S(O)N(R^{x4}R^{x4a}), -S(O)₂R^{x4}, -S(O)R^{x4}, -N(R^{x4})S(O)₂N(R^{x4a}R^{x4b}), -SR^{x4}, -N(R^{x4}R^{x4a}), -NO₂, -OC(O)R^{x4}, -N(R^{x4})C(O)R^{x4a}, -N(R^{x4})S(O)₂R^{x4a}, -N(R^{x4})S(O)R^{x4a}, -N(R^{x4})C(O)OR^{x4a}, -N(R^{x4})C(O)N(R^{x4a}R^{x4b}), -OC(O)N(R^{x4}R^{x4a}), and C₁₋₆ alkyl; wherein C₁₋₆ alkyl is optionally substituted with one or more halogen, which are the same or different;
each -R^{x3}, -R^{x3a}, -R^{x4}, -R^{x4a}, -R^{x4b} is independently selected from the group consisting of -H and C₁₋₆ alkyl; wherein C₁₋₆ alkyl is optionally substituted with one or more halogen, which are the same or different.

10. A pharmaceutical composition comprising at least one CNP prodrug or pharmaceutically acceptable salt thereof of any one of claims 1 to 9 and at least one excipient.

11. The CNP prodrug or a pharmaceutically acceptable salt thereof of any one of claims 1 to 9 or the pharmaceutical composition of claim 10 for use in the treatment of achondroplasia via subcutaneous injection.
